(19) Europäisches Patentamt
European Patent Office
Office européen des brevets

(11) **EP 1 100 795 B1**

(12) **EUROPÄISCHE PATENTSCHRIFT**

(45) Veröffentlichungstag und Bekanntmachung des Hinweises auf die Patenterteilung:
**09.06.2004 Patentblatt 2004/24**

(21) Anmeldenummer: **99938353.2**

(22) Anmeldetag: **27.07.1999**

(51) Int Cl.[7]: **C07D 401/12**

(86) Internationale Anmeldenummer:
**PCT/EP1999/005371**

(87) Internationale Veröffentlichungsnummer:
**WO 2000/008014 (17.02.2000 Gazette 2000/07)**

(54) **DISUBSTITUIERTE BICYCLISCHE HETEROCYCLEN MIT INSBESONDERE EINE THROMBINHEMMENDE WIRKUNG**

DISUBSTITUTED BICYCLIC HETEROCYCLES HAVING, IN PARTICULAR, A THROMBIN INHIBITIVE EFFECT

HETEROCYCLES BICYCLIQUES DISUBSTITUES, EN PARTICULIER A EFFET INHIBITEUR DE LA THROMBINE

(84) Benannte Vertragsstaaten:
**AT BE CH CY DE DK ES FI FR GB GR IE IT LI LU MC NL PT SE**

(30) Priorität: **01.08.1998 DE 19834751**

(43) Veröffentlichungstag der Anmeldung:
**23.05.2001 Patentblatt 2001/21**

(73) Patentinhaber: **Boehringer Ingelheim Pharma GmbH & Co.KG**
**55218 Ingelheim am Rhein (DE)**

(72) Erfinder:
- **HAUEL, Norbert**
  **D-88433 Schemmerhofen (DE)**
- **RIES, Uwe**
  **D-88400 Biberach (DE)**
- **PRIEPKE, Henning**
  **D-88447 Warthausen (DE)**
- **MIHM, Gerhard**
  **D-88400 Biberach (DE)**
- **WIENEN, Wolfgang**
  **D-88400 Biberach (DE)**
- **STASSEN, Jean, Marie**
  **D-88447 Warthausen (DE)**
- **BINDER, Klaus**
  **D-65187 Wiesbaden (DE)**
- **ZIMMERMANN, Rainer**
  **D-88441 Mittelbiberach (DE)**

(74) Vertreter: **Laudien, Dieter, Dr.**
**Boehringer Ingelheim GmbH**
**Abteilung Patente**
**55216 Ingelheim (DE)**

(56) Entgegenhaltungen:
**EP-A- 0 540 051** **WO-A-97/21437**
**WO-A-97/30971**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 100 795 B1

Printed by Jouve, 75001 PARIS (FR)

## Beschreibung

Gegenstand der vorliegenden Erfindung sind neue disubstituierte bicyclische Heterocyclen der allgemeinen Formel

$$R_a - Het - B - Ar - E , \quad (I)$$

deren Tautomere, deren Stereoisomere, deren Gemische und deren Salze, insbesondere deren physiologisch verträgliche Salze mit anorganischen oder organischen Säuren oder Basen, welche wertvolle Eigenschaften aufweisen.

Die Verbindungen der obigen allgemeinen Formel I, in denen E eine Cyanogruppe darstellt, stellen wertvolle Zwischenprodukte zur Herstellung der übrigen Verbindungen der allgemeinen Formel I dar, und die Verbindungen der obigen allgemeinen Formel I, in denen E eine $R_bNH-C(=NH)$-Gruppe darstellt, sowie deren Tautomere und deren Stereoisomere weisen wertvolle pharmakologische Eigenschaften auf, insbesondere eine Thrombinhemmende und die Thrombinzeit verlängernde Wirkung.

Gegenstand der vorliegenden Anmeldung sind somit die neuen Verbindungen der obigen allgemeinen Formel I sowie deren Herstellung, die die pharmakologisch wirksamen Verbindungen enthaltende Arzneimittel und deren Verwendung.

In der obigen allgemeinen Formel bedeutet

B eine gegebenenfalls durch eine oder zwei $C_{1-3}$-Alkylgruppen substituierte Ethylengruppe, wobei eine Methylengruppen der Ethylengruppe, die entweder mit dem Rest Het oder Ar verknüpft ist, durch ein Sauerstoff- oder Schwefelatom, durch eine Sulfinyl-, Sulfonyl-, Carbonyl- oder $-NR_1$-Gruppe ersetzt sein kann, wobei

$R_1$ ein Wasserstoffatom oder eine $C_{1-6}$-Alkylgruppe darstellt,

oder B auch eine geradkettige $C_{3-5}$-Alkylengruppe, in der eine Methylengruppe, die weder mit dem Rest Het noch mit dem Rest Ar verknüpft ist, durch eine $-NR_1$-Gruppe ersetzt ist, in der $R_1$ wie vorstehend erwähnt definiert ist,

E eine Cyano- oder $R_bNH-C(=NH)$-Gruppe, in der
$R_b$ ein Wasserstoffatom, eine Hydroxygruppe, eine $C_{1-3}$-Alkylgruppe oder einen in vivo abspaltbaren Rest darstellt,

Ar eine gegebenenfalls durch ein Fluor-, Chlor- oder Bromatom, durch eine Trifluormethyl-, $C_{1-3}$-Alkyl- oder $C_{1-3}$-Alkoxygruppe substituierte Phenylen- oder Naphthylengruppe,
eine gegebenenfalls im Kohlenstoffgerüst durch eine $C_{1-3}$-Alkylgruppe substituierte Thienylen-, Thiazolylen-, Pyridinylen-, Pyrimidinylen-, Pyrazinylen- oder Pyridazinylengruppe,

Het einen bicyclischen Heterocyclus der Formel

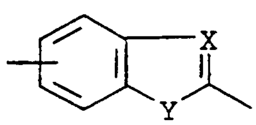 ,

in der
X ein Stickstoffatom oder eine gegebenenfalls durch eine $C_{1-3}$-Alkylgruppe substituierte Methingruppe und
Y eine gegebenenfalls durch eine $C_{1-5}$-Alkyl- oder $C_{3-7}$-Cycloalkylgruppe substituierte Iminogruppe, ein Sauerstoffoder Schwefelatom oder
X ein Stickstoffatom und
Y eine durch eine $C_{1-5}$-Alkyl- oder $C_{3-7}$-Cycloalkylgruppe substituierte Iminogruppe, wobei der Alkyl- und Cycloalkylsubstituent jeweils durch eine Carboxygruppe oder eine in-vivo in eine Carboxygruppe überführbare Gruppe substituiert ist, wobei in einem der vorstehend erwähnten Heterocyclen zusätzlich eine nicht angulare Methingruppe durch ein Stickstoffatom ersetzt sein kann,
oder Het eine Gruppe der Formeln

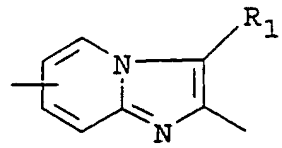

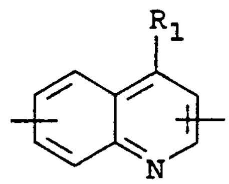

oder

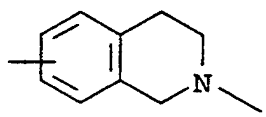
,

wobei

$R_1$ wie vorstehend erwähnt definiert ist,

und $R_a$ eine Phenyl-$C_{1-3}$-alkoxygruppe,
eine Aminogruppe,
eine $C_{1-3}$-Alkylaminogruppe, die am Stickstoffatom zusätzlich durch eine Phenyl-$C_{1-3}$-alkylgruppe substituiert ist,
eine $R_3$-CO-$R_4$N- oder $R_3$-$SO_2$-$R_4$N-Gruppe, in denen
$R_3$ eine $C_{1-5}$-Alkyl-, Phenyl-$C_{1-3}$-alkyl-, $C_{3-7}$-Cycloalkyl-, Phenyl-, Naphthyl-, Pyridyl-, Chinolyl-, Isochinolyl-, Tetrahydrochinolyl- oder Tetrahydroisochinolylgruppe und
$R_4$ ein Wasserstoffatom, $C_{1-5}$-Alkyl- oder Phenyl-$C_{1-3}$-alkylgruppe, die jeweils im Alkylteil durch eine in vivo in eine Carboxygruppe überführbare Gruppe, durch eine Carboxy- oder Tetrazolylgruppe, durch eine Aminocarbonyl- oder $C_{1-3}$-Alkylaminocarbonylgruppe, die jeweils am Stickstoffatom zusätzlich durch eine in vivo in eine Carboxy-$C_{1-3}$-alkylgruppe überführbare Gruppe oder durch eine Carboxygruppe substituiert sind, eine endständig durch eine Di-($C_{1-3}$-Alkyl)-aminogruppe substituierte $C_{2-5}$-Alkylgruppe oder eine $C_{3-7}$-Cycloalkylgruppe darstellen.

Unter einer in-vivo in eine Carboxygruppe überführbare Gruppe ist beispielsweise eine Hydroxymethylgruppe, eine mit einem Alkohol veresterte Carboxygruppe, in der der alkoholische Teil vorzugsweise ein $C_{1-6}$-Alkanol, ein Phenyl-$C_{1-3}$-alkanol, ein $C_{3-9}$-Cycloalkanol, wobei ein $C_{5-8}$-Cycloalkanol zusätzlich durch ein oder zwei $C_{1-3}$-Alkylgruppen substituiert sein kann, ein $C_{5-8}$-Cycloalkanol, in dem eine Methylengruppe in 3- oder 4-Stellung durch ein Sauerstoffatom oder durch eine gegebenenfalls durch eine $C_{1-3}$-Alkyl-, Phenyl-$C_{1-3}$-alkyl-, Phenyl-$C_{1-3}$-alkoxycarbonyl- oder $C_{2-6}$-Alkanoylgruppe substituierte Iminogruppe ersetzt ist und der Cycloalkanolteil zusätzlich durch ein oder zwei $C_{1-3}$-Alkylgruppen substituiert sein kann, ein $C_{4-7}$-Cycloalkenol, ein $C_{3-5}$-Alkenol, ein Phenyl-$C_{3-5}$-alkenol, ein $C_{3-5}$-Alkinol oder Phenyl-$C_{3-5}$-alkinol mit der Maßgabe, daß keine Bindung an das Sauerstoffatom von einem Kohlenstoffatom ausgeht, welches eine Doppel- oder Dreifachbindung trägt, ein $C_{3-8}$-Cycloalkyl-$C_{1-3}$-alkanol, ein Bicycloalkanol mit insgesamt 8 bis 10 Kohlenstoffatomen, das im Bicycloalkylteil zusätzlich durch ein oder zwei $C_{1-3}$-Alkylgruppen substituiert ist, ein 1,3-Dihydro-3-oxo-1-isobenzfuranol oder ein Alkohol der Formel

$$R_5\text{-CO-O-}(R_6CR_7)\text{-OH},$$

in dem

$R_5$ eine $C_{1-8}$-Alkyl-, $C_{5-7}$-Cycloalkyl-, Phenyl- oder Phenyl-$C_{1-3}$-alkylgruppe.
$R_6$ ein Wasserstoffatom, eine $C_{1-3}$-Alkyl-, $C_{5-7}$-Cycloalkyloder Phenylgruppe und
$R_7$ ein Wasserstoffatom oder eine $C_{1-3}$-Alkylgruppe darstellen,

oder unter einem von einer Imino- oder Aminogruppe in-vivo abspaltbaren Rest beispielsweise eine Hydroxygruppe, eine Acylgruppe wie die Benzoyl- oder Pyridinoylgruppe oder eine $C_{1-16}$-Alkanoylgruppe wie die Formyl-, Acetyl-, Propionyl-, Butanoyl-, Pentanoyl- oder Hexanoylgruppe, eine Allyloxycarbonylgruppe, eine $C_{1-16}$-Alkoxycarbonylgruppe wie die Methoxycarbonyl-, Ethoxycarbonyl-, Propoxycarbonyl-, Isopropoxycarbonyl-, Butoxycarbonyl-, tert.Butoxycarbonyl-, Pentoxycarbonyl-, Hexoxycarbonyl-, Octyloxycarbonyl-, Nonyloxycarbonyl-, Decyloxycarbonyl-, Undecyloxycarbonyl-, Dodecyloxycarbonyloder Hexadecyloxycarbonylgruppe, eine Phenyl-$C_{1-16}$-alkoxycarbonylgruppe wie die Benzyloxycarbonyl-, Phenylethoxycarbonyloder Phenylpropoxycarbonylgruppe, eine $C_{1-3}$-Alkylsulfonyl-$C_{2-4}$-alkoxycarbonyl-, $C_{1-3}$-Alkoxy-$C_{2-4}$-alkoxy-$C_{2-4}$-alkoxycarbonyl- oder $R_5CO$-O-($R_6CR_7$)-O-CO-Gruppe, in der $R_5$ bis $R_7$ wie vorstehend erwähnt definiert sind,
zu verstehen.

Desweiteren schließen die bei der Definition der vorstehend erwähnten gesättigten Alkyl- und Alkoxyteile, die mehr als 2 Kohlenstoffatome enthalten, sowie die Alkanoyl- und ungesättigten Alkylteile, die mehr als 3 Kohlenstoffatomen enthalten, auch deren verzweigte Isomere wie beispielsweise die Isopropyl-, tert. Butyl-, Isobutylgruppe etc. ein.

Bevorzugte Verbindungen der obigen allgemeinen Formel I sind diejenigen, in denen

B eine gegebenenfalls durch eine oder zwei Methylgruppen substituierte Ethylengruppe, wobei eine Methylengruppen der Ethylengruppe, die entweder mit dem Rest Het oder Ar verknüpft ist, durch ein Sauerstoff- oder Schwefelatom, durch eine Carbonyl- oder -$NR_1$-Gruppe ersetzt sein kann, wobei

$R_1$ ein Wasserstoffatom oder eine Methylgruppe darstellt,

oder B auch eine n-Propylengruppe, in der die mittlere Methylengruppe durch eine -$NR_1$-Gruppe ersetzt ist, in der $R_1$ wie vorstehend erwähnt definiert ist,

E eine Cyano- oder $R_bNH$-C(=NH)-Gruppe, in der
$R_b$ ein Wasserstoffatom, eine $C_{1-8}$-Alkyloxy-carbonyl-, $C_{5-7}$-Cycloalkyloxy-carbonyl-, Benzoyl-, Nicotinoyl- oder Isonicotinoylgruppe darstellt,

Ar eine gegebenenfalls durch ein Fluor-, Chlor- oder Bromatom, durch eine Trifluormethyl-, Methyl- oder Methoxygruppe substituierte Phenylengruppe, oder eine gegebenenfalls im Kohlenstoffgerüst durch eine Methylgruppe substituierte Thienylengruppe,

Het einen bicyclischen Heterocyclus der Formel

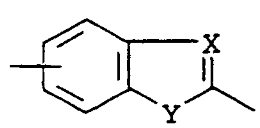
,

in der
X ein Stickstoffatom oder eine gegebenenfalls durch eine Methylgruppe substituierte Methingruppe und
Y eine gegebenenfalls durch eine $C_{1-3}$-Alkyl- oder $C_{3-7}$-Cycloalkylgruppe substituierte Iminogruppe, ein Sauerstoffoder Schwefelatom oder
X ein Stickstoffatom und
Y eine durch eine $C_{1-3}$-Alkylgruppe substituierte Iminogruppe, wobei der Alkylteil zusätzlich durch eine Carboxy- oder $C_{1-3}$-Alkyloxy-carbonylgruppe substituiert ist,
oder Het eine Gruppe der Formeln

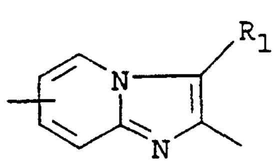
,

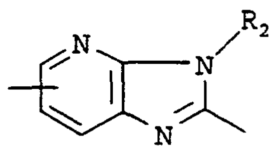

,

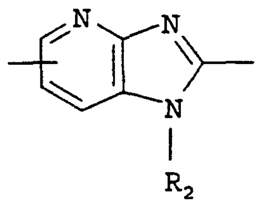

,

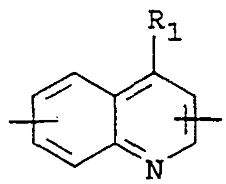

oder

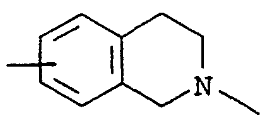

,

wobei

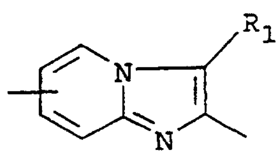

,

$R_1$ wie vorstehend erwähnt definiert ist und

$R_2$ eine durch eine Carboxy- oder $C_{1-3}$-Alkoxy-carbonylgruppe substituiert $C_{1-3}$-Alkylgruppe darstellt,

und $R_a$ eine Benzyloxygruppe,

eine Aminogruppe,

eine $C_{1-3}$-Alkylaminogruppe, die am Stickstoffatom zusätzlich durch eine Benzylgruppe substituiert ist,

eine $R_3$-CO-$R_4$N- oder $R_3$-$SO_2$-$R_4$N-Gruppe, in denen

$R_3$ eine $C_{1-4}$-Alkyl-, Benzyl-, $C_{5-7}$-Cycloalkyl-, Phenyl-, Pyridyl-, Chinolyl-, Isochinolyl-, Tetrahydrochinolyl- oder Tetrahydroisochinolylgruppe und

$R_4$ ein Wasserstoffatom, eine $C_{1-3}$-Alkylgruppe, die durch eine Carboxy-, $C_{1-3}$-Alkoxy-carbonyl-, Tetrazolyl-, Aminocarbonyloder $C_{1-3}$-Alkylaminocarbonylgruppe substituiert ist, wobei die Aminocarbonyl- und $C_{1-3}$-Alkylaminocarbonylgruppe jeweils am Stickstoffatom zusätzlich durch eine eine Carboxy-$C_{1-3}$-alkyl- oder $C_{1-3}$-Alkoxy-carbonyl-$C_{1-3}$-alkylgruppe substituiert sind, oder eine endständig durch eine Di-($C_{1-3}$-alkyl)-aminogruppe substituierte $C_{2-3}$-Alkylgruppe darstellen,

bedeuten, deren Isomere und deren Salze.

Besonders bevorzugte Verbindungen der obigen allgemeinen Formel I sind diejenigen, in denen B eine gegebenenfalls durch eine oder zwei Methylgruppen substituierte Ethylengruppe, wobei eine Methylengruppen der Ethylengruppe, die entweder mit dem Rest Het oder Ar verknüpft ist, durch ein Sauerstoff- oder Schwefelatom, durch eine Carbonyl- oder -$NR_1$-Gruppe ersetzt sein kann, wobei

$R_1$ ein Wasserstoffatom oder eine Methylgruppe darstellt,

oder B auch eine n-Propylengruppe, in der die mittlere Methylengruppe durch eine -$NR_1$-Gruppe ersetzt ist, in der $R_1$ wie vorstehend erwähnt definiert ist,

E eine $R_bNH$-C(=NH)-Gruppe, in der
$R_b$ ein Wasserstoffatom, eine $C_{1-8}$-Alkyloxy-carbonyl-, $C_{5-7}$-Cycloalkyloxy-carbonyl- oder Benzoylgruppe darstellt,

Ar eine gegebenenfalls durch ein Fluor-, Chlor- oder Bromatom, durch eine Trifluormethyl-, Methyl- oder Methoxygruppe substituierte Phenylengruppe, oder eine gegebenenfalls im Kohlenstoffgerüst durch eine Methylgruppe substituierte Thienylengruppe,

Het einen bicyclischen Heterocyclus der Formel

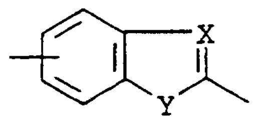

,

in der
X ein Stickstoffatom oder eine gegebenenfalls durch eine Methylgruppe substituierte Methingruppe und
Y eine gegebenenfalls durch eine $C_{1-3}$-Alkyl- oder $C_{3-7}$-Cycloalkylgruppe substituierte Iminogruppe, ein Sauerstoffoder Schwefelatom oder
X ein Stickstoffatom und
Y eine durch eine $C_{1-3}$-Alkylgruppe substituierte Iminogruppe, wobei der Alkylteil zusätzlich durch eine Carboxy- oder $C_{1-3}$-Alkyloxy-carbonylgruppe substituiert ist,
und $R_a$ eine Benzyloxygruppe,
eine Aminogruppe,
eine $C_{1-3}$-Alkylaminogruppe, die am Stickstoffatom zusätzlich durch eine Benzylgruppe substituiert ist,
eine $R_3$-CO-$R_4$N- oder $R_3$-$SO_2$-$R_4$N-Gruppe, in denen
$R_3$ eine $C_{1-4}$-Alkyl-, Benzyl-, $C_{5-7}$-Cycloalkyl-, Phenyl-, Pyridyl-, Chinolyl-, Isochinolyl-, Tetrahydrochinolyl- oder Tetrahydroisochinolylgruppe und
$R_4$ ein Wasserstoffatom, eine $C_{1-3}$-Alkylgruppe, die durch eine Carboxy-, $C_{1-3}$-Alkoxy-carbonyl-, Tetrazolyl-, Aminocarbonyloder $C_{1-3}$-Alkylaminocarbonylgruppe substituiert ist, wobei die Aminocarbonyl- und $C_{1-3}$-Alkylaminocarbonylgruppe jeweils am Stickstoffatom zusätzlich durch eine eine Carboxy-$C_{1-3}$-alkyl- oder $C_{1-3}$-Alkoxy-carbonyl-$C_{1-3}$-alkylgruppe substituiert sind, oder eine endständig durch eine Di-($C_{1-3}$-alkyl)-aminogruppe substituierte $C_{2-3}$-Alkylgruppe darstellen,
bedeuten, insbesondere diejenigen Verbindungen der obigen allgemeinen Formel I, in denen
$R_a$ in 5-Stellung eine $R_3$-CO-$R_4$N- oder $R_3$-$SO_2$-$R_4$N-Gruppe, in denen $R_3$ und $R_4$ wie vorstehend erwähnt definiert sind, deren Isomere und deren Salze.

Ganz besonders bevorzugte Verbindungen sind diejenigen der allgemeinen Formel Ia, in der

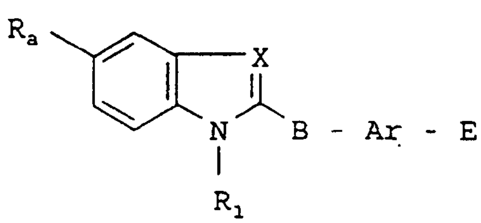

, (Ia)

in der
X eine Methingruppe oder ein Stickstoffatom,

B eine Ethlengruppe, wobei die mit Ar verknüpte Methylengruppe durch ein Sauerstoffatom oder eine Iminogruppe ersetzt sein kann,
Ar eine 1,4-Phenylengruppe,
E eine Amidinogruppe,
$R_1$ eine Methylgruppe und
$R_a$ eine $R_3$-CO-$R_4$N- oder $R_3$-$SO_2$-$R_4$N-Gruppe, wobei
$R_4$ eine durch eine Carboxy-, $C_{1-3}$-Alkoxy-carbonyl-, Carboxymethylaminocarbonyl- oder $C_{1-3}$-Alkoxy-carbonylmethylaminocarbonylgruppe substituierte Methylgruppe und
$R_3$ eine Isochinolin-8-yl-Gruppe darstellen,
bedeuten, insbesondere diejenigen vorstehend erwähnten Verbindungen der allgemeinen Formel Ia, in denen $R_a$ eine $R_3$-$SO_2$-$R_4$N-Gruppe darstellt,
deren Isomere und deren Salze.

Als besonders besonders bevorzugte Verbindungen der obigen allgemeinen Formel I seien beispielsweise folgende erwähnt:

(a) 1-Methyl-2-[(4-amidinophenyl)-oxymethyl]-5-[N-(hydroxycarbonylmethyl)-chinolin-8-sulfonylamino]-benzimidazol,

(b) 1-Methyl-2-[2-(4-amidinophenyl)-ethyl]-5-[N-(N'-(hydroxycarbonylmethyl)-aminocarbonylmethyl)-chinolin-8-sulfonylamino]-benzimidazol,

(c) 1-Methyl-2-[N-(4-amidinophenyl)-aminomethyl]-5-[N-(hydroxycarbonylmethyl)-chinolin-8-sulfonylamino]-benzimidazol und

(d) 1-Methyl-2-[N-(4-amidinophenyl)-aminomethyl]-5-[N-(hydroxycarbonylmethyl)-chinolin-8-sulfonylamino]-indol

sowie deren Salze.

Die neuen Verbindungen lassen sich nach an sich bekannten Verfahren herstellen, beispielsweise nach folgenden Verfahren:

a. Zur Herstellung einer Verbindung der allgemeinen Formel I, in der E eine $R_b$NH-C(=NH)-Gruppe bedeutet, in der $R_b$ ein Wasserstoffatom, eine Hydroxy- oder $C_{1-3}$-Alkylgruppe darstellt:
Umsetzung einer gegebenenfalls im Reaktionsgemisch gebildeten Verbindung der allgemeinen Formel

$$R_a \text{ - Het - B - Ar - C(=NH) - } Z_1 \text{ ,} \qquad \text{(II)}$$

in der
B, Ar, Het und $R_a$ wie eingangs erwähnt definiert sind und
$Z_1$ eine Alkoxy- oder Aralkoxygruppe wie die Methoxy-, Ethoxy-, n-Propoxy-, Isopropoxy- oder Benzyloxygruppe oder eine Alkylthio- oder Aralkylthiogruppe wie die Methylthio-, Ethylthio-, n-Propylthio- oder Benzylthiogruppe darstellt, mit einem Amin der allgemeinen Formel

$$H_2N \text{ - } R_b' \text{ ,} \qquad \text{(III)}$$

in der
$R_b'$ ein Wasserstoffatom, eine Hydroxy- oder $C_{1-3}$-Alkylgruppe darstellt.

Die Umsetzung wird zweckmäßigerweise in einem Lösungsmittel wie Methanol, Ethanol, n-Propanol, Wasser, Methanol/Wasser, Tetrahydrofuran oder Dioxan bei Temperaturen zwischen 0 und 150°C, vorzugsweise bei Temperaturen zwischen 20 und 120°C, mit einer Verbindung der allgemeinen Formel III oder mit einem entsprechenden Säureadditionssalz wie beispielsweise Ammoniumcarbonat durchgeführt.

Eine Verbindung der allgemeinen Formel II erhält man beispielsweise durch Umsetzung einer Verbindung der allgemeinen Formel I, in der E eine Cyanogruppe darstellt, mit einem entsprechenden Alkohol wie Methanol, Ethanol, n-Propanol, Isopropanol oder Benzylalkohol in Gegenwart einer Säure wie Salzsäure oder durch Umsetzung eines entsprechenden Amids mit einem Trialkyloxoniumsalz wie Triethyloxonium-tetrafluorborat in einem Lösungsmittel wie Methylenchlorid, Tetrahydrofuran oder Dioxan bei Temperaturen zwischen 0 und 50°C, vorzugsweise

jedoch bei 20°C, oder eines entsprechenden Nitrils mit Schwefelwasserstoff zweckmäßigerweise in einem Lösungsmittel wie Pyridin oder Dimethylformamid und in Gegenwart einer Base wie Triethylamin und anschließender Alkylierung des gebildeten Thioamids mit einem entsprechenden Alkyl- oder Aralkylhalogenid.

b. Zur Herstellung einer Verbindung der allgemeinen Formel I, in der die $R_a$-Gruppe und E mit der Maßgabe wie eingangs erwähnt definiert sind, daß die $R_a$-Gruppe eine Carboxygruppe enthält und E wie eingangs definiert ist oder die $R_a$-Gruppe wie eingangs erwähnt definiert ist und E eine $NH_2$-C(=NH)-Gruppe darstellt oder die $R_a$-Gruppe eine Carboxygruppe enthält und E eine $NH_2$-C(=NH)-Gruppe darstellt:

Überführung einer Verbindung der allgemeinen Formel

$$R_a' - Het - B - Ar - E' , \qquad (IV)$$

in der

A, B, Ar und Het wie eingangs definiert sind und

die $R_a$'-Gruppe und E' die für die $R_a$-Gruppe und E eingangs erwähnten Bedeutungen mit der Maßgabe besitzen, daß die $R_a$'-Gruppe eine durch Hydrolyse, Behandeln mit einer Säure oder Base, Thermolyse oder Hydrogenolyse in eine Carboxylgruppe überführbare Gruppe enthält und E wie eingangs definiert ist oder E' eine durch Hydrolyse, Behandeln mit einer Säure oder Base, Thermolyse oder Hydrogenolyse in eine $NH_2$-C(=NH)-Gruppe überführbare Gruppe darstellt und die $R_a$'-Gruppe die für die $R_a$-Gruppe eingangs erwähnten Bedeutungen aufweist oder die $R_a$'-Gruppe eine durch Hydrolyse, Behandeln mit einer Säure oder Base, Thermolyse oder Hydrogenolyse in eine Carboxylgruppe überführbare Gruppe enthält und E' eine durch Hydrolyse, Behandeln mit einer Säure oder Base, Thermolyse oder Hydrogenolyse in eine $NH_2$-C(=NH)-Gruppe überführbare Gruppe darstellt,

mittels Hydrolyse, Behandeln mit einer Säure oder Base, Thermolyse oder Hydrogenolyse in eine Verbindung der allgemeinen Formel I übergefürt wird, in der die $R_a$-Gruppe und E mit der Maßgabe wie eingangs erwähnt definiert sind, daß die $R_a$-Gruppe eine Carboxygruppe enthält und E wie eingangs definiert ist oder die $R_a$-Gruppe die eingangs erwähnten Bedeutungen aufweist und E eine $NH_2$-C(=NH)-Gruppe darstellt oder die $R_a$-Gruppe eine Carboxygruppe enthält und E eine $NH_2$-(C=NH)-Gruppe darstellt.

Als eine in eine Carboxygruppe überführbare Gruppe kommt beispielsweise eine durch einen Schutzrest geschützte Carboxylgruppe wie deren funktionelle Derivate, z. B. deren unsubstituierte oder substituierte Amide, Ester, Thioester, Trimethylsilylester, Orthoester oder Iminoester, welche zweckmäßigerweise mittels Hydrolyse in eine Carboxylgruppe übergeführt werden, deren Ester mit tertiären Alkoholen, z.B. der tert.Butylester, welche zweckmäßigerweise mittels Behandlung mit einer Säure oder Thermolyse in eine Carboxylgruppe übergeführt werden, und

deren Ester mit Aralkanolen, z.B. der Benzylester, welche zweckmäßigerweise mittels Hydrogenolyse in eine Carboxylgruppe übergeführt werden, in Betracht.

Die Hydrolyse wird zweckmäßigerweise entweder in Gegenwart einer Säure wie Salzsäure, Schwefelsäure, Phosphorsäure, Essigsäure, Trichloressigsäure, Trifluoressigsäure oder deren Gemischen oder in Gegenwart einer Base wie Lithiumhydroxid, Natriumhydroxid oder Kaliumhydroxid in einem geeigneten Lösungsmittel wie Wasser, Wasser/Methanol, Wasser/Ethanol, Wasser/Isopropanol, Methanol, Ethanol, Wasser/Tetrahydrofuran oder Wasser/Dioxan bei Temperaturen zwischen -10 und 120°C, z.B. bei Temperaturen zwischen Raumtemperatur und der Siedetemperatur des Reaktionsgemisches, durchgeführt.

Enthält die $R_a$'-Gruppe und/oder E' in einer Verbindung der Formel IV beispielsweise die tert. Butyl- oder tert. Butyloxycarbonylygruppe, so können diese auch durch Behandlung mit einer Säure wie Trifluoressigsäure, Ameisensäure, p-Toluolsulfonsäure, Schwefelsäure, Salzsäure, Phosphorsäure oder Polyphosphorsäure gegebenenfalls in einem inerten Lösungsmittel wie Methylenchlorid, Chloroform, Benzol, Toluol, Diethylether, Tetrahydrofuran oder Dioxan vorzugsweise bei Temperaturen zwischen -10 und 120°C, z.B. bei Temperaturen zwischen 0 und 60°C, oder auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 und 120°C, abgespalten werden.

Enthält die $R_a$'-Gruppe und/oder E' in einer Verbindung der Formel IV beispielsweise die Benzyloxy- oder Benzyloxycarbonylgruppe, so können diese auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureethylester, Dioxan oder Dimethylformamid vorzugsweise bei Temperaturen zwischen 0 und 50°C, z.B. bei Raumtemperatur, und einem Wasserstoffdruck von 1 bis 5 bar abgespalten werden.

c. Zur Herstellung einer Verbindung der allgemeinen Formel I, in der die $R_a$-Gruppe eine der bei der Definition der $R_a$-Gruppe eingangs erwähnten Estergruppen enthält:

Umsetzung einer Verbindung der allgemeinen Formel

$$R_a'' - Het - B - Ar - E , \qquad (V)$$

in der

B, E, Ar und Het wie eingangs definiert sind und

$R_a''$-Gruppe die für die $R_a$-Gruppe eingangs erwähnten Bedeutungen mit der Maßgabe aufweist, daß die $R_a''$-Gruppe eine Carboxylgruppe oder eine mittels eines Alkohols in eine entsprechende Estergruppe überführbare Gruppe enthält, mit einem Alkohol der allgemeinen Formel

$$HO - R_8 , \qquad (VI)$$

in der

$R_8$ der Alkylteil einer der eingangs erwähnten in-vivo abspaltbaren Reste mit Ausnahme der $R_5$-CO-O-($R_5CR_7$) -Gruppe für eine Carboxylgruppe darstellt, oder mit deren Formamidacetalen

oder mit einer Verbindung der allgemeinen Formel

$$Z_2 - R_9 , \qquad (VII)$$

in der

$R_9$ der Alkylteil einer der eingangs erwähnten in-vivo abspaltbaren Reste mit Ausnahme der $R_5$-CO-O-($R_5CR_7$) -Gruppe für eine Carboxylgruppe und

$Z_2$ eine Austrittsgruppe wie ein Halogenatom, z. B. ein Chloroder Bromatom, darstellen.

Die Umsetzung mit einem Alkohol der allgemeinen Formel VI wird zweckmäßigerweise in einem Lösungsmittel oder Lösungsmittelgemisch wie Methylenchlorid, Benzol, Toluol, Chlorbenzol, Tetrahydrofuran, Benzol/Tetrahydrofuran oder Dioxan, vorzugsweise jedoch in einem Alkohol der allgemeinen Formel VI, gegebenenfalls in Gegenwart einer Säure wie Salzsäure oder in Gegenwart eines wasserentziehenden Mittels, z.B. in Gegenwart von Chlorameisensäureisobutylester, Thionylchlorid, Trimethylchlorsilan, Salzsäure, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol- oder N,N'-Thionyldiimidazol, Triphenylphosphin/Tetrachlorkohlenstoff oder Triphenylphosphin/Azodicarbonsäurediethylester gegebenenfalls in Gegenwart einer Base wie Kaliumcarbonat, N-Ethyl-diisopropylamin oder N,N-Dimethylamino-pyridin zweckmäßigerweise bei Temperaturen zwischen 0 und 150°C, vorzugsweise bei Temperaturen zwischen 0 und 80°C, durchgeführt.

Mit einer Verbindung der allgemeinen Formel VII wird die Umsetzung zweckmäßigerweise in einem Lösungsmittel wie Methylenchlorid, Tetrahydrofuran, Dioxan, Dimethylsulfoxid, Dimethylformamid oder Aceton gegebenenfalls in Gegenwart eines Reaktionsbeschleunigers wie Natrium- oder Kaliumiodid und vorzugsweise in Gegenwart einer Base wie Natriumcarbonat oder Kaliumcarbonat oder in Gegenwart einer tertiären organischen Base wie N-Ethyl-diisopropylamin oder N-Methyl-morpholin, welche gleichzeitig auch als Lösungsmittel dienen können, oder gegebenenfalls in Gegenwart von Silberkarbonat oder Silberoxid bei Temperaturen zwischen -30 und 100°C, vorzugsweise jedoch bei Temperaturen zwischen -10 und 80°C, durchgeführt.

d. Zur Herstellung einer Verbindung der allgemeinen Formel I, in der $R_b$ einen in vivo abspaltbaren Rest darstellt:

Umsetzung einer Verbindung der allgemeinen Formel

$$R_a - Het - B - Ar - C(=NH) - NH_2 , \qquad (VIII)$$

in der

$R_a$, Het, B und Ar wie eingangs definiert sind, mit einer Verbindung der allgemeinen Formel

$$Z_3 - R_{10} , \qquad (IX)$$

in der
$R_{10}$ einen in vivo abspaltbaren Rest und
$Z_3$ eine nukleofuge Austrittsgruppe wie ein Halogenatom, z.B. ein Chlor-, Brom- oder Jodatom, bedeuten.

Die Umsetzung wird vorzugsweise in einem Lösungsmittel wie Methanol, Ethanol, Methylenchlorid, Tetrahydrofuran, Toluol, Dioxan, Dimethylsulfoxid oder Dimethylformamid gegebenenfalls in Gegenwart einer anorganischen oder einer tertiären organischen Base, vorzugsweise bei Temperaturen zwischen 20°C und der Siedetemperatur des verwendeten Lösungsmittel, durchgeführt.

Mit einer Verbindung der allgemeinen Formel IX, in der $Z_3$ eine nukleofuge Austrittsgruppe darstellt, wird die Umsetzung vorzugsweise in einem Lösungsmittel wie Methylenchlorid, Acetonitril, Tetrahydrofuran, Toluol, Dimethylformamid oder Dimethylsulfoxid gegebenenfalls in Gegenwart einer Base wie Natriumhydrid, Kaliumcarbonat, Kalium-tert.butylat oder N-Ethyl-diisopropylamin bei Temperaturen zwischen 0 und 60°C, durchgeführt.

e. Zur Herstellung einer Verbindung der allgemeinen Formel I, in der $R_a$ eine Aminogruppe und E eine Cyanogruppe darstellen:

Reduktion einer Nitroverbindung der allgemeinen Formel

$$NO_2 \text{ - Het - B - Ar - CN ,} \qquad (X)$$

in der
B, Ar und Het wie eingangs erwähnt definiert sind.

Die Reduktion wird vorzugsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar, durchgeführt. Dies kann auch mit nascierendem Wasserstoff, z.B. mit Zink/Eisessig, Zink/Salzsäure oder Eisen und dessen geeigneten Salzen/Salzsäure, durchgeführt werden.

f. Zur Herstellung einer Verbindung der allgemeinen Formel I, in der $R_a$ eine Aminogruppe und E eine Cyanogruppe darstellen:

Abspaltung eines Schutzrestes für eine Aminogruppe von einer Verbindung der allgemeinen Formel

$$R_a''' \text{ - Het - B - Ar - CN ,} \qquad (XI)$$

in der
B, Ar und Het wie eingangs erwähnt definiert sind und
$R_a'''$ eine durch einen Schutzrest geschützete Aminogruppe bedeutet.

Als Schutzrest für eine Aminogruppe kommt beispielsweise die Acetyl-, Trifluoracetyl-, Benzoyl-, Ethoxycarbonyl-, tert.Butoxycarbonyl-, Benzyloxycarbonyl-, Benzyl-, Methoxybenzyl-, 2,4-Dimethoxybenzyl- oder Phthalylgruppe in Betracht.

Die Abspaltung eines verwendeten Schutzrestes erfolgt vorzugsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z.B. in Gegenwart von Jodtrimethylsilan, bei Temperaturen zwischen 0 und 100°C, vorzugsweise bei Temperaturen zwischen 10 und 50°C,
die Abspaltung eines Benzyl-, Methoxybenzyl- oder Benzyloxycarbonylrestes vorzugsweise hyrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar,
die Abspaltung einer Methoxybenzylgruppe auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur,
die Abspaltung eines 2,4-Dimethoxybenzylrestes vorzugsweise in Trifluoressigsäure in Gegenwart von Anisol,
die Abspaltung eines tert.Butyl- oder tert.Butyloxycarbonylrestes vorzugsweise durch Behandlung mit einer Säure wie Trifluoressigsäure oder Salzsäure gegebenenfalls unter Verwendung eines Lösungsmittels wie Methylenchlorid, Dioxan oder Ether,
die Abspaltung eines Phthalylrestes vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C,
die Abspaltung eines Allyloxycarbonylrestes auch durch Behandlung mit einer katalytischen Menge Tetrakis-(triphenylphosphin)-palladium(O) vorzugsweise in einem Lösungsmittel wie Tetrahydrofuran und vorzugsweise in Gegenwart eines Überschusses von einer Base wie Morpholin oder 1,3-Dimedon bei Temperaturen zwischen 0 und 100°C, vorzugsweise bei Raumtemperatur und unter Inertgas, oder durch Behandlung mit einer katalytischen Menge von Tris-(triphenylphosphin)-rhodium(I)-chlorid in einem Lösungsmittel wie wässrigem Ethanol und gegebenenfalls in Gegenwart einer Base wie 1,4-Diazabicyclo[2.2.2]octan bei Temperaturen zwischen 20 und 70°C.

g. Zur Herstellung einer Verbindung der allgemeinen Formel I, in der $R_a$ eine $R_3$-CO-$R_4$N- oder $R_3$-$SO_2$-$R_4$N-Gruppe und E eine Cyanogruppe darstellen:

Umsetzung einer Verbindung der allgemeinen Formel

$$R_4NH - Het - B - Ar - CN , \qquad (XII)$$

in der
$R_4$, Het, B und Ar wie eingangs erwähnt definiert sind, mit einer Verbindung der allgemeinen Formel

$$R_3 - X - Z_4 , \qquad (XIII)$$

in der
$R_3$ wie eingangs erwähnt definiert ist,
X eine Carbonyl- oder Sulfonylgruppe und
$Z_4$ eine nukleofuge Austrittsgruppe wie ein Halogenatom, z.B. ein Chlor-, Brom- oder Jodatom, oder auch, falls X eine Carbonylgruppe darstellt, zusammen mit einem Wasserstoffatom des benachbarten Stickstoffatoms eine weitere Kohlenstoff-Stickstoffbindung bedeuten.

Die Umsetzung wird vorzugsweise in einem Lösungsmittel wie Methanol, Ethanol, Methylenchlorid, Tetrahydrofuran, Toluol, Dioxan, Dimethylsulfoxid oder Dimethylformamid gegebenenfalls in Gegenwart einer anorganischen oder einer tertiären organischen Base, vorzugsweise bei Temperaturen zwischen 20°C und der Siedetemperatur des verwendeten Lösungsmittel, durchgeführt.

Mit einer Verbindung der allgemeinen Formel XIII, in der $Z_4$ eine nukleofuge Austrittsgruppe darstellt, wird die Umsetzung vorzugsweise in einem Lösungsmittel wie Methylenchlorid, Acetonitril, Tetrahydrofuran, Toluol, Dimethylformamid oder Dimethylsulfoxid gegebenenfalls in Gegenwart einer Base wie Natriumhydrid, Kaliumcarbonat, Pyridin, Kalium-tert.butylat oder N-Ethyl-diisopropylamin bei Temperaturen zwischen 0 und 60°C, durchgeführt.

h. Zur Herstellung einer Verbindung der allgemeinen Formel I, in der $R_a$ eine $R_3$-CO-$R_4$N- oder $R_3$-$SO_2$-$R_4$N-Gruppe und E eine Cyanogruppe darstellen, wobei $R_4$ mit Ausnahme des Wasserstoffatoms wie eingangs erwähnt definiert ist:

Umsetzung einer Verbindung der allgemeinen Formel

$$R_3 - X - NH - Het - B - Ar - CN , \qquad (XIV)$$

in der
$R_3$, Het, B, Ar und X wie eingangs erwähnt definiert sind, mit einer Verbindung der allgemeinen Formel

$$R_4' - Z_5 , \qquad (XV)$$

in der
$R_4'$ mit Ausnahme des Wasserstoffatoms die für $R_4$ eingangs erwähnten Bedeutungen besitzt und
$Z_5$ eine nukleofuge Austrittsgruppe wie ein Halogenatom, z.B. ein Chlor-, Brom- oder Jodatom, bedeutet.

Die Umsetzung wird vorzugsweise in einem Lösungsmittel wie Methylenchlorid, Acetonitril, Aceton, Tetrahydrofuran, Toluol, Dimethylformamid oder Dimethylsulfoxid zweckmäßigerweise in Gegenwart einer Base wie Natriumhydrid, Kaliumcarbonat, Pyridin, 1,8-Diazobicycl[5.4.0]undec-7-en, Kalium-tert.butylat oder N-Ethyl-diisopropylamin bei Temperaturen zwischen 0 und 60°C, durchgeführt.

i. Zur Herstellung einer Verbindung der allgemeinen Formel I, in der $R_4$ eine $C_{1-5}$-Alkyl- oder Phenyl-$C_{1-3}$-alkylgruppe, die jeweils im Alkylteil durch eine in vivo in eine Carboxygruppe überführbare Gruppe, durch eine Tetrazolylgruppe, durch eine Aminocarbonyl- oder $C_{1-3}$-Alkylaminocarbonylgruppe, die jeweils am Stickstoffatom zusätzlich durch eine durch eine in vivo in eine Carboxy-$C_{1-3}$-alkylgruppe überführbare Gruppe substituiert sind und E eine Cyanogruppe darstellen:

Umsetzung einer Verbindung der allgemeinen Formel

$$R_3 - X - NR_4' - Het - B - Ar - CN , \qquad (XVI)$$

in der
$R_3$, Het, B, Ar und X wie eingangs erwähnt definiert sind und
$R_4'$ eine $C_{1-5}$-Alkyl- oder Phenyl-$C_{1-3}$-alkylgruppe, die jeweils im Alkylteil durch eine in vivo in eine Carboxygruppe überführbare Gruppe, durch eine Tetrazolylgruppe, durch eine Aminocarbonyl- oder $C_{1-3}$-Alkylaminocarbonylgruppe, die jeweils am Stickstoffatom zusätzlich durch eine durch eine in vivo in eine Carboxy-$C_{1-3}$-alkylgruppe überführbare Gruppe substituiert sind, bedeutet, oder deren reaktionsfähigen Derivaten mit einer Verbindung der allgemeinen Formel

$$R_{11} - H , \qquad (XVII)$$

in der
$R_4'$ mit Ausnahme des Wasserstoffatoms die für $R_4$ eingangs erwähnten Bedeutungen besitzt und
$R_{11}$ ein der bei der Definition der Restes $R_4$ eingangs erwähnten Substituenten der $C_{1-5}$-Alkyl- oder Phenyl-$C_{1-3}$-alkylgruppe darstellt, der über eine Carbonylgruppe mit der Rest $R_{11}$ verbunden ist.

Die Umsetzung einer Carbonsäure der allgemeinen Formel XVI wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan gegebenenfalls in Gegenwart eines wasserentziehenden Mittels, z.B. in Gegenwart von Chlorameisensäureisobutylester, Orthokohlensäuretetraethylester, Orthoessigsäuretrimethylester, 2,2-Dimethoxypropan, Tetramethoxysilan, Thionylchlorid, Trimethylchlorsilan, Phosphortrichlorid, Phosphorpentoxid, N,N'-Dicyclohexylcarbodiimid, N,N'-Dicyclohexylcarbodiimid/N-Hydroxysuccinimid, N,N'-Dicyclohexylcarbodiimid/1-Hydroxy-benztriazol, 2-(1H-Benzotriazol-1-yl)-1,1,3,3-tetramethyluronium-tetrafluorborat, 2-(1H-Benzotriazol-1-yl)-1,1,3,3-tetramethyluronium-tetrafluorborat/1-Hydroxy-benztriazol, N,N'-Carbonyldiimidazol oder Triphenylphosphin/Tetrachlorkohlenstoff, und gegebenenfalls unter Zusatz einer Base wie Pyridin, 4-Dimethylaminopyridin, N-Methyl-morpholin oder Triethylamin zweckmäßigerweise bei Temperaturen zwischen 0 und 150°C, vorzugsweise bei Temperaturen zwischen 0 und 100°C, durchgeführt.

Die Umsetzung einer entsprechenden reaktionsfähigen Verbindung der allgemeinen Formel XVI wie deren Ester, Imidazolide oder Halogeniden mit einem Amin der allgemeinen Formel XVII wird vorzugsweise in einem entsprechenden Amin als Lösungsmittel gegebenenfalls in Gegenwart eines weiteren Lösungsmittels wie Methylenchlorid oder Ether und vorzugsweise in Gegenwart einer tertiären organische Base wie Triethylamin, N-Ethyl-diisopropylamin oder N-Methyl-morpholin bei Temperaturen zwischen 0 und 150°C, vorzugsweise bei Temperaturen zwischen 50 und 100°C, durchgeführt.

j. Zur Herstellung einer Benzimidazolyl-, Benzthiazolyl- oder Benzoxazolylverbindung der allgemeinen Formel I, in der B eine Ethylengruppe darstellt:

Umsetzung einer gegebenenfalls im Reaktionsgemisch gebildeten Verbindung der allgemeinen Formel

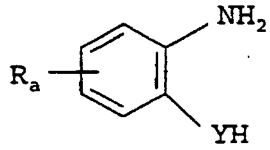

, (XVIII)

in der
$R_a$ und Y wie eingangs erwähnt definiert sind, mit einer Verbindung der allgemeinen Formel

$$HO\text{-}CO - B' - Ar - E, \qquad (IXX)$$

in der
Ar und E wie eingangs erwähnt definiert sind und
B' eine gegebenenfalls durch eine oder zwei $C_{1-3}$-Alkylgruppen substituierte Ethylengruppe bedeutet.

Die Umsetzung wird zweckmäßigerweise in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan gegebenenfalls in Gegenwart eines wasserentziehenden Mittels, z.B. in Gegenwart von Chlorameisensäureisobutylester, Orthokohlensäuretetraethylester, Orthoessigsäuretrimethylester, 2,2-Dimethoxypropan, Tetramethoxysilan, Thionylchlorid, Trimethylchlorsilan, Phosphortrichlorid, Phosphorpentoxid, N,N'-Dicyclohexylcarbodiimid, N, N'-Dicyclohexylcarbodiimid/N-Hydroxysuccinimid, N,N'-Dicyclohexylcarbodiimid/1-Hydroxy-benztriazol, 2-(1H-Benzotriazol-1-yl)-1,1,3,3-tetramethyluronium-tetrafluorborat, 2-(1H-Benzotriazol-1-yl)-1,1,3,3-tetramethyluronium-tetrafluorborat/1-Hydroxybenztriazol, N,N'-Carbonyldiimidazol oder Triphenylphosphin/Tetrachlorkohlenstoff, und gegebenenfalls unter Zusatz einer Base wie Pyridin, 4-Dimethylaminopyridin, N-Methyl-morpholin oder Triethylamin zweckmäßigerweise bei Temperaturen zwischen 0 und 150°C, vorzugsweise bei Temperaturen zwischen 0 und 100°C, durchgeführt.

Die Umsetzung einer entsprechenden reaktionsfähigen Verbindung der allgemeinen Formel IXX wie deren Ester, Imidazolide oder Halogeniden mit einem Amin der allgemeinen Formel XVIII wird vorzugsweise in einem Lösungsmittel wie Methylenchlorid, Ether oder Tetrahydrofuran und vorzugsweise in Gegenwart einer tertiären organische Base wie Triethylamin, N-Ethyl-diisopropylamin oder N-Methyl-morpholin, welche gleichzeitig als Lösungsmittel dienen können, bei Temperaturen zwischen 0 und 150°C, vorzugsweise bei Temperaturen zwischen 50 und 100°C, durchgeführt.

k. Zur Herstellung einer Verbindung der allgemeinen Formel I, die eine der eingangs erwähnten Tetrahydro-chinolin- oder - isochinolinreste enthält:

Hydrierung einer Verbindung der allgemeinen Formel I, die eine der eingangs erwähnten Chinolin- oder -isochinolinreste enthält.

Die Hydrierung wird vorzugsweise in Gegenwart einer Säure wie Salzsäure mit Wasserstoff in Gegenwart eines Katalysators wie Palladium/Kohle und in einem Lösungsmittel wie Methanol, Ethanol, Essigsäureethylester, Dimethylformamid, Dimethylformamid/Aceton oder Eisessig bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar, durchgeführt.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,
als Schutzreste für eine Carboxylgruppe die Trimethylsilyl-, Methyl-, Ethyl-, tert.Butyl- oder Benzylgruppe und als Schutzrest für eine Amino-, Alkylamino- oder Iminogruppe die Acetyl-, Hydroxy-, Trifluoracetyl-, Benzoyl-, Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z.B. in Gegenwart von Jodtrimethylsilan, bei Temperaturen zwischen 0 und 100°C, vorzugsweise bei Temperaturen

zwischen 10 und 50°C.

Die Abspaltung eines Hydroxy-, Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Die Abspaltung eines Allyloxycarbonylrestes erfolgt durch Behandlung mit einer katalytischen Menge Tetrakis-(triphenylphosphin)-palladium(O) vorzugsweise in einem Lösungsmittel wie Tetrahydrofuran und vorzugsweise in Gegenwart eines Überschusses von einer Base wie Morpholin oder 1,3-Dimedon bei Temperaturen zwischen 0 und 100°C, vorzugsweise bei Raumtemperatur und unter Inertgas, oder durch Behandlung mit einer katalytischen Menge von Tris-(triphenylphosphin)-rhodium(I)-chlorid in einem Lösungsmittel wie wässrigem Ethanol und gegebenenfalls in Gegenwart einer Base wie 1,4-Diazabicyclo[2.2.2]octan bei Temperaturen zwischen 20 und 70°C.

Die als Ausgangsstoffe verwendeten Verbindungen der allgemeinen Formeln II bis IXX, welche teilweise literaturbekannt sind, erhält man nach literaturbekannten Verfahren, des weiteren wird ihre Herstellung in den Beispielen beschrieben.

So erhält man beispielsweise eine Verbindung der allgemeinen Formel II durch Umsetzung eines entsprechenden Nitrils, welches seinerseits zweckmäßigerweise gemäß den Verfahren f bis h erhalten wird, mit einem entsprechenden Thio- oder Alkohol in Gegenwart von Chlor- oder Bromwasserstoff.

Eine als Ausgangsstoff verwendete Verbindung der allgemeinen Formeln IV, V, VIII, X, XI und IXX erhält man zweckmäßigerweise gemäß einem Verfahren der vorliegenden Erfindung.

Ferner können die erhaltenen Verbindungen der allgemeinen Formel I in ihre Enantiomeren und/oder Diastereomeren aufgetrennt werden.

So lassen sich beispielsweise die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder

Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Dio-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise der (+)- oder (-)-Menthyloxycarbonylrest in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure, Maleinsäure, Benzoesäure, Methansulfonsäure oder Toluolsulfonsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Der Stand der Tecknik, WO 97/21 437 offenbart naphthylsubstituent Imidazolderivate und ihre Verwendung als Antikoagulantien.

Wie bereits eingangs erwähnt, weisen die neuen Verbindungen der allgemeinen Formel I und deren Salze wertvolle Eigenschaften auf. So stellen die Verbindungen der allgemeinen Formel I, in der E eine Cyanogruppe oder $R_a$ eine Aminogruppe und E eine Cyanogruppe darstellen, wertvolle Zwischenprodukte zur Herstellung der übrigen Verbindungen der allgemeinen Formel I dar und die Verbindungen der allgemeinen Formel I, in der E eine $R_b$NH-C(=NH)-Gruppe darstellt, sowie deren Tautomeren, deren Stereoisomeren, deren physiologisch verträglichen Salze weisen wertvolle pharmakologische Eigenschaften auf, insbesondere eine thrombinhemmende Wirkung, eine die Thrombinzeit verlängernde Wirkung und eine Hemmwirkung auf verwandte Serinproteasen wie z. B. Trypsin, Urokinase, Faktor VIIa, Faktor Xa, Faktor IX, Faktor XI und Faktor XII, wobei auch einige Verbindungen wie beispielsweise die Verbindung des Beispiels 16 gleichzeitig eine thrombozytenaggregationshemmende Wirkung aufweist.

Beispielsweise wurden die Verbindungen

A = 1-Methyl-2-[(4-amidinophenyl)-oxymethyl]-5-[N-(hydroxycarbonylmethyl)-chinolin-8-sulfonylamino]-benzimidazol,

B = 1-Methyl-2-[2-(4-amidinophenyl)-ethyl]-5-[N-(N'-(hydroxycarbonylmethyl)-aminocarbonylmethyl)-chinolin-8-sulfonylamino]-benzimidazol,

C = 1-Methyl-2-[N-(4-amidinophenyl)-aminomethyl]-5-[N-(hydroxycarbonylmethyl)-chinolin-8-sulfonylamino]-benzimidazol und

D = 1-Methyl-2-[N-(4-amidinophenyl)-aminomethyl]-5-[N-(hydroxycarbonylmethyl)-chinolin-8-sulfonylamino]-indol

auf ihre Wirkung auf die Thrombinzeit wie folgt untersucht:

Material: Plasma, aus humanem Citratblut.
Test-Thrombin (Rind), 30 U/ml, Behring Werke, Marburg Diethylbarbituratacetat-Puffer, ORWH 60/61, Behring Werke, Marburg
Biomatic B10 Koagulometer, Sarstedt

Durchführung:

Die Bestimmung der Thrombinzeit erfolgte mit einem Biomatic B10-Koagulometer der Firma Sarstedt.

Die Testsubstanz wurde in die vom Hersteller vorgeschriebenen Testgefäßen mit 0,1 ml humanem Citrat-Plasma und 0,1 ml Diethylbarbiturat-Puffer (DBA-Puffer) gegeben. Der Ansatz wurde für eine Minute bei 37°C inkubiert. Durch Zugabe von 0,3 U Test-Thrombin in 0,1 ml DBA-Puffer wurde die Gerinnungsreaktion gestartet. Gerätebedingt erfolgt mit der Eingabe von Thrombin die Messung der Zeit bis zur Gerinnung des Ansatzes. Als Kontrolle dienten Ansätze bei denen 0,1 ml DBA-Puffer zugegeben wurden.

Gemäß der Definition wurde über eine Dosis-Wirkungskurve die effective Substanzkonzentration ermittelt, bei der die Thrombinzeit gegenüber der Kontrolle verdoppelt wurde.

Die nachfolgende Tabelle enthält die gefundenen Werte:

| Substanz | Thrombinzeit ($ED_{200}$ in $\mu$M) |
|---|---|
| A | 0.015 |
| B | 0.016 |

(fortgesetzt)

| Substanz | Thrombinzeit ($ED_{200}$ in µM) |
|---|---|
| C | 0.031 |
| D | 0.054 |

Beispielsweise konnte an Ratten bei der Applikation der Verbindungen in dem obigen Dosisbereich keine akuten toxischen Nebenwirkungen beobachtet werden. Diese Verbindungen sind demnach gut verträglich.

Aufgrund ihrer pharmakologischen Eigenschaften eignen sich die neuen Verbindungen und deren physiologisch verträglichen Salze zur Vorbeugung und Behandlung venöser und arterieller thrombotischer Erkrankungen, wie zum Beispiel der Behandlung. von tiefen Beinvenen-Thrombosen, der Verhinderung von Reokklusionen nach Bypass-Operationen oder Angioplastie (PT(C)A), sowie der Okklusion bei peripheren arteriellen Erkrankungen wie Lungenembolie, der disseminierten intravaskulären Gerinnung, der Prophylaxe der Koronarthrombose, der Prophylaxe des Schlaganfalls und der Verhinderung der Okklusion von Shunts oder Stents. Zusätzlich sind die erfindungsgemäßen Verbindungen zur antithrombotischen Unterstützung bei einer thrombolytischen Behandlung, wie zum Beispiel mit rt-PA oder Streptokinase, zur Verhinderung der Langzeitrestenose nach PT(C)A, zur Verhinderung der Metastasierung und des Wachstums von koagulationsabhängigen Tumoren und von fibrinabhängigen Entzündungsprozessen, z.B. bei der Behandlung der pulmonaren Fibrose, geeignet.

Die zur Erzielung einer entsprechenden Wirkung erforderliche Dosierung beträgt zweckmäßigerweise bei intravenöser Gabe 0,01 bis 10 mg/kg, vorzugsweise 0,03 bis 3 mg/kg, und bei oraler Gabe 0,1 bis 10 mg/kg, vorzugsweise 0,3 bis 5 mg/kg, jeweils 1 bis 4 x täglich. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Die nachfolgenden Beispiele sollen die Erfindung näher erläutern:

Vorbemerkungen

Bei der Bestimmung der $R_f$-Werte wurden, soweit nichts anderes angegeben wurde, immer Polygram-Kieselgelplatten der Firma E. Merck, Darmstadt, verwendet.

Die EKA-Massenspektren (Elektrospray-Massenspektren von Kationen) werden beispielsweise in Chemie unserer Zeit 6, 308-316 (1991) beschrieben.

Beispiel 1

1-Methyl-2-[2-(4-amidinophenyl)-ethyl]-5-[N-(ethoxycarbonylmethyl)-methansulfonylamino]-benzimidazol

a. 1-Methyl-2-[2-(4-cyanophenyl)-ethyl]-5-nitro-benzimidazol

2.3 g (0.014 Mol) 2-Methylamino-5-nitro-anilin und 2.7 g (0.0154 Mol) 4-Cyanophenylpropionsäure werden in 25 ml Phosphoroxychlorid 1 Stunde zum Rückfluß erhitzt. Nach Abkühlung wird mit Wasser zersetzt und mit Ammoniak alkalisch gestellt. Der Niederschlag wird abgesaugt, mit Wasser gewaschen und getrocknet.
Ausbeute: 3.8 g (89 % der Theorie),
$R_f$-Wert: 0.28 (Kieselgel; Dichlormethan/Methanol = 50:1)

b. 1-Methyl-2-[2-(4-cyanophenyl)-ethyl]-5-amino-benzimidazol

3.8 g (0.0124 Mol) 1-Methyl-2-[2-(4-cyanophenyl)-ethyl]-5-nitro-benzimidazol werden in 100 ml Methanol und 100 ml Dichlormethan gelöst und nach Zugabe von 0.5 g 10%igem Palladium auf Aktivkohle mit Wasserstoff hydriert. Anschließend wird vom Katalysator abfiltriert und eingedampft.
Ausbeute: 3.2 g (93 % der Theorie),
$R_f$-Wert: 0.38 (Kieselgel; Dichlormethan/Methanol = 9:1)

c. 1-Methyl-2-[2-(4-cyanophenyl)-ethyl]-5-methansulfonylaminobenzimidazol

1.6 g (5.8 mMol) 1-Methyl-2-[2-(4-cyanophenyl)-ethyl]-5-aminobenzimidazol und 0.66 g (5.8 mMol) Methansulfonsäurechlorid werden in 30 ml Pyridin 3 Stunden bei Raumtemperatur gerührt. Anschließend wird 1 ml Wasser zugesetzt und eingedampft. Der Rückstand wird mit Essigester und Wasser verdünnt, das kristalline Produkt abgesaugt und getrocknet.
Ausbeute: 1.4 g (68 % der Theorie),
$R_f$-Wert: 0.70 (Kieselgel; Dichlormethan/Methanol = 9:1)

d. 1-Methyl-2-[2-(4-cyanophenyl)-ethyl]-5-[N-(ethoxycarbonylmethyl)-methansulfonylamino]-benzimidazol

1.4 g (3.95 mMol) 1-Methyl-2-[2-(4-cyanophenyl)-ethyl]-5-methansulfonylamino-benzimidazol, 0.73 g (4.4 mMol) Bromessigsäureethylester und 2.8 g (20 mMol) Kaliumcarbonat werden in 200 ml Aceton gelöst und 2 Stunden zum Rückfluß erhitzt. Anschließend wird abfiltriert und die Lösung eingedampft. Ausbeute: 1.6 g (92 % der Theorie),
$R_f$-Wert: 0.76 (Kieselgel; Dichlormethan/Methanol = 9:1)

e. 1-Methyl-2-[2-(4-amidinophenyl)-ethyl]-5-[N-(ethoxycarbonylmethyl)-methansulfonylamino]-benzimidazol

1.6 g (3.63 mMol) 1-Methyl-2-[2-(4-cyanophenyl)-ethyl]-5-[N-(ethoxycarbonylmethyl)-methansulfonylamino]-benzimidazol werden in 50 ml gesättigter ethanolischer Salzsäure gelöst und 5 Stunden bei Raumtemperatur gerührt. Anschließend wird das Solvens abdestilliert, der Rückstand in 30 ml absolutem Ethanol gelöst und mit 3.5 g (3.63 mMol) Ammoniumcarbonat versetzt. Nach 18 Stunden bei Raumtemperatur wird zur Trockene eingedampft und der Rückstand an Kieselgel (Methylenchlorid/-Methanol = 5:1) chromatographiert. Die entsprechende Fraktionen werden eingeengt, der erhaltene Rückstand mit Ether verrieben und abgesaugt.
Ausbeute: 0.9 g (50 % der Theorie),
$R_f$-Wert: 0.36 (Kieselgel; Dichlormethan/Methanol = 5:1)
$C_{22}H_{27}N_5O_4S$ (457.55)

Massenspektrum: $(M+H)^+ = 458$
$(M+Na)^+ = 480$

Beispiel 2

1-Methyl-2-[2-(4-amidinophenyl)-ethyl]-5-[N-(ethoxycarbonylmethyl)-benzolsulfonylamino]-benzimidazol

a. 1-Methyl-2-[2-(4-cyanophenyl)-ethyl]-5-[N-(ethoxycarbonylmethyl)-benzolsulfonylamino]-benzimidazol

Hergestellt analog Beispiel 1d aus 1-Methyl-2-[2-(4-cyanophenyl)-ethyl]-5-benzolsulfonylamino-benzimidazol, Bromessigsäureethylester und Kaliumcarbonat in Aceton.
Ausbeute: 54 % der Theorie,
$R_f$-Wert: 0.84 (Kieselgel; Dichlormethan/Methanol = 9:1)

b. 1-Methyl-2-[2-(4-amidinophenyl)-ethyl]-5-[N-(ethoxycarbonylmethyl)-benzolsulfonylamino]-benzimidazo]

Hergestellt analog Beispiel le aus 1-Methyl-2-[2-(4-cyanophenyl)-ethyl]-5-[N-(ethoxycarbonylmethyl)-benzolsulfonylamino]-benzimidazol und Salzsäure/Ammoniumcarbonat in Ethanol. Ausbeute: 59 % der Theorie,
$R_f$-Wert: 0.38 (Kieselgel; Dichlormethan/Methanol = 5:1)
$C_{27}H_{29}N_5O_4S$ (519.6)

Massenspektrum: $(M+H)^+ = 520$
$(M+Na)^+ = 542$

Beispiel 3

1-Methyl-2-[2-(4-amidinophenyl)-ethyl]-5-[N-(hydroxycarbonylmethyl)-benzolsulfonylamino]-benzimidazol

0.52 g (0.93 mMol) 1-Methyl-2-[2-(4-amidinophenyl)-ethyl]-5-[N-(ethoxycarbonylmethyl)-benzolsulfonylamino]-benzimidazol und 0.4 g (0.01 Mol) Natriumhydroxid werden in 5 ml Wasser und 10 ml Ethanol drei Stunden bei

Raumtemperatur gerührt. Anschließend wird mit Wasser verdünnt und mit Eisessig auf pH 4 eingestellt. Der kristalline Niederschlag wird abgesaugt und getrocknet.
Ausbeute: 77 % der Theorie,
$C_{25}H_{25}N_5O_4S$ (491.66)

Massenspektrum: $(M+H)^+ = 492$
$(M+Na)^+ = 514$
$(M-H+2Na)^+ = 536$
$(2M+H+Na)^{++} = 503$

Beispiel 4

1-Methyl-2-[2-(4-amidinophenyl)-ethyl]-5-[N-(hydroxycarbonylmethyl)-methansulfonylamino]-benzimidazol

Hergestellt analog Beispiel 3 aus 1-Methyl-2-[2-(4-amidinophenyl)-ethyl]-5-[N-(ethoxycarbonylmethyl)-methansulfonylamino]-benzimidazol und Natronlauge.
Ausbeute: 97 % der Theorie,
$C_{20}H_{23}N_5O_4S$ (429.5)

Massenspektrum: $(M+H)^+ = 430$
$(M+Na)^+ = 452$
$(2M+H+Na)^{2+} = 441$
$(2M+3Na)^{3+} = 309$

Beispiel 5

1-Ethoxycarbonylmethyl-2-[(4-amidinophenyl)-oxymethyl]-5-[N-(2-dimethylaminoethyl)-benzolsulfonylamino]-benzimidazol

a. Gemisch aus 1-Ethoxycarbonylmethyl-2-[(4-cyanophenyl)-oxymethyl]-5-nitro-benzimidazol und 1-Ethoxycarbonylmethyl-2-[(4-cyanophenyl)-oxymethyl]-6-nitrobenzimidazol

Hergestellt analog Beispiel 1d aus 1H-2-[(4-Cyanophenyl)-oxymethyl]-5-nitro-benzimidazol, Bromessigsäureethylester und Kaliumcarbonat in Aceton.
Ausbeute: 3.6 g (95 % der Theorie),
$R_f$-Wert: 0.56 (Kieselgel; Dichlormethan/Methanol = 19:1)

b. Gemisch aus 1-Ethoxycarbonylmethyl-2-[(4-cyanophenyl)-oxymethyl]-5-amino-benzimidazol und 1-Ethoxycarbonylmethyl-2-[(4-cyanophenyl)-oxymethyl]-6-aminobenzimidazol

3.6 g (9.5 mMol) des Gemisches aus 1-Ethoxycarbonylmethyl-2-[(4-cyanophenyl)-oxymethyl]-5-nitro-benzimidazol und 1-Ethoxycarbonylmethyl-2-[(4-cyanophenyl)-oxymethyl]-6-nitrobenzimidazol werden in 200 ml Methanol gelöst und nach Zugabe von 0.5 g 10%igem Palladium auf Aktivkohle, mit Wasserstoff hydriert. Anschließend wird vom Katalysator abfiltriert und eingedampft. Der Rückstand wird an Kieselgel (Methylenchlorid + 1 bis 5 % Ethanol) chromatographiert.
Ausbeute: 1.2 g (36 % der Theorie) 1-Ethoxycarbonylmethyl-2-[(4-cyanophenyl)-oxymethyl]-5-amino-benzimidazol $R_f$-Wert: 0.10 (Kieselgel; Dichlormethan/Methanol = 19:1) Ausbeute: 1.0 g (30 % der Theorie) 1-Ethoxycarbonylmethyl-2-[(4-cyanophenyl)-oxymethyl]-6-amino-benzimidazol
$R_f$-Wert: 0.32 (Kieselgel; Dichlormethan/Methanol = 19:1)

c. 1-Ethoxycarbonylmethyl-2-[(4-cyanophenyl)-oxymethyl]-5-benzolsulfonylamino-benzimidazol

Hergestellt analog Beispiel 1c aus 1-Ethoxycarbonylmethyl-2-[(4-cyanophenyl)-oxymethyl]-5-amino-benzimidazol und Benzolsulfonsäurechlorid in Pyridin.
Ausbeute: 100 % der Theorie,
$R_f$-Wert: 0.43 (Kieselgel; Dichlormethan/Methanol = 9:1)

d. 1-Ethoxycarbonylmethyl-2-[(4-cyanophenyl)-oxymethyl]-5-[N-(2-dimethylaminoethyl)-benzolsulfonylamino]-benzimidazol

1.65 g (3.4 mMol) 1-Ethoxycarbonylmethyl-2-[(4-cyanophenyl)-oxymethyl]-5-benzolsulfonylamino-benzimidazol und 518.4 mg (3.6 mMol) 2-Dimethylaminoethylchlorid-hydrochlorid werden in 100 ml Aceton gelöst und nach Zugabe von 2.0 g Kaliumcarbonat und 737 mg (4.85 mMol) 1,8-Diazabicyclo[5.4.0]undec-7-en 11 Stunden zum Rückfluß erhitzt. Anschließend wird abfiltriert und eingedampft. Der Rückstand wird an Kieselgel (Methylenchlorid/5-10 % Ethanol) chromatographiert.
Ausbeute: 750 mg (39 % der Theorie),
$R_f$-Wert: 0.21 (Kieselgel; Dichlormethan/Methanol = 9:1)

e. 1-Ethoxycarbonylmethyl-2-[(4-amidinophenyl)-oxymethyl]-5-[N-(2-dimethylaminoethyl)-benzolsulfonylamino]-benzimidazol

Hergestellt analog Beispiel le aus 1-Ethoxycarbonylmethyl-2-[(4-cyanophenyl)-oxymethyl]-5-[N-(2-dimethylaminoethyl)-benzolsulfonylamino]-benzimidazol und Salzsäure/Ammoniumcarbonat in Ethanol.
Ausbeute: 85 % der Theorie,
$C_{29}H_{34}N_6O_5S$ (578.7)

Massenspektrum: $(M+H)^+ = 579$
$(M+2H)^{++} = 290$

Beispiel 6

1-Ethoxycarbonylmethyl-2-[(4-amidinophenyl)-oxymethyl]-6-[N-(2-dimethylaminoethyl)-benzolsulfonylamino]-benzimidazol

a. 1-Ethoxycarbonylmethyl-2-[(4-cyanophenyl)-oxymethyl]-6-benzolsulfonylamino-benzimidazol

Hergestellt analog Beispiel 1c aus 1-Ethoxycarbonylmethyl-2-[(4-cyanophenyl)-oxymethyl]-6-amino-benzimidazol und Benzolsulfonsäurechlorid in Pyridin.
Ausbeute: 80 % der Theorie,
$R_f$-Wert: 0.72 (Kieselgel; Dichlormethan/Methanol = 9:1)

b. 1-Ethoxycarbonylmethyl-2-[(4-cyanophenyl)-oxymethyl]-6-[N-(2-dimethylaminoethyl)-benzolsulfonylamino]-benzimidazol

Hergestellt analog Beispiel 5d aus 1-Ethoxycarbonylmethyl-2-[(4-cyanophenyl)-oxymethyl]-6-benzolsulfonylamino-benzimidazol, 2-Dimethylaminoethylchlorid-hydrochlorid, Kaliumcarbonat und 1,8-Diazabicyclo[5.4.0]undec-7-en in Aceton.
Ausbeute: 24 % der Theorie,
$R_f$-Wert: 0.34 (Kieselgel; Dichlormethan/Methanol = 9:1)

c. 1-Ethoxycarbonylmethyl-2-[(4-amidinophenyl)-oxymethyl]

6-[N-(2-dimethylaminoethyl)-benzolsulfonylamino]-benzimidazol

Hergestellt analog Beispiel le aus 1-Ethoxycarbonylmethyl-2-[(4-cyanophenyl)-oxymethyl]-6-[N-(2-dimethylaminoethyl)-benzolsulfonylamino]-benzimidazol und Salzsäure/Ammoniumcarbonat in Ethanol.
Ausbeute: 67 % der Theorie,
$C_{29}H_{34}N_6O_5S$ (578.7)

Massenspektrum: $(M+H)^+ = 579$
$(M+2H)^{++} = 290$

Beispiel 7

1-Hydroxycarbonylmethyl-2-[(4-amidinophenyl)-oxymethyl]-5-[N-(2-dimethylaminoethyl)-benzolsulfonylamino]-benzimidazol

Hergestellt analog Beispiel 3 aus 1-Ethoxycarbonylmethyl-2-[(4-amidinophenyl)-oxymethyl]-5-[N-(2-dimethylaminoethyl)-benzolsulfonylamino]-benzimidazol und Natronlauge.
Ausbeute: 91 % der Theorie,
$C_{27}H_{30}N_6O_5S$ (550.65)

Massenspektrum (EKA): $(M+H)^+ = 551$
$(M+2H)^{++} = 276$

Beispiel 8

1-Ethoxycarbonylmethyl-2-[(4-amidinophenyl)-oxymethyl]-5-[N-(2-dimethylaminoethyl)-methansulfonylamino]-benzimidazol

Hergestellt analog Beispiel le aus 1-Ethoxycarbonylmethyl-2-[(4-cyanophenyl)-oxymethyl]-5-[N-(2-dimethylaminoethyl)-methansulfonylamino]-benzimidazol mit ethanolischer Salzsäure und Ammoniumcarbonat.
Ausbeute: 96 % der Theorie,
$C_{24}H_{32}N_6O_5S$ (516.6)

Massenspektrum (EKA): $(M+H)^+ = 517$
$(M+2H)^{++} = 259$

Beispiel 9

1-Ethoxycarbonylmethyl-2-[(4-amidinophenyl)-oxymethyl]-6-[N-(2-dimethylaminoethyl)-methansulfonylamino]-benzimidazol

Hergestellt analog Beispiel le aus 1-Ethoxycarbonylmethyl-2-[(4-cyanophenyl)-oxymethyl]-6-[N-(2-dimethylaminoethyl)-methansulfonylamino]-benzimidazol und ethanolischer Salzsäure, Ethanol und Ammoniumcarbonat.
Ausbeute: 69 % der Theorie,
$C_{24}H_{32}N_6O_5S$ (516.6)

Massenspektrum (EKA): $(M+H)^+ = 517$
$(M+2H)^{++} = 259$

Beispiel 10

1-Hydroxycarbonylmethyl-2-[(4-amidinophenyl)-oxymethyl]-6-[N-(2-dimethylaminoethyl)-methansulfonylamino]-benzimidazol

Hergestellt analog Beispiel 3 aus 1-Ethoxycarbonylmethyl-2-[(4-amidinophenyl)-oxymethyl]-6-[N-(2-dimethylaminoethyl)-methansulfonylamino]-benzimidazol Natronlauge.
Ausbeute: 92 % der Theorie,
$C_{22}H_{28}N_6O_5S$ (488.67)

Massenspektrum (EKA): $(M+H)^+ = 489$
$(M+2H)^{++} = 245$

Beispiel 11

1-Hydroxycarbonylmethyl-2-[(4-amidinophenyl)-oxymethyl]-5-[N-(2-dimethylaminoethyl)-methansulfonylamino)-benzimidazol

Hergestellt analog Beispiel 3 aus 1-Ethoxycarbonylmethyl-2-[(4-amidinophenyl)-oxymethyl]-5-[N-(2-dimethylaminoethyl)-methansulfonylamino]-benzimidazol und Natronlauge.
Ausbeute: 98 % der Theorie,
$C_{22}H_{28}N_6O_5S$ (488.6)

Massenspektrum (EKA): $(M+H)^+ = 489$
$(M+2H)^{++} = 245$

Beispiel 12

1-Methyl-2-[(4-amidinophenyl)-oxymethyl]-5-[N-(ethoxycarbonylmethyl)-chinolin-8-sulfonylamino]-benzimidazol

Hergestellt analog Beispiel le aus 1-Methyl-2-[(4-cyanophenyl)-oxymethyl]-5-[N-(ethoxycarbonylmethyl)-chinolin-8-sulfonylamino]-benzimidazol und ethanolischer Salzsäure, Ethanol und Ammoniumcarbonat.
Ausbeute: 70 % der Theorie,
$C_{29}H_{28}N_6O_5S$ (572.65)

Massenspektrum (EKA): $(M+H)^+ = 573$
$(M+2H)^{++} = 287$
$(M+H+Na)^{++} = 298$

Beispiel 13

1-Ethyl-2-[2-(4-amidinophenyl)-ethyl]-5-[N-(ethoxycarbonylmethyl)-chinolin-8-sulfonylamino]-benzimidazol

Hergestellt analog Beispiel 1e aus 1-Ethyl-2-[2-(4-cyanophenyl)-ethyl]-5-[N-(ethoxycarbonylmethyl)-chinolin-8-sulfonylamino]-benzimidazol und ethanolischer Salzsäure, Ethanol und Ammoniumcarbonat.
Ausbeute: 85 % der Theorie,
$C_{31}H_{32}N_6O_4S$ (584.71)
$R_f$-Wert: 0.32 (Kieselgel; Dichlormethan/Methanol = 5:1)

Massenspektrum (EKA): $(M+H)^+ = 585$
$(M+H+Na)^{++} = 304$
$(M+2H)^{++} = 293$

Beispiel 14

1-Methyl-2-[2-(4-amidinophenyl)-ethyl]-5-[N-(ethoxycarbonylmethyl)-chinolin-8-sulfonylamino]-benzimidazol

Hergestellt analog Beispiel 1e aus 1-Methyl-2-[2-(4-cyanophenyl)-ethyl]-5-[N-(ethoxycarbonylmethyl)-chinolin-8-sulfonylamino]-benzimidazol und ethanolischer Salzsäure, Ethanol und Ammoniumcarbonat.
Ausbeute: 71 % der Theorie,
$C_{30}H_{30}N_6O_4S$ (570.68)
$R_f$-Wert: 0.30 (Kieselgel; Dichlormethan/Methanol = 5:1)

Massenspektrum (EKA): $(M+H)^+ = 571$
$(M+H+Na)^{++} = 297$
$(M+2H)^{++} = 286$

Beispiel 15

1-Cyclopropyl-2-[2-(4-amidinophenyl)-ethyl]-5-[N-(ethoxycarbonylmethyl)-chinolin-8-sulfonylamino]-benzimidazol

Hergestellt analog Beispiel le aus 1-Cyclopropyl-2-[2-(4-cyanophenyl)-ethyl]-5-[N-(ethoxycarbonylmethyl)-chinolin-8-sulfonylamino]-benzimidazol und ethanolischer Salzsäure, Ethanol und Ammoniumcarbonat.
Ausbeute: 76 % der Theorie,
$C_{32}H_{32}N_6O_4S$ (596.72)
$R_f$-Wert: 0.34 (Kieselgel; Dichlormethan/Methanol = 5:1)

Massenspektrum (EKA): $(M+H)^+$ = 597
$(M+H+Na)^{++}$ = 310
$(M+2H)^{++}$ = 299

Beispiel 16

1-Methyl-2-[(4-amidinophenyl)-oxymethyl]-5-[N-(hydroxycarbonylmethyl)-chinolin-8-sulfonylamino]-benzimidazol

Hergestellt analog Beispiel 3 aus 1-Methyl-2-[(4-amidinophenyl)-oxymethyl]-5-[N-(ethoxycarbonylmethyl)-chinolin-8-sulfonylamino]-benzimidazol und Natronlauge.
Ausbeute: 43 % der Theorie,
$C_{27}H_{24}N_6O_5S$ (544.6)

Massenspektrum (EKA) : $(M+H)^+$ = 545
$(M+Na)^+$ = 567
$(M-H)^-$ = 543
$(2M+2Na+H)^{3+}$ = 378.7

$^1$H-NMR ($d_6$-DMSO):
δ = 3.90 (s,3H); 5.05 (s,2H); 5.73 (s,2H); 7.09 (dd,1H); 7.38 (d,2H); 7.52 (d,1H); 7.57-7.74 (m,2H); 7.80 (dd,1H); 7.92 (d,2H); 8.13 (d,1H); 8.31 (d,1H); 8,61 (dd,1H); 9.12-9.30 (m,3H); 9.38 (s,2H) ppm

Beispiel 17

1-Ethyl-2-[2-(4-amidinophenyl)-ethyl]-5-[N-(hydroxycarbonylmethyl)-chinolin-8-sulfonylamino]-benzimidazol

Hergestellt analog Beispiel 3 aus 1-Ethyl-2-[2-(4-amidinophenyl)-ethyl]-5-[N-(ethoxycarbonylmethyl)-chinolin-8-sulfonylamino]-benzimidazol und Natronlauge.
Ausbeute: 74 % der Theorie,
$C_{29}H_{28}N_6O_4S$ (556.65)

Massenspektrum (EKA) : $(M+H)^+$ = 557
$(M+Na)^+$ = 579

Beispiel 18

1-Methyl-2-[2-(4-amidinophenyl)-ethyl]-5-[N-(hydroxycarbonylmethyl)-chinolin-8-sulfonylamino]-benzimidazol

Hergestellt analog Beispiel 3 aus 1-Methyl-2-[2-(4-amidinophenyl)-ethyl]-5-[N-(ethoxycarbonylmethyl)-chinolin-8-sulfonylamino]-benzimidazol und Natronlauge.
Ausbeute: 96 % der Theorie,
$C_{28}H_{26}N_6O_4S$ (542.59)

Massenspektrum (EKA): $(M+H)^+$ = 543
$(M+Na)^+$ = 565
$(2M+3Na)^{3+}$ = 385

Beispiel 19

1-Cyclopropyl-2-[2-(4-amidinophenyl)-ethyl]-5-[N-(hydroxycarbonylmethyl)-chinolin-8-sulfonylamino]-benzimidazol

Hergestellt analog Beispiel 3 aus 1-Cyclopropyl-2-[2-(4-amidinophenyl)-ethyl]-5-[N-(ethoxycarbonylmethyl)-chinolin-8-sulfonylamino]-benzimidazol und Natronlauge.
Ausbeute: 88 % der Theorie,
$C_{30}H_{28}N_6O_4S$ (568.66)

Massenspektrum (EKA): $(M+H)^+$ = 569
$(M+Na)^+$ = 591

$(2M+3Na)^{3+}$ = 402

Beispiel 20

1-Ethyl-2-[(4-amidinophenyl)-oxymethyl]-5-[N-(ethoxycarbonylmethyl)-chinolin-8-sulfonylamino]-benzimidazol

Hergestellt analog Beispiel 1 e aus 1-Ethyl-2-[(4-cyanophenyl)-oxymethyl]-5-[N-(ethoxycarbonylmethyl)-chinolin-8-sulfonylamino]-benzimidazol und ethanolischer Salzsäure, Ethanol und Ammoniumcarbonat.
Ausbeute: 81 % der Theorie,
$C_{30}H_{30}N_6O_5S$ (586.7)

Massenspektrum (EKA): $(M+H)^+$ = 587
$(M+H+Na)^{++}$ = 305
$(M+2H)^{++}$ = 294

Beispiel 21

1-Ethyl-2-[(4-amidinophenyl)-oxymethyl]-5-[N-(hydroxycarbonylmethyl)-chinolin-8-sulfonylamino]-benzimidazol

Hergestellt analog Beispiel 3 aus 1-Ethyl-2-[(4-amidinophenyl)-oxymethyl]-5-[N-(ethoxycarbonylmethyl)-chinolin-8-sulfonylamino]-benzimidazol und Natronlauge.
Ausbeute: 96 % der Theorie,
$C_{28}H_{26}N_6O_5S$ (558.6)

Massenspektrum (EKA): $(M+H)^+$ = 559
$(M+Na)^+$ = 581
$(M-H)^-$ = 557

Beispiel 22

1-Methyl-2-[(4-amidinophenyl)-oxymethyl]-5-[N-(ethoxycarbonylmethyl)-benzolsulfonylamino]-benzimidazol

Hergestellt analog Beispiel 1e aus 1-Methyl-2-[(4-cyanophenyl)-oxymethyl]-5-[N-(ethoxycarbonylmethyl)-benzolsulfonylamino]-benzimidazol und ethanolischer Salzsäure, Ethanol und Ammoniumcarbonat.
Ausbeute: 48 % der Theorie,
$C_{26}H_{27}N_5O_5S$ (521.6)

Massenspektrum (EKA): $(M+H)^+$ = 522
$(M+H+Na)^{++}$ = 272.8

Beispiel 23

1-Methyl-2-[2-(4-amidinophenyl)-ethyl]-5-[N-(N'-(ethoxycarbonylmethyl)-aminocarbonylmethyl)-chinolin-8-sulfonylamino]-benzimidazol

a. 1-Methyl-2-[2-(4-cyanophenyl)-ethyl]-5-[N-(hydroxycarbonylmethyl)-chinolin-8-sulfonylamino]-benzimidazol

Hergestellt analog Beispiel 3 aus 1-Methyl-2-[2-(4-cyanophenyl)-ethyl]-5-[N-(ethoxycarbonylmethyl)-chinolin-8-sulfonylamino]-benzimidazol und Natriumhydroxid in Ethanol/Wasser. Ausbeute: 92 % der Theorie,
$R_f$-Wert: 0.24 (Kieselgel; Dichlormethan/Methanol = 9:1)

b. 1-Methyl-2-[2-(4-cyanophenyl)-ethyl]-5-[N-(N'-(ethoxycarbonylmethyl)-aminocarbonylmethyl)-chinolin-8-sulfonylamino]-benzimidazol

2.1 g (0.004 Mol) 1-Methyl-2-[2-(4-cyanophenyl)-ethyl]-5-[N-(hydroxycarbonylmethyl)-chinolin-8-sulfonylamino]-benzimidazol und 0.65 g (0.004 Mol) N,N'-Carbonyldiimidazol werden in 30 ml Dimethylformamid gelöst und 45 Minuten bei 80°C gerührt. Anschließend werden 0.71 g (0.0046 Mol) Glycinethylester und 0.51 g (0.005 Mol) Triethylamin hinzugefügt und weitere vier Stunden bei 80°C gerührt. Das Solvens wird eingedampft und der Rückstand an

Kieselgel (Methylenchlorid/Ethanol = 50:1) chromatographiert.
Ausbeute: 44 % der Theorie,
$R_f$-Wert: 0.74 (Kieselgel; Dichlormethan/Methanol = 9:1)

c. 1-Methyl-2-[2-(4-amidinophenyl)-ethyl]-5-[N-(N'-(ethoxycarbonylmethyl)-aminocarbonylmethyl)-chinolin-8-sulfonylamino]-benzimidazol

Hergestellt analog Beispiel le aus 1-Methyl-2-[2-(4-cyanophenyl)-ethyl]-5-[N-(N'-(ethoxycarbonylmethyl)-aminocarbonylmethyl)-chinolin-8-sulfonylamino]-benzimidazol und Salzsäure/-Ammoniumcarbonat in Ethanol.
Ausbeute: 86 % der Theorie,
$C_{32}H_{33}N_7O_5S$ (627.73)

Massenspektrum: $(M+H)^+ = 628$
$(M+2H)^{++} = 314.8$
$(M+H+Na)^{++} = 325.7$

Beispiel 24

1-Methyl-2-[2-(4-amidinophenyl)-ethyl]-5-[N-(N'-ethoxycarbonylmethyl-N'-methyl-aminocarbonylmethyl)-chinolin-8-sulfonylamino]-benzimidazol

Hergestellt analog Beispiel le aus 1-Methyl-2-[2-(4-cyanophenyl)-ethyl]-5-[N-(N'-ethoxycarbonylmethyl-N'-methyl-aminocarbonylmethyl)-chinolin-8-sulfonylamino]-benzimidazol und ethanolischer Salzsäure, Ethanol und Ammoniumcarbonat.
Ausbeute: 63 % der Theorie,
$C_{33}H_{35}N_7O_5S$ (641.76)
$R_f$-Wert: 0.30 (Kieselgel; Dichlormethan/Methanol = 5:1)

Massenspektrum (EKA): $(M+H)^+ = 642$
$(M+H+Na)^{++} = 332.8$
$(M+2H)^{++} = 321.7$

Beispiel 25

1-Methyl-2-[2-(4-amidinophenyl)-ethyl]-5-[N- (N'- (hydroxycarbonylmethyl)-aminocarbonylmethyl)-chinolin-8-sulfonylamino]-benzimidazol

Hergestellt analog Beispiel 3 aus 1-Methyl-2-[2-(4-amidinophenyl) -ethyl]-5- [N- (N'- (ethoxycarbonylmethyl) -aminocarbonylmethyl)-chinolin-8-sulfonylamino]-benzimidazol und Natronlauge. Ausbeute: 87 % der Theorie,
$C_{30}H_{29}N_7O_5S$ (599.68)

Massenspektrum (EKA): $(M+H)^+ = 600$
$(M+H+Na)^{++} = 311.8$
$(M+Na)^+ = 622$
$(M+2H)^{++} = 300.8$
$(2M+H+2Na)^{3+} = 415$
$(2M+3Na)^{3+} = 422.7$

$^1$H-NMR ($d_6$-DMSO + DCl) :
δ = 3.29 (t,2H); 3.51 (t,2H); 3.80 (s,2H); 3.87 (s,3H); 5.01 (s,2H); 7.10 (dd,1H); 7.56-7.90 (m,8H); 8.17 (d,1H); 8.38 (d, 1H); 8,68 (dd,1H); 9.26 (dd,1H) ppm

Beispiel 26

1-Methyl-2-[2-(4-amidinophenyl)-ethyl]-5-[N- (N'-hydroxycarbonylmethyl-N'-methyl-aminocarbonylmethyl)-chinolin-8-sulfonylamino]-benzimidazol

Hergestellt analog Beispiel 3 aus 1-Methyl-2-[2-(4-amidinophenyl)-ethyl] -5- [N- (N'-ethoxycarbonylmethyl-N'-methyl-aminocarbonylmethyl)-chinolin-8-sulfonylamino]-benzimidazol und Natronlauge.
Ausbeute: 97 % der Theorie,
$C_{31}H_{31}N_7O_5S$ (613.71)

Massenspektrum (EKA): $(M+H)^+$ = 614
$(M+Na)^+$ = 636
$(M+2H)^{++}$ = 307.7
$(M+H+Na)^{++}$ = 318.6
$(M+2Na)^{++}$ = 329.6

Beispiel 27

1-Methyl-2-[(4-amidinophenyl)-oxymethyl]-5-[N-(hydroxycarbonylmethyl)-benzolsulfonylamino]-benzimidazol

Hergestellt analog Beispiel 3 aus 1-Methyl-2-[(4-amidinophenyl)-oxymethyl]-5-[N-(ethoxycarbonylmethyl)-benzolsulfonylamino]-benzimidazol und Natronlauge.
Ausbeute: 79 % der Theorie,
$C_{24}H_{23}N_5O_5S$ (493.6)

Massenspektrum (EKA): $(M+H)^+$ = 494
$(M+Na)^+$ = 516
$(M-H)^-$ = 492

Beispiel 28

1-Methyl-2-[N-(4-amidinophenyl)-aminomethyl]-5-[N-(methoxycarbonylmethyl)-chinolin-8-sulfonylamino]-benzimidazol

Hergestellt analog Beispiel le aus 1-Methyl-2-[N-(4-cyanophenyl)-aminomethyl]-5-[N-(methoxycarbonylmethyl)-chinolin-8-sulfonylamino]-benzimidazol und methanolischer Salzsäure, Methanol und Ammoniumcarbonat.
Ausbeute: 65 % der Theorie,
$C_{28}H_{27}N_7O_4S$ (557.64)
$R_f$-Wert: 0.33 (Kieselgel; Dichlormethan/Methanol = 5:1)

Massenspektrum (EKA): $(M+H)^+$ = 558
$(M+2H)^{++}$ = 279.7
$(M+H+Na)^{++}$ = 290.7

Beispiel 29

1-Methyl-2- [2- (4-amidinophenyl)-ethyl]-5- [N- (methoxycarbonylmethyl)-chinolin-8-sulfonylamino]-benzimidazol

Hergestellt analog Beispiel le aus 1-Methyl-2-[2-(4-cyanophenyl)-ethyl]-5-[N-(methoxycarbonylmethyl)-chinolin-8-sulfonylamino]-benzimidazol und methanolischer Salzsäure, Methanol und Ammoniumcarbonat.
Ausbeute: 78 % der Theorie,
$C_{29}H_{28}N_6O_4S$ (556.65)
$R_f$-Wert: 0.29 (Kieselgel; Dichlormethan/Methanol = 5:1)

Massenspektrum (EKA): $(M+H)^+$ = 557
$(M+2H)^{++}$ = 579
$(M+H+Na)^{++}$ = 290.3

Beispiel 30

1-Methyl-2-[N-(4-amidinophenyl)-aminomethyl]-5-[N-(hydroxycarbony]methyl)-chinolin-8-sulfonylamino]-benzimidazol

Hergestellt analog Beispiel 3 aus 1-Methyl-2-[N-(4-amidinophenyl)-aminomethyl]-5-[N-(ethoxycarbonylmethyl)-chinolin-8-sulfonylamino]-benzimidazol und Natronlauge.
Ausbeute: 91 % der Theorie,
$C_{27}H_{25}N_7O_4S$ (543.62)

Massenspektrum (EKA) : $(M+H)^+ = 544$
$(M+Na)^+ = 566$
$(M+H+Na)^{++} = 283.8$
$(M+2Na)^{++} = 294.6$

$^1$H-NMR ($d_6$-DMSO) :
δ = 3.70 (s,3H); 4.60 (breites s,4H); 6.45 (dd,1H); 6.72 (d,2H); 7.09 (d,1H); 7.30-7.60 (m,5H); 7.73 (dd,1H); 8.08 (d, 1H); 8.11-8.35 (m,3H); 8.53 (dd,1H); 9,18 (dd,1H); 11.55 (breites s,2H) ppm

Beispiel 31

1-Methyl-2-[2-(4-(N-ethoxycarbonylamidino)phenyl)-ethyl]-5-[N-(methoxycarbonylmethyl)-chinolin-8-sulfonylamino]-benzimidazol

0.8 g (1.34 mMol) 1-Methyl-2-[2-(4-amidinophenyl)-ethyl]-5-[N-(methoxycarbonylmethyl)-chinolin-8-sulfonylamino]-benzimidazol werden in 50 ml Tetrahydrofuran und 10 ml Wasser gelöst und nach Zugabe von 0.55 g (4.0 mMol) Kaliumcarbonat 10 Minuten bei Raumtemperatur gerührt. Anschließend werden 0.17 g (1.6 mMol) Chlorameisensäureethylester hinzugegeben und weitere 60 Minuten bei Raumtemperatur gerührt. Danach wird die organische Phase abgetrennt, getrocknet und eingedampft. Der Rückstand wird an Kieselgel (Methylenchlorid/Methanol = 30:1) chromatographiert. Die entsprechende Fraktionen werden eingeengt, mit Ether verrieben und abgesaugt.
Ausbeute: 0.41 g (49 % der Theorie),
$R_f$-Wert: 0.57 (Kieselgel; Dichlormethan/Methanol = 9:1)
$C_{32}H_{32}N_6O_6S$ (628.71)

Massenspektrum: $(M+H)^+ = 629$
$(M+Na)^+ = 651$
$(M+2H)^{++} = 315$

Beispiel 32

1-Methyl-2-[1-(4-amidinophenoxy)-1-methyl-ethyl]-5-[N-(ethoxycarbonylmethyl)-benzolsulfonylamino]-benzimidazol

Hergestellt analog Beispiel le aus 1-Methyl-2-[1-(4-cyanophenoxy) -1-methyl-ethyl] -5- [N- (ethoxycarbonylmethyl)-benzolsulfonylamino]-benzimidazol und ethanolischer Salzsäure, Ethanol und Ammoniumcarbonat.
Ausbeute: 31 % der Theorie,
$C_{28}H_{31}N_5O_5S$ (549.7)

Massenspektrum (EKA) : $(M+H)^+ = 550$
$(M+Na)^+ = 572$

Beispiel 33

1-Methyl-2-[N-(4-amidinophenyl)-aminomethyl]-5-benzyloxy-benzimidazol

a. 1-Methyl-2-[N-(4-cyanophenyl)-aminomethyl)-5-benzyloxybenzimidazol

Hergestellt analog Beispiel 1a aus 2-Methylamino-5-benzyloxyanilin und 4-Cyanophenylaminoessigsäure in Phosphoroxychlorid. Ausbeute: 11 % der Theorie,
Schmelzpunkt: >350°C
$R_f$-Wert: 0.60 (Kieselgel; Essigester)

b. 1-Methyl-2-[N-(4-amidinophenyl)-aminomethyl]-5-benzyloxybenzimidazol

Hergestellt analog Beispiel le aus 1-Methyl-2-[N-(4-cyanophenyl)-aminomethyl]-5-benzyloxy-benzimidazol

und Salzsäure/Ammoniumcarbonat in Ethanol.
Ausbeute: 66 % der Theorie,
$R_f$-Wert: 0.23 (Kieselgel; Dichlormethan/Methanol = 4:1)
$C_{23}H_{23}N_5O$ (385.47)

Massenspektrum: $(M+H)^+ = 386$
$(M+2H)^{++} = 193.5$

Beispiel 34

1-Methyl-2-[(4-amidinophenyl)-oxymethyl]-5-[N-(methoxycarbonylmethyl)-chinolin-8-sulfonylamino)-benzimidazol

Hergestellt analog Beispiel le aus 1-Methyl-2-[(4-cyanophenyl)-oxymethyl]-5-[N-(methoxycarbonylmethyl)-chinolin-8-sulfonylamino)-benzimidazol und methanolischer Salzsäure, Methanol und Ammoniumcarbonat.
Ausbeute: 68 % der Theorie,
$C_{28}H_{26}N_6O_5S$ (558.6)

Massenspektrum (EKA): $(M+H)^+ = 559$
$(M+2H)^{++} = 280$
$(M+H+Na)^{++} = 291$

Beispiel 35

1-Ethoxycarbonylmethyl-2-[(4-amidinophenyl)-oxymethyl]-5-(chinolin-8-sulfonylamino)-benzimidazol

Hergestellt analog Beispiel 1e aus 1-Ethoxycarbonylmethyl-2-[(4-cyanophenyl)-oxymethyl]-5-(chinolin-8-sulfonylamino)-benzimidazol und ethanolischer Salzsäure, Ethanol und Ammoniumcarbonat.
Ausbeute: 86 % der Theorie,
$C_{28}H_{26}N_6O_5S$ (558.6)

Massenspektrum (EKA): $(M+H)^+ = 559$
$(M+Na)^+ = 581$
$(M+2H)^{++} = 280$

Beispiel 36

1-Methyl-2-[(4-(N-ethoxycarbonylamidino)phenyl)-oxymethyl]-5- [N-(methoxycarbonylmethyl)-chinolin-8-sulfonylamino]-benzimidazol

Hergestellt analog Beispiel 31 aus 1-Methyl-2-[(4-amidinophenyl)-oxymethyl]-5-[N-(methoxycarbonylmethyl)-chinolin-8-sulfonylamino]-benzimidazol und Chlorameisensäureethylester.
Ausbeute: 25 % der Theorie,
$C_{31}H_{30}N_6O_7S$ (630,7)
$R_f$-wert: 0.34 (Kieselgel; Dichlormethan/Ethanol = 19:1)

Massenspektrum (EKA): $(M+H)^+ = 631$
$(M+Na)^+ = 653$
$(M+H+Na)^{++} = 327$

Beispiel 37

1-(3-Ethoxycarbonylpropyl)-2-[(4-amidinophenyl)-oxymethyl]-5-(chinolin-8-sulfonylamino)-benzimidazol

Hergestellt analog Beispiel le aus 1-(3-Ethoxycarbonylpropyl)-2-[(4-cyanophenyl)-oxymethyl]-5-(chinolin-8-sulfonylamino)-benzimidazol und ethanolischer Salzsäure, Ethanol und Ammoniumcarbonat.
Ausbeute: 63 % der Theorie,
$C_{30}H_{30}N_5O_5S$ (586.7)

Massenspektrum (EKA): $(M+H)^+ = 587$
$(M+Na)^+ = 609$
$(M+2H)^{++} = 294$

Beispiel 38

1-Hydroxycarbonylmethyl-2-[(4-amidinophenyl)-oxymethyl]-5-(chinolin-8-sulfonylamino)-benzimidazol

Hergestellt analog Beispiel 3 aus 1-Ethoxycarbonylmethyl-2-[(4-amidinophenyl)-oxymethyl]-5-(chinolin-8-sulfonylamino)-benzimidazol und Natronlauge.
Ausbeute: 97 % der Theorie,
$C_{26}H_{22}N_6O_5S$ (530.6)

Massenspektrum (EKA): $(M+H)^+ = 531$
$(M+Na)^+ = 553$

Beispiel 39

1-(3-Hydroxycarbonylpropyl)-2-[(4-amidinophenyl)-oxymethyl]-5-(chinolin-8-sulfonylamino)-benzimidazol

Hergestellt analog Beispiel 3 aus 1-(3-Ethoxycarbonylpropyl)-2-[(4-amidinophenyl)-oxymethyl]-5-(chinolin-8-sulfonylamino)-benzimidazol und Natronlauge.
Ausbeute: 91 % der Theorie,
$C_{28}H_{26}N_6O_5S$ (558.6)

Massenspektrum (EKA): $(M+H)^+ = 559$
$(M+Na)^+ = 581$
$(M+2H)^{++} = 280$
$(M+H+Na)^{++} = 291$
$(M+H+K)^{++} = 299$

Beispiel 40

1-Methyl-2-[(4-(N-cyclohexyloxycarbonylamidino)phenyl)-oxymethyl]-5-[N-(methoxycarbonylmethyl)-chinolin-8-sulfonylamino)-benzimidazol

Hergestellt analog Beispiel 31 aus 1-Methyl-2-[(4-amidinophenyl)-oxymethyl]-5-[N-(methoxycarbonylmethyl)-chinolin-8-sulfonylamino)-benzimidazol und Chlorameisensäurecyclohexylester.
Ausbeute: 44 % der Theorie,
$C_{35}H_{36}N_6O_7S$ (684.8)

Massenspektrum (EKA): $(M+H)^+ = 685$
$(M+Na)^+ = 707$
$(M+H+Na)^{++} = 354$

Beispiel 41

1-Methyl-2-[(3-amidinophenyl)-oxymethyl]-5-benzolsulfonylamino-benzimidazo]

Hergestellt analog Beispiel le aus 1-Methyl-2-[(3-cyanophenyl)-oxymethyl]-5-(benzolsulfonylamino)-benzimidazol und ethanolischer Salzsäure, Ethanol und Ammoniumcarbonat. Ausbeute: 65 % der Theorie,
$C_{22}H_{21}N_5O_3S$ (435,52)

Massenspektrum (EKA): $(M+H)^+ = 436$
$(M+Na)^+ = 458$

Beispiel 42

1-Methyl-2-[(3-amidinophenyl)-oxymethyl]-5-(chinolin-8-sulfonylamino)-benzimidazol

Hergestellt analog Beispiel le aus 1-Methyl-2-[(3-cyanophenyl)-oxymethyl]-5-(chinolin-8-sulfonylamino)-benzimidazol und ethanolischer Salzsäure, Ethanol und Ammoniumcarbonat.
Ausbeute: 89 % der Theorie,
$C_{25}H_{22}N_6O_3S$ (486,57)
$R_f$-Wert: 0.16 (Kieselgel; Dichlormethan/Ethanol = 4:1)

Massenspektrum (EKA): $(M+H)^+$ = 487
$(M+Na)^+$ = 509

Beispiel 43

1-Methyl-2-[N-(4-amidinophenyl)-N-methyl-aminomethyl]-5-[N-(methoxycarbonylmethyl)-chinolin-8-sulfonylamino]-benzimidazol

Hergestellt analog Beispiel le aus 1-Methyl-2-[N-(4-cyanophenyl)-N-methyl-aminomethyl]-5-[N-(methoxycarbonylmethyl)-chinolin-8-sulfonylamino]-benzimidazol und methanolischer Salzsäure, Methanol und Ammoniumcarbonat.
Ausbeute: 32 % der Theorie,
$C_{29}H_{29}N_7O_4S$ (571.67)
$R_f$-Wert: 0.28 (Kieselgel; Dichlormethan/Methanol = 5:1)

Massenspektrum (EKA): $(M+H)^+$ = 572
$(M+H+Na)^{++}$ = 297.7

Beispiel 44

1-Methyl-2-[N-(4-(N-ethoxycarbonylamidino)phenyl)-aminomethyl]-5-[N-(methoxycarbonylmethyl)-chinolin-8-sulfonylamino]-benzimidazol

Hergestellt analog Beispiel 31 aus 1-Methyl-2-[N-(4-amidinophenyl)-aminomethyl]-5-[N-(methoxycarbonylmethyl)-chinolin-8-sulfonylamino)-benzimidazol und Chlorameisensäureethylester.
Ausbeute: 71 % der Theorie,
$C_{31}H_{31}N_7O_6S$ (629.70)
$R_f$-Wert: 0.62 (Kieselgel; Dichlormethan/Methanol = 9:1)

Massenspektrum (EKA): $(M+H)^+$ = 630
$(M+H+Na)^{++}$ = 326.6
$(M+2H)^{++}$ = 315.6

Beispiel 45

1-Methyl-2-[N-(4-(N-cyclohexyloxycarbonylamidino)phenyl)-aminomethyl]-5-[N-(methoxycarbonylmethyl)-chinolin-8-sulfonylamino]-benzimidazol

Hergestellt analog Beispiel 31 aus 1-Methyl-2-[N-(4-amidinophenyl)-aminomethyl]-5-[N-(methoxycarbonylmethyl)-chinolin-8-sulfonylamino]-benzimidazol und Chlorameisensäurecyclohexylester.
Ausbeute: 59 % der Theorie,
$C_{35}H_{37}N_7O_6S$ (683,79)
$R_f$-Wert: 0.66 (Kieselgel; Dichlormethan/Methanol = 9:1)

Massenspektrum (EKA): $(M+H)^+$ = 684
$(M+H+Na)^{++}$ = 353.7
$(M+2H)^{++}$ = 342.6

Beispiel 46

2-[2-(4-Amidinophenyl)-ethyl]-6-[N-(methoxycarbonylmethyl)-chinolin-8-sulfonylamino]-benzoxazol

a. 2-[(4-Aminocarbonylphenyl)-ethyl]-6-nitro-benzoxazol

2.64 g (15 mMol) 4-Cyanophenylpropionsäure und 2.31 g (15 mMol) 2-Amino-5-nitro-phenol werden in 50 ml Polyphosphorsäure unter Stickstoffatmosphäre drei Stunden bei 130°C gerührt. Anschließend wird auf Wasser gegossen, der Niederschlag abgesaugt, in Methylenchlorid/Methanol gelöst und über Aktivkohle filtriert. Das Filtrat wird im Vakuum eingedampft, der kristalline Rückstand abgesaugt und getrocknet.
Ausbeute: 3.0 g (64 % der Theorie),
$R_f$-Wert: 0.43 (Kieselgel; Dichlormethan/Methanol = 19:1)

h. 2-[2-(4-Cyanophenyl)-ethyl]-6-nitro-benzoxazol

2.0 g (6.43 mMol) 2-[(4-Aminocarbonylphenyl)-ethyl]-6-nitrobenzoxazol werden in 50 ml Phosphoroxychlorid 60 Minuten unter Rückfluß erhitzt. Anschließend wird im Vakuum abdestilliert, der Rückstand mit Eiswasser zersetzt, das kristalline Produkt abgesaugt, gewaschen und getrocknet. Der Rückstand wird an Kieselgel (Methylenchlorid/Ethanol = 99.5:0.5) chromatographiert. Die entsprechenden Fraktionen werden eingeengt, mit Ether verrieben, abgesaugt und getrocknet.
Ausbeute: 1.25 g (66.5 % der Theorie),
$R_f$-Wert: 0.40 (Kieselgel; Dichlormethan/Ethanol = 50:1)

c. 2-[2-(4-Cyanophenyl)-ethyl]-6-amino-benzoxazol

Hergestellt analog Beispiel 1b aus 2-[2-(4-Cyanophenyl)-ethyl]-6-nitro-benzoxazol und Palladium auf Aktivkohle in Methanol/Methylenchlorid.
Ausbeute: 100 % der Theorie,
$R_f$-Wert: 0.59 (Kieselgel; Dichlormethan/Ethanol = 19:1)

d. 2-[2-(4-Cyanophenyl)-ethyl]-6-(N-chinolin-8-sulfonylamino)-benzoxazol

Hergestellt analog Beispiel 1c aus 2-[2-(4-Cyanophenyl)-ethyl]-6-amino-benzoxazol und 8-Chinolinsulfonsäurechlorid in Pyridin.
Ausbeute: 57 % der Theorie,
$R_f$-Wert: 0.61 (Kieselgel; Dichlormethan/Ethanol = 19:1)

e. 2-[2-(4-Cyanophenyl)-ethyl]-6-[N-(methoxycarbonylmethyl)-chinolin-8-sulfonylamino]-benzoxazol

Hergestellt analog Beispiel 1d aus 2-[2-(4-Cyanophenyl)-ethyl]-6-[N-chinolin-8-sulfonylamino]-benzoxazol, Bromessigsäuremethylester und Kaliumcarbonat in Aceton.
Ausbeute: 88.5 % der Theorie,
$R_f$-Wert: 0.30 (Kieselgel; Petrolether/Essigester = 1:1)

f. 2-[2-(4-Amidinophenyl)-ethyl]-6-[N-(methoxycarbonylmethyl)-chinolin-8-sulfonylamino]-benzoxazol

Hergestellt analog Beispiel le aus 2-[2-(4-Cyanophenyl)-ethyl]-6-[N-(methoxycarbonylmethyl)-chinolin-8-sulfonylamino]-benzoxazol und Salzsäure/Ammoniumcarbonat in Methanol. Ausbeute: 82 % der Theorie,
$R_f$-Wert: 0.32 (Kieselgel; Dichlormethan/Ethanol = 4:1)
$C_{28}H_{25}N_5O_5S$ (543.61)

Massenspektrum: $(M+H)^+ = 544$
$(M+2H)^{++} = 272.7$
$(M+H+Na)^{++} = 283.7$

Beispiel 47

2-[2-(4-Amidinophenyl)-ethyl]-6-[N-(hydroxycarbonylmethyl)-chinolin-8-sulfonylamino]-benzoxazol

Hergestellt analog Beispiel 3 aus 2-[2-(4-Amidinophenyl)-ethyl]-6-[N-(methoxycarbonylmethyl)-chinolin-8-sulfonylamino]-benzoxazol und Natronlauge.
Ausbeute: 64 % der Theorie,
$C_{27}H_{23}N_5O_5S$ (529,59)
$R_f$-Wert: 0.11 (Kieselgel; Dichlormethan/Methanol = 4:1)

Massenspektrum (EKA): $(M+H)^+ = 530$
$(M+Na)^+ = 552$
$(M+2H)^{++} = 265.7$
$(M+H+Na)^{++} = 276.7$
$(2M+3Na)^{3+} = 376$

Beispiel 48

2-[(4-Amidinophenyl)-oxymethyl]-5-[N-(ethoxycarbonylmethyl)-chinolin-8-sulfonylamino]-1H-benzimidazol

Hergestellt analog Beispiel le aus 2-[(4-Cyanophenyl)-oxymethyl]-5-[N-(ethoxycarbonylmethyl)-chinolin-8-sulfonylamino]-1H-benzimidazol und ethanolischer Salzsäure, Ethanol und Ammoniumcarbonat.
Ausbeute: 79 % der Theorie,
$C_{28}H_{26}N_6O_5S$ (558,63)
$R_f$-Wert: 0.26 (Kieselgel; Dichlormethan/Methanol = 5:1)

Massenspektrum (EKA): $(M+H)^+ = 559$
$(M+2H)^{++} = 280$
$(M+H+Na)^{++} = 291$

Beispiel 49

1-Methyl-2-[N-(4-amidinobenzyl)-aminomethyl]-5-[N-(ethoxycarbonylmethyl)-chinolin-8-sulfonylamino]-benzimidazol

Hergestellt analog Beispiel le aus 1-Methyl-2-[N-(4-cyanobenzyl)-aminomethyl]-5-[N-(ethoxycarbonylmethyl)-chinolin-8-sulfonylamino]-benzimidazol und ethanolischer Salzsäure, Ethanol und Ammoniumcarbonat.
Ausbeute: 82 % der Theorie,
$C_{30}H_{31}N_7O_4S$ (585.70)
$R_f$-Wert: 0.30 (Kieselgel; Dichlormethan/Methanol = 5:1)

Massenspektrum (EKA): $(M+H)^+ = 586$
$(M+2H)^{++} = 293.7$

Beispiel 50

1-Methyl-2-[N-(4-amidinobenzyl)-aminomethyl]-5-[N-(hydroxycarbonylmethyl)-chinolin-8-sulfonylamino]-benzimidazol

Hergestellt analog Beispiel 3 aus 1-Methyl-2-[N-(4-amidinobenzyl)-aminomethyl]-5-[N-(ethoxycarbonylmethyl)-chinolin-8-sulfonylamino]-benzimidazol und Natronlauge.
Ausbeute: 94 % der Theorie,
$C_{28}H_{27}N_7O_4S$ (557.64)

Massenspektrum (EKA) : $(M+H)^+ = 558$
$(M+Na)^+ = 580$

Beispiel 51

2-[2-(4-Amidinophenyl)-ethyl]-5-[N-(ethoxycarbonylmethyl)-chinolin-8-sulfonylamino]-benzoxazol

a. 2-[2-(4-Cyanophenyl)-ethyl]-5-nitro-benzoxazol

Hergestellt analog Beispiel 46b aus 2-[(4-Aminocarbonylphenyl)-ethyl]-5-nitro-benzoxazol und Phosphoroxychlorid.
Ausbeute: 36 % der Theorie,
$R_f$-Wert: 0.90 (Kieselgel; Dichlormethan/Methanol = 19:1)

b. 2-[2-(4-Cyanophenyl)-ethyl]-5-amino-benzoxazol

Hergestellt analog Beispiel 1b aus 2-[2-(4-Cyanophenyl)-ethyl]-5-nitro-benzoxazol und Palladium auf Aktivkohle in Methanol/Methylenchlorid.
Ausbeute: 100 % der Theorie,
$R_f$-Wert: 0.36 (Kieselgel; Dichlormethan/Methanol = 19:1)

c. 2-[2-(4-Cyanophenyl)-ethyl]-5-(N-chinolin-8-sulfonylamino)-benzoxazol

Hergestellt analog Beispiel 1c aus 2-[2-(4-Cyanophenyl)-ethyl]-5-amino-benzoxazol und 8-Chinolinsulfonsäurechlorid in Pyridin.
Ausbeute: 27 % der Theorie,
$R_f$-Wert: 0.70 (Kieselgel; Dichlormethan/Methanol = 19:1)

d. 2-[2-(4-Cyanophenyl)-ethyl]-5-[N-(ethoxycarbonylmethyl)-chinolin-8-sulfonylamino]-benzoxazol

Hergestellt analog Beispiel 1d aus 2-[2-(4-Cyanophenyl)-ethyl]-5-(N-chinolin-8-sulfonylamino)-benzoxazol, Bromessigsäureethylester und Kaliumcarbonat in Aceton.
Ausbeute: 100 % der Theorie,
$R_f$-Wert: 0.78 (Kieselgel; Dichlormethan/Methanol = 50:1)

e. 2-[2-(4-Amidinophenyl)-ethyl]-5-[N-(ethoxycarbonylmethyl)-chinolin-8-sulfonylamino]-benzoxazol

Hergestellt analog Beispiel le aus 2-[2-(4-Cyanophenyl)-ethyl]-5-[N-(ethoxycarbonylmethyl)-chinolin-8-sulfonylamino]-benzoxazol und Salzsäure/Ammoniumcarbonat in Ethanol. Ausbeute: 98 % der Theorie,
$R_f$-Wert: 0.44 (Kieselgel; Dichlormethan/Methanol = 5:1)
$C_{29}H_{27}N_5O_5S$ (557.63)

Massenspektrum: $(M+H)^+ = 558$
$(M+2H)^{++} = 279.7$
$(M+H+Na)^{++} = 290.7$

Beispiel 52

2-[2-(4-Amidinophenyl)-ethyl]-5-[N-(hydroxycarbonylmethyl)-chinolin-8-sulfonylamino]-benzoxazol

Hergestellt analog Beispiel 3 aus 2-[2-(4-Amidinophenyl)-ethyl]-5-[N-(ethoxycarbonylmethyl)-chinolin-8-sulfonylamino]-benzoxazol und Natronlauge.
Ausbeute: 77 % der Theorie,
$C_{27}H_{23}N_5O_5S$ (529.58)

Massenspektrum (EKA): $(M+H)^+ = 530$
$(M+Na)^+ = 552$
$(M+H+Na)^{++} = 276.6$
$(M-H+2Na)^+ = 574$
$(M+2Na)^{++} = 287.6$

Beispiel 53

1-Methyl-2-[2-(2-amidinothiophen-5-yl)-ethyl]-5-[N-(ethoxycarbonylmethyl)-chinolin-8-sulfonylamino]-benzimidazol

Hergestellt analog Beispiel 1e aus 1-Methyl-2-[2-(2-cyanothiophen-5-yl)-ethyl]-5-[N-(ethoxycarbonylmethyl)-chinolin-8-sulfonylamino]-benzimidazol und ethanolischer Salzsäure, Ethanol und Ammoniumcarbonat.
Ausbeute: 42 % der Theorie,
$C_{28}H_{28}N_6O_4S_2$ (576.71)
$R_f$-Wert: 0.36 (Kieselgel; Dichlormethan/Methanol = 5:1)

Massenspektrum (EKA): $(M+H)^+ = 577$
$(M+2H)^{++} = 289$
$(M+H+Na)^{++} = 300$

Beispiel 54

1-Methyl-2-[2-(2-amidinothiophen-5-yl)-ethyl]-5-[N-(hydroxycarbonylmethyl)-chinolin-8-sulfonylamino]-benzimidazol

Hergestellt analog Beispiel 3 aus 1-Methyl-2-[2-(2-amidinothiophen-5-yl)-ethyl]-5-[N-(ethoxycarbonylmethyl)-chinolin-8-sulfonylamino]-benzimidazol und Natronlauge.
Ausbeute: 98 % der Theorie,
$C_{26}H_{24}N_6O_4S_2$ (548.66)

Massenspektrum (EKA): $(M+H)^+ = 549$
$(M+Na)^+ = 571$

Beispiel 55

1-Methyl-2-[N-(4-amidinophenyl)-aminomethyl]-5-[N-(ethoxycarbonylmethyl)-benzolsulfonylamino]-benzimidazol

Hergestellt analog Beispiel 1e aus 1-Methyl-2-[N-(4-cyanophenyl)-aminomethyl]-5-[N-(ethoxycarbonylmethyl)-benzolsulfonylamino]-benzimidazol und ethanolischer Salzsäure, Ethanol und Ammoniumcarbonat.
Ausbeute: 50 % der Theorie,
$C_{26}H_{28}N_6O_4S$ (520.62)
$R_f$-Wert: 0.34 (Kieselgel; Dichlormethan/Methanol = 5:1) Massenspektrum (EKA): $(M+H)^+ = 521$

Beispiel 56

1-Methyl-2-[N-(4-amidinophenyl)-aminomethyl]-5-[N-(hydroxycarbonylmethyl)-benzolsulfonylamino]-benzimidazol

Hergestellt analog Beispiel 3 aus 1-Methyl-2-[N-(4-amidinophenyl)-aminomethyl]-5-[N-(ethoxycarbonylmethyl)-benzolsulfonylamino]-benzimidazol und Natronlauge.
Ausbeute: 97 % der Theorie,
$C_{24}H_{24}N_6O_4S$ (492.56)

Massenspektrum (EKA): $(M+H)^+ = 493$
$(M+Na)^+ = 515$
$(M-H+2Na)^+ = 537$
$(M+2Na)^{++} = 269$

Beispiel 57

1-Methyl-2-[N-(4-amidinophenyl)-aminomethyl]-5-(N-benzyl-N-methylamino)-benzimidazol

Hergestellt analog Beispiel le aus 1-Methyl-2-[N-(4-cyanophenyl)-aminomethyl]-5-(N-benzyl-N-methylamino)-benzimidazol und ethanolischer Salzsäure, Ethanol und Ammoniumcarbonat.
Ausbeute: 85 % der Theorie,
$C_{24}H_{26}N_6$ (398.51)

$R_f$-Wert: 0.27 (Kieselgel; Dichlormethan/Methanol = 4:1)

Massenspektrum (EKA): $(M+H)^+ = 399$
$(M+2H)^{++} = 200$

Beispiel 58

1-Methyl-2-[N-(4-amidinophenyl)-aminomethyl]-5-[N-(ethoxycarbonylmethyl)-n-butansulfonylamino]-benzimidazol

Hergestellt analog Beispiel le aus 1-Methyl-2-[N-(4-cyanophenyl)-aminomethyl]-5-[N-(ethoxycarbonylmethyl)-n-butansulfonylamino]-benzimidazol und ethanolischer Salzsäure, Ethanol und Ammoniumcarbonat.
Ausbeute: 71 % der Theorie,
$C_{24}H_{32}N_6O_4S$ (500.63)
$R_f$-Wert: 0.32 (Kieselgel; Dichlormethan/Methanol = 5:1)

Massenspektrum (EKA): $(M+H)^+ = 501$
$(M+H+Na)^{++} = 262$

Beispiel 59

1-Methyl-2-[N-(4-amidinophenyl)-aminomethyl]-5-[N-(ethoxycarbonylmethyl)-benzoylamino]-benzimidazol

Hergestellt analog Beispiel le aus 1-Methyl-2-[N-(4-cyanophenyl)-aminomethyl]-5-[N-(ethoxycarbonylmethyl)-benzoylamino]-benzimidazol und ethanolischer Salzsäure, Ethanol und Ammoniumcarbonat.
Ausbeute: 56 % der Theorie,
$C_{27}H_{28}N_6O_3$ (484.57)
$R_f$-Wert: 0.34 (Kieselgel; Dichlormethan/Methanol = 5:1)

Massenspektrum (EKA): $(M+H)^+ = 485$
$(M+H+Na)^{++} = 254$

Beispiel 60

1-Methyl-2-[N-(4-amidinophenyl)-aminomethyl]-5-[N-(ethoxycarbonylmethyl)-pyridin-2-yl-carbonylamino]-benzimidazol

Hergestellt analog Beispiel le aus 1-Methyl-2-[N-(4-cyanophenyl)-aminomethyl]-5-[N-(ethoxycarbonylmethyl)-pyridin-2-ylcarbonylaminol-benzimidazol und ethanolischer Salzsäure, Ethanol und Ammoniumcarbonat.
Ausbeute: 64 % der Theorie,
$C_{26}H_{27}N_7O_3$ (485.56)
$R_f$-Wert: 0.31 (Kieselgel; Dichlormethan/Methanol = 5:1)

Massenspektrum (EKA): $(M+H)^+ = 486$
$(M+H+Na)^{++} = 254.7$

Beispiel 61

1-Methyl-2-[N-(4-amidinophenyl)-aminomethyl]-5-[N-(hydroxycarbonylmethyl)-n-butansulfonylamino]-benzimidazol

Hergestellt analog Beispiel 3 aus 1-Methyl-2-[N-(4-amidinophenyl)-aminomethyl]-5-[N-(ethoxycarbonylmethyl)-n-butansulfonylamino]-benzimidazol und Natronlauge.
Ausbeute: 98 % der Theorie,
$C_{22}H_{28}N_6O_4S$ (472.57)

Massenspektrum (EKA): $(M+H)^+ = 473$
$(M+Na)^+ = 495$
$(M+2Na)^{++} = 259$

Beispiel 62

1-Methyl-2-[N-(4-amidinophenyl)-aminomethyl]-5-[N-(hydroxycarbonylmethyl)-benzoylamino]-benzimidazol

Hergestellt analog Beispiel 3 aus 1-Methyl-2-[N-(4-amidinophenyl)-aminomethyl]-5-[N-(ethoxycarbonylmethyl)-benzoylamino]-benzimidazol und Natronlauge.
Ausbeute: 69 % der Theorie,
$C_{25}H_{24}N_6O_3$ (456.51)

Massenspektrum (EKA): $(M+H)^+ = 457$
$(M+2Na)^{++} = 251$
$(M+Na)^+ = 479$

Beispiel 63

1-Methyl-2-[N-(4-amidinophenyl)-aminomethyl]-5-[N-(hydroxycarbonylmethyl)-pyridin-2-yl-carbonylamino]-benzimidazol

Hergestellt analog Beispiel 3 aus 1-Methyl-2-[N-(4-amidinophenyl)-aminomethyl]-5-[N-(ethoxycarbonylmethyl)-pyridin-2-ylcarbonylamino]-benzimidazol und Natronlauge.
Ausbeute: 96 % der Theorie,
$C_{24}H_{23}N_7O_3$ (457.50)

Massenspektrum (EKA): $(M+H)^+ = 458$
$(M+Na)^+ = 480$
$(M+H+Na)^{++} = 240.6$
$(M+2Na)^{++} = 251.6$

Beispiel 64

1-Methyl-2-[(4-amidinophenyl)-oxymethyl]-5-(N-cyclohexyl-methansulfonylamino)-benzimidazol

Hergestellt analog Beispiel 1e aus 1-Methyl-2-[(4-cyanophenyl)-oxymethyl]-5-(N-cyclohexyl-methansulfonylamino)-benzimidazol und ethanolischer Salzsäure, Ethanol und Ammoniumcarbonat.
Ausbeute: 79 % der Theorie,
$C_{23}H_{29}N_5O_3S$ (455.59)
$R_f$-Wert: 0.21 (Kieselgel; Dichlormethan/Ethanol = 4:1)

Massenspektrum (EKA) : $(M+H)^+ = 456$
$(M+Na)^+ = 478$

Beispiel 65

1-Methyl-2-[(4-amidinophenyl)-oxymethyl]-6-[N-(ethoxycarbonylmethyl)-benzolsulfonylamino]-benzimidazol

Hergestellt analog Beispiel 1e aus 1-Methyl-2-[(4-cyanophenyl)-oxymethyl]-6-[N-(ethoxycarbonylmethyl)-benzolsulfonylamino]-benzimidazol und ethanolischer Salzsäure, Ethanol und Ammoniumcarbonat.
Ausbeute: 45 % der Theorie,
$C_{26}H_{27}N_5O_5S$ (521.6)

Massenspektrum (EKA): $(M+H)^+ = 522$
$(M+H+Na)^{++} = 272.7$

Beispiel 66

1-Methyl-2-[N-(4-amidinophenyl)-aminomethyl]-5-(N-cyclopentylmethansulfonylamino)-benzimidazol

Hergestellt analog Beispiel le aus 1-Methyl-2-[N-(4-cyanophenyl)-aminomethyl]-5-(N-cyclopentyl-methansulfonylamino)-benzimidazol und ethanolischer Salzsäure, Ethanol und Ammoniumcarbonat.
Ausbeute: 89 % der Theorie,
$C_{22}H_{28}N_6O_2S$ (440.58)
$R_f$-Wert: 0.17 (Kieselgel; Dichlormethan/Ethanol = 4:1)

Massenspektrum (EKA): $(M+H)^+$ = 441
$(M+Na)^+$ = 463

Beispiel 67

1-Methyl-2-[(4-amidinophenyl)-oxymethyl]-5-[N-(N'-(ethoxycarbonylmethyl)-aminocarbonylmethyl)-chinolin-8-sulfonylamino]-benzimidazol

Hergestellt analog Beispiel 1e aus 1-Methyl-2-[(4-cyanophenyl)-oxymethyl]-5-[N-(N'-(ethoxycarbonylmethyl)-aminocarbonylmethyl)-chinolin-8-sulfonylamino]-benzimidazol und ethanolischer Salzsäure, Ethanol und Ammoniumcarbonat.
Ausbeute: 49 % der Theorie,
$C_{31}H_{31}N_7O_6S$ (629.7)

Massenspektrum (EKA): $(M+H)^+$ = 630
$(M+2H)^{++}$ = 315.7
$(M+H+Na)^{++}$ = 326.7

Beispiel 68

1-Methyl-2-[(4-amidinophenyl)-oxymethyl]-5-[N-(N'-(hydroxycarbonylmethyl)-aminocarbonylmethyl)-chinolin-8-sulfonylamino]-benzimidazol

Hergestellt analog Beispiel 3 aus 1-Methyl-2-[(4-amidinophenyl)-oxymethyl]-5-[N-(N'-(ethoxycarbonylmethyl)-aminocarbonylmethyl)-chinolin-8-sulfonylamino]-benzimidazol und Natronlauge.
Ausbeute: 79 % der Theorie,
$C_{29}H_{27}N_7O_6S$ (601,7)

Massenspektrum (EKA): $(M+H)^+$ = 602
$(M+Na)^+$ = 624
$(M+2H)^{++}$ = 301.7
$(M+H+Na)^{++}$ = 312.7

Beispiel 69

1-Methyl-2-[(4-amidinophenyl)-oxymethyl]-5-[N-(2-ethoxycarbonylethyl)-chinolin-8-sulfonylamino]-benzimidazol

Hergestellt analog Beispiel le aus 1-Methyl-2-[(4-cyanophenyl)-oxymethyl]-5-[N-(2-ethoxycarbonylethyl)-chinolin-8-sulfonylamino]-benzimidazol und ethanolischer Salzsäure, Ethanol und Ammoniumcarbonat.
Ausbeute: 62 % der Theorie,
$C_{30}H_{30}N_6O_5S$ (586,7)

Massenspektrum (EKA): $(M+H)^+$ = 587
$(M+2H)^{++}$ = 294
$(M+H+Na)^{++}$ = 305

Beispiel 70

2-[N-(4-Amidinophenyl)-aminomethyl]-3-methyl-6-[N-(ethoxycarbonylmethyl)-1,2,3,4-tetrahydro-chinolin-8-sulfonylamino]-benzofuran

Hergestellt analog Beispiel le aus 2-[N-(4-Cyanophenyl)-aminomethyl]-3-methyl-6-[N-(ethoxycarbonylmethyl)-1,2,3,4-tetrahydro-chinolin-8-sulfonylamino]-benzofuran (hergestellt analog Beispiel 107) und ethanolischer Salzsäure, Ethanol und Ammoniumcarbonat.
Ausbeute: 55 % der Theorie,
$C_{30}H_{33}N_5O_5S$ (575.70)
$R_f$-Wert: 0.25 (Kieselgel; Dichlormethan/Ethanol = 4:1)

Massenspektrum (EKA): $(M+H)^+ = 576$
$(M+H+Na)^{++} = 299.7$

Beispiel 71

2-[N-(4-Amidinophenyl)-aminomethyl]-3-methyl-6-[N-(hydroxycarbonylmethyl)-1,2,3,4-tetrahydro-chinolin-8-sulfonylamino]-benzofuran

Hergestellt analog Beispiel 3 aus 2-[N-(4-Amidinophenyl)-aminomethyl]-3-methyl-6-[N-(ethoxycarbonylmethyl)-1,2,3,4-tetrahydro-chinolin-8-sulfonylamino]-benzofuran und Natronlauge.
Ausbeute: 94 % der Theorie,
$C_{28}H_{29}N_5O_5S$ (547.65)

Massenspektrum (EKA): $(M+H)^+ = 548$
$(M+Na)^+ = 570$
$(M+2Na)^{++} = 296.7$

Beispiel 72

2-[2-(4-Amidinophenyl)-ethyl]-4-methyl-7-[N-(ethoxycarbonylmethyl)-chinolin-8-sulfonylamino]-chinolin

Hergestellt analog Beispiel le aus 2-[2-(4-Cyanophenyl)-ethyl]-4-methyl-7-[N-(ethoxycarbonylmethyl)-chinolin-8-sulfonylamino]-chinolin (hergestellt analog Beispiel 102) und ethanolischer Salzsäure, Ethanol und Ammoniumcarbonat.
Ausbeute: 50 % der Theorie,
$C_{32}H_{31}N_5O_4S$ (581.6)
$R_f$-Wert: 0.18 (Kieselgel; Dichlormethan/Ethanol = 4:1)

Massenspektrum (EKA): $(M+H)^+ = 582$
$(M+2H)^{++} = 291.7$
$(M+H+Na)^{++} = 302.7$

Beispiel 73

2-[2-(4-Amidinophenyl)-ethyl]-4-methyl-7-[N-(hydroxycarbonylmethyl)-chinolin-8-sulfonylamino]-chinolin

Hergestellt analog Beispiel 3 aus 2-[2-(4-Amidinophenyl)-ethyl]-4-methyl-7-[N-(ethoxycarbonylmethyl)-chinolin-8-sulfonylamino]-chinolin und Natronlauge.
Ausbeute: 38 % der Theorie,
$C_{30}H_{27}N_5O_4S$ (553.60)

Massenspektrum (EKA): $(M+H)^+ = 554$
$(M+Na)^+ = 576$
$(M+2H)^{++} = 277.7$

Beispiel 74

1-Methyl-2-[2-(4-amidinophenyl)-ethyl]-5-(N-chinolin-8-sulfonylamino)-indol-hydrochlorid

a. (E)-4-[2-(1-Methyl-indol-2-yl)ethenyl]benzonitril

3.25 g (ca. 67 mMol) einer 50%igen Natriumhydrid-Suspension in Mineralöl wird in 70 ml Dimethylsulfoxid 45 Minuten auf 80°C erhitzt. Nach Abkühlen auf Raumtemperatur werden weitere 140 ml Dimethylsulfoxid und portionsweise 18.2 g (44 mMol) 4-Cyanbenzyltriphenylphosphoniumbromid zugegeben und 90 Minuten bei Raumtemperatur gerührt. Anschließend tropft man 7.0 g (44 mMol) 1-Methyl-indol-2-yl-carbaldehyd (J. Org. Chem. 52, 104 (1987)) in 70 ml Dimethylsulfoxid zu und läßt 30 Minuten bei Raumtemperatur, 20 Minuten bei 40°C und 16 Stunden wiederum bei Raumtemperatur rühren. Das Rohprodukt wird mit 200 ml Essigester verdünnt, mit 400 ml 14%iger Natriumchlorid-Lösung gewaschen und die Wasserphase wird mit 2 x 300 ml Essigester extrahiert. Die vereinigten organischen Phasen werden mit Natriumsulfat getrocknet, das Lösungsmittel wird im Vakuum abdestilliert, und das Rohprodukt wird mittels Flash-Chromatographie (Kieselgel, Petrolether/Essigester = 9:1) gereinigt.
Ausbeute: 2.4 g (21 % der Theorie),
$R_f$-Wert: 0.52 (Kieselgel; Essigester/Petrolether = 3:7)

b. 4-[2-(1-Methyl-indol-2-yl)ethyl]benzonitril

2.3 g (8.9 mMol) (E)-4-[2-(1-Methyl-indol-2-yl)ethenyl]benzonitril werden in 150 ml Methanol und 50 ml Methylenchlorid gelöst und mit 0.20 g 10%igem Palladium auf Kohle bei 3 bar Wasserstoffdruck hydriert. Das Lösungsmittel wird im Vakuum abdestilliert, der erhaltene weiße Rückstand wird mit wenig Diethylether und Aceton gewaschen.
Ausbeute: 1.8 g (78% der Theorie),
$R_f$-Wert: 0.50 (Kieselgel; Essigester/Petrolether = 3:7)

c. 4-[2-(1-Methyl-5-nitro-indol-2-yl)ethyl]benzonitril

Innerhalb von 2 Stunden wird 1.7 g (6.53 mMol) 4-[2-(1-Methylindol-2-yl)ethyl]benzonitril in 20 ml konz. Schwefelsäure bei 15°C gelöst und dann auf 2°C abgekühlt. Anschließend werden 0.66 g (6.53 mMol) Kaliumnitrat portionsweise zugegeben (Temperaturanstieg auf ca. 10°C). Es wird 30 Minuten bei 2-5°C nachgerührt und dann auf Eis gegossen. Der ausgefallene gelbliche Niederschlag wird abfiltriert und mit Wasser gewaschen.
Ausbeute: 2.0 g (100 % der Theorie),
$R_f$-Wert: 0.24 (Kieselgel; Essigester/Petrolether = 3:7)

d. 4-[2-(1-Methyl-5-amino-indo]-2-yl)ethyl]benzonitril

2.0 g (6.55 mMol) 4-[2-(1-Methyl-5-nitro-indol-2-yl)ethyl]-benzonitril werden in 200 ml Methanol und 200 ml Methylenchlorid gelöst und mit 0.20 g 10%igem Palladium auf Kohle bei 3 bar Wasserstoffdruck hydriert. Anschließend wird das Lösungsmittel im Vakuum abdestilliert, und der Rückstand mit wenig Methanol gewaschen.
Ausbeute: 1.67 g (93 % der Theorie) beigegelber amorpher Feststoff,
$R_f$-Wert: 0.38 (Kieselgel; Methylenchlorid/Ethanol = 19:1)

e 1-Methyl-2-[2-(4-cyanophenyl)-ethyl]-5-(N-chinolin-8-sulfonylamino)-indol

Eine Lösung von 1.57 g (5.7 mMol) 4-[2-(1-Methyl-5-amino-indol-2-yl)-ethyl]benzonitril und 1.42 g (6.2 mMol) Chinolin-8-sulfonsäurechlorid in 30 ml Pyridin wird 1 Stunden bei Raumtemperatur gerührt. Anschließend wird das Lösungsmittel im Vakuum entfernt, der Rückstand in 50 ml Methylenchlorid aufgenommen, mit 50 ml gesättigter Natriumhydrogencarbonat-Lösung gewaschen, mit Natriumsulfat getrocknet und mittels Flash-Chromatographie (Kieselgel, Methylenchlorid/Ethanol = 99:1) gereinigt.
Ausbeute: 0.77 g (49 % der Theorie),
$R_f$-wert: 0.39 (Kieselgel; Methylenchlorid/Ethanol = 50:1)

f. 1-Methyl-2-[2-(4-amidinophenyl)ethyl]-5-(N-chinolin-8-sulfonylamino)-indol-hydrochlorid

Hergestellt analog Beispiel le aus 1-Methyl-2-[2-(4-cyanophenyl)ethyl]-5-(N-chinolin-8-sulfonylamino)-indol und ethanolischer Salzsäure, Ethanol und Ammoniumcarbonat.
Ausbeute: 39 % der Theorie,
$C_{27}H_{25}N_5O_2S$ (483.6)
Rf-Wert: 0.29 (Kieselgel; Methylenchlorid/Ethanol = 4:1 + einige Tropfen Essigsäure)
Massenspektrum (SKA): $(M+H)^+$ = 484

Beispiel 75

1-Methyl-2-[2-(4-amidinophenyl)-ethyl]-5-[N-(ethoxycarbonylmethyl)-chinolin-8-sulfonylamino]-indol

Hergestellt analog Beispiel le aus 1-Methyl-2-[2-(4-cyanophenyl)-ethyl]-5-[N-(ethoxycarbonylmethyl)-chinolin-8-sulfonylamino]-indol und ethanolischer Salzsäure, Ethanol und Ammoniumcarbonat.
Ausbeute: 53 % der Theorie,
$C_{31}H_{31}N_5O_4S$ (569.69)
$R_f$-Wert: 0.19 (Kieselgel; Dichlormethan/Ethanol = 4:1)

Massenspektrum (EKA): $(M+H)^+ = 570$
$(M+2H)^{++} = 285.7$
$(M+H+Na)^{++} = 296.6$

Beispiel 76

1-Methyl-2-[2-(4-amidinophenyl)-ethyl]-5-[N-(hydroxycarbonylmethyl)-chinolin-8-sulfonylamino]-indol

Hergestellt analog Beispiel 3 aus 1-Methyl-2-[2-(4-amidinophenyl)-ethyl]-5-[N-(ethoxycarbonylmethyl)-chinolin-8-sulfonylamino]-indol und Natronlauge.
Ausbeute: 96 % der Theorie,
$C_{29}H_{27}N_5O_4S$ (541.63)

Massenspektrum (EKA): $(M+H)^+ = 542$
$(M+Na)^+ = 564$
$(M+2H)^{++} = 271.7$
(M-H)- = 540

Beispiel 77

1-Methyl-2-(4-amidinobenzylamino)-5-(chinolin-8-sulfonylamino)-benzimidazol

Hergestellt analog Beispiel le aus 1-Methyl-2-(4-cyanobenzylamino)-5-(chinolin-8-sulfonylamino)-benzimidazol und ethanolischer Salzsäure, Ethanol und Ammoniumcarbonat.
Ausbeute: 85 % der Theorie,
$C_{25}H_{23}N_7O_2S$ (485,57)
Rf-Wert: 0.40 (Kieselgel; Essigester/Ethanol/Ammoniak = 50:45:5)

Massenspektrum (EKA): $(M+H)^+ = 486$
$(M+H+Na)^{++} = 254{,}7$

Beispiel 78

1-Methyl-2-(4-amidinobenzylthio)-5-(chinolin-8-sulfonylamino)-benzimidazol

Hergestellt analog Beispiel le aus 1-Methyl-2-(4-cyanobenzylthio)-5-(chinolin-8-sulfonylamino)-benzimidazol und ethanolischer Salzsäure, Ethanol und Ammoniumcarbonat.
Ausbeute: 69 % der Theorie,
$C_{25}H_{22}N_6O_2S_2$ (502,62)
$R_f$-Wert: 0.45 (Kieselgel; Essigester/Ethanol/Ammoniak = 50:45:5)

Massenspektrum (EKA): $(M+H)^+ = 503$
$(M+Na)^+ = 525$

Beispiel 79

2-[(4-Amidinophenyl)methylthio]-5-(N-chinolin-8-sulfonylamino)-benzothiazol-hydrochlorid

a. 2-[(4-Cyanophenyl)methylthio]-6-nitro-benzothiazol

Zu einer Lösung aus 1.5 g (7.06 mMol) 2-Mercapto-6-nitrobenzothiazol wird portionsweise 0.37 g (7.7 mMol) Natriumhydrid (50%ig in Mineralöl) zugegeben und anschließend 30 Minuten bei 50°C gerührt. Danach wird 1.45 g (7.4 mMol) 4-Brommethylbenzonitril zugetropft und eine weitere Stunde bei 50°C gerührt. Die Reaktionsmischung wird mit 30 ml Essigester und 70 ml 14%iger Natriumchloridlösung versetzt, woraufhin ein Großteil der Titelverbindung als beiger Niederschlag ausfällt. Die organische Phase wird im Vakuum konzentriert, das ausgefallene Rohprodukt wird mit Diethylether verrieben und die flüssige Phase abgetrennt. Der erhaltene Feststoff wird mit dem beigen Präzipitat vereinigt.
Ausbeute: 1.3 g (56 % der Theorie),
$R_f$-Wert: 0.52 (Kieselgel; Essigester/Petrolether = 3:7)

b. 2-[(4-Cyanophenyl)methylthio]-6-amino-benzothiazol

Eine Suspension von 1.0 g (3.05 mMol) 2-[(4-Cyanophenyl)-methylthio]-6-nitro-benzothiazol wird in 60 ml Eisessig solange zum Sieden erhitzt, bis eine klare Lösung entsteht. Anschließend gibt man in zwei Portionen 2.0 g (36 mMol) Eisenpulver zu und kocht 5 Minuten am Rückfluß. Es wird filtriert, und das Filtrat wird im Vakuum konzentriert. Das Rohprodukt wird durch Zugabe von konz. Ammoniak alkalisch gestellt und durch Flash-Chromatographie (Kieselgel, Essigester/Petrolether = 20:80 bis 35:65) gereinigt.
Ausbeute: 0.22 g (24 % der Theorie) beige-farbigen amorphen Feststoff,
$R_f$-Wert: 0.44 (Kieselgel; Essigester/Petrolether = 4:6)

c. 2-[(4-Cyanophenyl)methylthio]-6-(N-chinolin-8-sulfonylamino)-benzthiazol

Ein Gemisch aus 2.3 g (7.74 mMol) 2-[(4-Cyanophenyl)methylthio]-6-amino-benzothiazol und 1.85 g (8.1 mMol) Chinolin-8-sulfonsäurechlorid wird in 30 ml Pyridin 2 Stunden bei Raumtemperatur gerührt. Anschließend wird das Lösungsmittel im Vakuum abdestilliert und das Rohprodukt durch Flash-Chromatographie (Kieselgel, Methylenchlorid/Ethanol = 99:1) gereinigt. Ausbeute: 3.15 g (83 % der Theorie),
Schmelzpunkt: 106-108°C
$R_f$-Wert: 0.33 (Kieselgel; Essigester/Petrolether = 4:6)

d. 2-[(4-Amidinophenyl)methylthio]-6-(N-chinolin-8-sulfonylamino)-benzothiazol-hydrochlorid

Hergestellt analog Beispiel le aus 2-[(4-Cyanophenyl)methylthio]-6-(N-chinolin-8-sulfonylamino)-benzthiazol und ethanolischer Salzsäure, Ethanol und Ammoniumcarbonat.
Ausbeute: 91 % der Theorie,
$C_{24}H_{19}N_5O_2S_3$ (505.64)
$R_f$-Wert: 0.34 (Kieselgel; Methylenchlorid/Ethanol = 4:1 + einige Tropfen Essigsäure)

Massenspektrum (SKA): $(M+H)^+ = 506$

Beispiel 80

Gemisch aus 2-[2-(4-Amidinophenyl)-ethyl]-1-methyl-6-(chinolin-8-sulfonylamino)-imidazo[4,5-b]pyridin-hydrochlorid und 2-[2-(4-Amidinophenyl)-ethyl]-3-methyl-6-(chinolin-8-sulfonylamino)-imidazo[4,5-b]pyridin-hydrochlorid

a. 3-(4-Cyanophenyl)-N-(3,5-dinitro-pyrid-2-yl)-propionsäureamid

Eine Lösung aus 1.8 g (10 mMol) 2-Amino-3,5-dinitro-pyridin, 2.4 g (12 mMol) 3-(4-Cyanophenyl)propionylchlorid, 2.0 ml Triethylamin und 0.1 g Dimethylamin in 35 ml Chlorbenzol wird 3 Stunden auf 150°C erhitzt. Anschließend wird das Lösungsmittel im Vakuum abdestilliert, und der Rückstand wird in 50 ml Essigester aufgenommen. Es wird mit 50 ml Wasser und 50 ml gesättigter Natriumchloridlösung gewaschen, mit Natriumsulfat getrocknet, und nach Abdestillieren des Lösungsmittels mittels Flash-Chromatographie (Kieselgel, Methylenchlorid) gereinigt.
Ausbeute: 2.2 g (65 % der Theorie),

$R_f$-Wert: 0.67 (Kieselgel; Methylethylketon/Xylol = 1:1)

b) 2-[2-(4-Cyanophenyl)ethyl]-6-phtalimido-imidazo[4,5-b]pyridin

Eine Suspension aus 2.1 g (6.15 mMol) 3-(4-Cyanophenyl)-N-(3,5-dinitro-pyrid-2-yl)-propionsäureamid und 0.50 g 10%igem Palladium auf Kohle in 50 ml Eisessig wird bei 80°C bei 3 bar Wasserstoffdruck zur Reaktion gebracht. Nach dem Abkühlen wird der Katalysator abfiltriert, 1.1 g (7.4 mMol) Phthalsäureanhydrid zugesetzt und für 1 Stunden zum Sieden erhitzt. Das Lösungsmittel wird im Vakuum abdestilliert, und das Rohprodukt wird in 50 ml Methylenchlorid aufgenommen, 2x mit gesättigter Natriumhydrogencarbonatlösung gewaschen und durch Flash-Chromatographie (Kieselgel; Methylenchlorid/Ethanol = 40:1 bis 19:1) gereinigt.
Ausbeute: 0.95 g (41 % der Theorie),
$R_f$-Wert: 0.50 (Kieselgel; Essigester/Ethanol/Ammoniak = 90:10:1)

c. Isomerengemisch aus 2-[2-(4-Cyanophenyl)-ethyl]-1-methyl-6-phtalimido-imidazo[4,5-b]pyridin und

2-[2-(4-Cyanophenyl)-ethyl]-3-methyl-6-phtalimido-imidazo-[4,5-b]pyridin

Ein Gemisch aus 0.80 g (2.0 mMol) 2-[2-(4-Cyanophenyl)-ethyl]-6-phtalimido-imidazo[4,5-b]pyridin, 0.25 g (2.2 mMol) Kaliumtert.butylat und 0.32 g (2.2 mMol) Methyliodid wird in 10 ml Dimethylsulfoxid 1 Stunden bei Raumtemperatur gerührt. Anschließend wird auf Eiswasser gegossen, mit 100 ml Essigester extrahiert, mit Natriumsulfat getrocknet, und das Lösungsmittel im Vakuum abdestilliert.
Ausbeute: 0.80 g (98 % der Theorie),
$R_f$-Wert: 0.56 (Kieselgel; Essigester/Ethanol/Ammoniak = 90:10:1)

d. Isomerengemisch aus 2-[2-(4-Cyanophenyl)-ethyl]-1-methyl-6-imidazo[4,5-b]pyridin und

2-[2-(4-Cyanophenyl)-ethyl]-3-methyl-6-imidazo[4,5-b]pyridin

0.80 g (2.0 mMol) des Isomerengemisch aus 2-[2-(4-Cyanophenyl)-ethyl]-1-methyl-6-phtalimido-imidazo [4,5-b]pyridin und aus 2-[2-(4-Cyanophenyl)-ethyl]-3-methyl-6-phtalimido-imidazo[4,5-b]pyridin werden in 5 ml 40%iger wäßriger MethylaminLösung und 20 ml Ethanol 1 Stunden bei 40-60°C gerührt. Anschließend wird das Lösungsmittel im Vakuum abdestilliert, das Rohprodukt in 40 ml Essigester/Ethanol (9:1) aufgenommen und sukzessiv mit Wasser und gesättigter Natriumchlorid-Lösung gewaschen. Nach dem Entfernen des Lösungsmittels im Vakuum wird der Rückstand in Pyridin aufgenommen und analog Beispiel 79c mit 0.41 g (1.8 mMol) Chinolin-8-sulfonsäurechlorid umgesetzt, aufgearbeitet und durch Flash-Chromatographie (Kieselgel, Methylenchlorid bis Methylenchlorid/Ethanol = 19:1) gereinigt.
Ausbeute: 0.50 g (54 % der Theorie),
$R_f$-Wert: 0.63 + 0.50 (Kieselgel; Essigester/Ethanol/Ammoniak = 90:10:1)

e. Isomerengemisch aus 2-[2-(4-Amidinophenyl)-ethyl]-1-methyl-6-(chinolin-8-sulfonylamino)-imidazo[4,5-b]pyridin-hydrochlorid und

2-[2-(4-Amidinophenyl)-ethyl]-3-methyl-6-(chinolin-8-sulfonylamino)-imidazo[4,5-b]pyridin-hydrochlorid

Hergestellt analog Beispiel le aus dem Isomerengemisch aus 2-[2-(4-Cyanophenyl)-ethyl]-1-methyl-6-(chinolin-8-sulfonylamino)-imidazo[4,5-b]pyridin und 2-[2-(4-Cyanophenyl)-ethyl]-3-methyl-6-(chinolin-8-sulfonylamino)-imidazo[4,5-b]pyridin und methanolischer Salzsäure, Methanol und Ammoniumcarbonat. Ausbeute: 80 % der Theorie,
$C_{25}H_{23}N_7O_2S$ (485.57)
$R_f$-Wert: 0.25 + 0.21 (Kieselgel; Essigester/Ethanol/Ammoniak = 50:45:5)

Massenspektrum (SKA): $(M+H)^+ = 486$

Beispiel 81

2-(4-Amidinobenzylthio)-6-[N-(methoxycarbonylmethyl)-chinolin-8-sulfonylamino]-benzthiazol

Hergestellt analog Beispiel le aus 2-(4-Cyanobenzylthio)-6-[N-(methoxycarbonylmethyl)-chinolin-8-sulfonylamino]-benzthiazol und ethanolischer Salzsäure, Ethanol und Ammoniumcarbonat.

Ausbeute: 28 % der Theorie,
$C_{27}H_{23}N_5O_4S_3$ (577.41)
$R_f$-Wert: 0.21 (Kieselgel; Dichlormethan/Ethanol = 4:1)

Massenspektrum (EKA): $(M+H)^+ = 578$
$(M+H+Na)^{++} = 300.7$

Beispiel 82

2-[(4-Amidinophenyl)oxymethyl]-5-[N-(methoxycarbonylmethyl)-chinolin-8-sulfonylamino]-benzothiazol-hydrochlorid

a. (4-Cyanophenyl)oxy-N-(5-nitro-2-mercaptophenyl)-essigsäureamid

Eine Lösung aus 1.05 g (6.5 mMol) Carbonyldiimidazolid und 1.15 g (6.5 mMol) (4-Cyanophenyl)oxyessigsäure in 10 ml Tetrahydrofuran wird 30 Minuten auf 50°C erwärmt. Anschließend versetzt man mit 1.0 g (5.9 mMol) 2-Mercapto-5-nitroanilin und erhitzt weitere 3 Stunden auf 50°C. Man filtriert, konzentriert das Filtrat im Vakuum und reinigt das Rohprodukt durch Flash-Chromatographie (Kieselgel, Methylenchlorid/Ethanol = 19:1 bis 4:1).
Ausbeute: 1.05 g (54 % der Theorie),
Schmelpunkt: 274-276°C
$R_f$-Wert: 0.54 (Kieselgel; Methylenchlorid/Ethanol = 19:1 + einige Tropfen konz. Ammoniak)

b. 2-[(4-Cyanophenyl)oxymethyl]-5-nitrobenzothiazol

Man erhitzt eine Lösung aus 2.1 g (6.4 mMol) (4-Cyanophenyl)-oxy-N-(5-nitro-2-mercaptophenyl)-essigsäureamid in 20 ml Eisessig 1 Stunden auf 80°C, verdünnt mit Eiswasser und filtriert das ausgefallene Rohprodukt ab. Nach Flash-Chromatographie (Kieselgel, Methylenchlorid:Ethanol = 99:1) erhält man einen beigen amorphen Feststoff.
Ausbeute: 0.77 g (37 % der Theorie),
$R_f$-Wert: 0.56 (Kieselgel; Methylenchlorid/Ethanol = 50:1)

c. 2-[(4-Cyanophenyl)oxymethyl]-5-amino-benzothiazol

Eine Lösung aus 0.62 g (2.0 mMol) 2-[(4-Cyanophenyl)oxymethyl]-5-nitro-benzothiazol in 20 ml Pyridin wird sukzessiv mit 1.0 g (5.7 mMol) Natriumdithionit und 4 ml Wasser versetzt und 2 Stunden bei 95°C gerührt. Anschließend wird das Lösungsmittel im Vakuum abdestilliert und der Rückstand mit Eiswasser verdünnt. Es wird filtriert und der Filterrückstand mehrmals mit wenig kaltem Wasser gewaschen.
Ausbeute: 0.44 g (79 % der Theorie),
$R_f$-Wert: 0.37 (Kieselgel; Methylenchlorid/Ethanol = 50:1)

d. 2-[(4-Cyanophenyl)oxymethyl]-5-(chinolin-8-sulfonylamino)-benzothiazol

Hergestellt analog Beispiel 79c aus 0.40 g (1.42 mMol) 2-[(4-Cyanophenyl)oxymethyl]-5-amino-benzothiazol und 0.34 g (1.5 mMol) Chinolin-8-sulfonsäurechlorid. Die weitere Reinigung erfolgt durch Flash-Chromatographie (Kieselgel, Methylenchlorid/Ethanol = 99:1).
Ausbeute: 0.41 g (61 % der Theorie),
$R_f$-wert: 0.49 (Kieselgel; Methylenchlorid/Ethanol = 50:1)

e. 2-[(4-Cyanophenyl)oxymethyl]-5-[N-[ethoxycarbonylmethyl)-chinolin-8-sulfonylamino]-benzothiazol

Eine Suspension aus 0.39 g (0.83 mMol) 2-[(4-Cyanophenyl)oxymethyl]-5-(chinolin-8-sulfonylamino)-benzothiazol, 0.23 ml (0.35 g, 2.1 mMol) Bromessigsäureethylester, 0.14 ml (0.14 g, 0.94 mMol) 1,8-Diazabicyclo[5,4,0]undec-7-en und 0.60 g (4.1 mMol) Kaliumcarbonat in 30 ml Aceton wird 3 Stunden zum Sieden erhitzt. Anschließend wird filtriert, das Lösungsmittel im Vakuum abdestilliert und der Rückstand durch Flash-Chromatographie (Kieselgel, Methylenchlorid:Ethanol = 99:1) gereinigt.
Ausbeute: 0.41 g (61 % der Theorie),
$R_f$-Wert: 0.54 (Kieselgel; Methylenchlorid/Ethanol = 50:1 + einige Tropfen Ammoniak)

f. 2-[(4-Amidinophenyl)oxymethyl]-5-[N-(methoxycarbonylmethyl)-chinolin-8-sulfonylamino]-benzothiazol-hydrochlorid

Hergestellt analog Beispiel le aus 2-[(4-Cyanophenyl)oxymethyl)-5-[N-[ethoxycarbonylmethyl)-chinolin-8-sulfonylamino]-benzothiazol und methanolischer Salzsäure, Methanol und Ammoniumcarbonat.
Ausbeute: 67 % der Theorie,
$C_{27}H_{23}N_5O_5S_2$ (561.64)
$R_f$-Wert: 0.36 (Kieselgel; Methylenchlorid/Ethanol 4:1)

Massenspektrum (SKA): $(M+H)^+ = 562$
$(M+H+Na)^{++} = 292.7$

Beispiel 83

1-Methyl-2-(4-amidinobenzylthio)-5-[N-(methoxycarbonylmethyl)-chinolin-8-sulfonylamino]-benzimidazol

Hergestellt analog Beispiel le aus 1-Methyl-2-(4-cyanobenzylthio)-5-[N-(methoxycarbonylmethyl)-chinolin-8-sulfonylamino]-benzimidazol und methanolischer Salzsäure, Methanol und Ammoniumcarbonat.
Ausbeute: 50 % der Theorie,
$C_{28}H_{26}N_6O_4S_2$ (574,69)
$R_f$-Wert: 0.35 (Kieselgel; Essigester/Ethanol/Ammoniak = 50:45:5)

Massenspektrum (EKA): $(M+H)^+ = 575$
$(M+H+Na)^{++} = 299$

Beispiel 84

1-Methyl-2-[4-(N-ethoxycarbonyl-amidino)-benzylthio]-5-[N-(methoxycarbonylmethyl)-chinolin-8-sulfonylamino]-benzimidazol

Hergestellt analog Beispiel 31 aus 1-Methyl-2-(4-amidinobenzylthio)-5-[N-(methoxycarbonylmethyl)-chinolin-8-sulfonylamino]-benzimidazol und Chlorameisensäureethylester.
Ausbeute: 62 % der Theorie,
$C_{31}H_{30}N_6O_6S_2$ (646,75)
$R_f$-Wert: 0.55 (Kieselgel; Essigester/Ethanol/Ammoniak = 50:45:5)

Massenspektrum (EKA): $(M+H)^+ = 647$
$(M+Na)^+ = 669$

Beispiel 85

1-Methyl-2-(4-amidinobenzylthio)-5-[N-(hydroxycarbonylmethyl)-chinolin-8-sulfonylamino]-benzimidazol

Hergestellt analog Beispiel 3 aus 1-Methyl-2-(4-amidinobenzylthio)-5-[N-(methoxycarbonylmethyl)-chinolin-8-sulfonylamino]-benzimidazol und Natronlauge.
Ausbeute: 61,5 % der Theorie,
$C_{27}H_{24}N_6O_4S$ (560,66)
$R_f$-Wert: 0.20 (Kieselgel; Essigester:Ethanol:Ammoniak = 50:45:5)

Massenspektrum (EKA): $(M+H)^+ = 561$
$(M+Na)^+ = 583$

Beispiel 86

2-[(4-Amidinophenyl)-oxymethyl]-5-[N-(hydroxycarbonylmethyl)-chinolin-8-sulfonylamino]-benzthiazol

Hergestellt analog Beispiel 3 aus 2-[(4-Amidinophenyl)-oxymethyl]-5-[N-(methoxycarbonylmethyl)-chinolin-8-sulfonylamino]-benzthiazol und Natronlauge.

Ausbeute: 93 % der Theorie,
$C_{26}H_{21}N_5O_5S_2$ (547.62)
$R_f$-Wert: 0.13 (Kieselgel; Dichlormethan/Ethanol = 4:1)

Massenspektrum (EKA): $(M+H)^+$ = 548
$(M+2H)^{++}$ = 274.6
$(M+H+Na)^{++}$ = 285.6
$(M+2Na)^{++}$ = 296.6

Beispiel 87

1-Methyl-2-(4-amidinobenzylthio)-5-benzoylamino-benzimidazol

Hergestellt analog Beispiel le aus 1-Methyl-2-(4-cyanobenzylthio)-5-benzoylaminobenzimidazol und ethanolischer Salzsäure, Ethanol und Ammoniumcarbonat.
Ausbeute: 54,8 % der Theorie,
$C_{23}H_{21}N_5OS$ (415,52)
$R_f$-Wert: 0.35 (Kieselgel; Essigester/Ethanol/Ammoniak = 50:45:5)

Massenspektrum (EKA): $(M+H)^+$ = 416

Beispiel 88

2-[N-(4-Amidinophenyl)-aminomethyl]-5-[N-(ethoxycarbonylmethyl)chinolin-8-sulfonylamino]-benzthiazol

Hergestellt analog Beispiel le aus 2-[N-(4-Cyanophenyl)-aminomethyl]-5-[N-(ethoxycarbonylmethyl)-chinolin-8-sulfonylamino]-benzthiazol und ethanolischer Salzsäure, Ethanol und Ammoniumcarbonat.
Ausbeute: 80 % der Theorie,
$C_{28}H_{26}N_6O_4S_2$ (574.69)
$R_f$-Wert: 0.24 (Kieselgel; Dichlormethan/Ethanol = 4:1)

Massenspektrum (EKA): $(M+H)^+$ = 575
$(M+H+Na)^{++}$ = 299

Beispiel 89

2-[N-(4-Amidinophenyl)-aminomethyl]-5-[N-(hydroxycarbonylmethyl)-chinolin-8-sulfonylamino]-benzthiazol

Hergestellt analog Beispiel 3 aus 2-[N-(4-Amidinophenyl)-aminomethyl]-5-[N-(ethoxycarbonylmethyl)-chinolin-8-sulfonylamino]-benzthiazol und Natronlauge.
Ausbeute: 94 % der Theorie,
$C_{26}H_{22}N_6O_4S_2$ (546.63)
$R_f$-Wert: 0.15 (Kieselgel; Dichlormethan/Ethanol = 4:1)

Massenspektrum (EKA): $(M+H)^+$ = 547
$(M+Na)^+$ = 569
$(M+2H)^{++}$ = 274
$(M+H+Na)^{++}$ = 285
$(M+2Na)^{++}$ = 296
$(2M+3Na)^{3+}$ = 387

Beispiel 90

1-Methyl-2-(4-amidinobenzylthio)-5-[N-(ethoxycarbonylmethyl)-benzoylamino]-benzimidazol

Hergestellt analog Beispiel le aus 1-Methyl-2-(4-cyanobenzylthio)-5-[N-(ethoxycarbonylmethyl)-benzoylamino]-benzimidazol und ethanolischer Salzsäure, Ethanol und Ammoniumcarbonat.
Ausbeute: 68,8 % der Theorie,

$C_{27}H_{27}N_5O_3S$ (538,08)
$R_f$-Wert: 0.27 (Kieselgel; Essigester/Ethanol/Ammoniak = 50:45:5)

Massenspektrum (EKA): $(M+H)^+ = 502$
$(M+H+Na)^{++} = 262,8$

Beispiel 91

1-Methyl-2-(4-amidinobenzylthio)-5-[N-(hydroxycarbonylmethyl)-benzoylamino]-benzimidazol

Hergestellt analog Beispiel 3 aus 1-Methyl-2-(4-amidinobenzylthio)-5-[N-(ethoxycarbonylmethyl)-benzoylamino]-benzimidazol und Natronlauge.
Ausbeute: 79 % der Theorie,
$C_{25}H_{23}N_5O_3S$ (473.53)
$R_f$-Wert: 0.21 (Kieselgel; Essigester/Ethanol/Ammoniak = 50:45:5)

Massenspektrum (EKA) : $(M+H)^+ = 474$
$(M+Na)^+ = 496$

Beispiel 92

2- [2-(4-Amidinophenyl)-ethyl]-3-methyl-6-[N-(ethoxycarbonylmethyl)-chinolin-8-sulfonylamino]-imidazo[4,5-b]pyridin

a. 3,5-Di-[3-(4-cyanophenyl)propionylamido]-2-methylaminopyridin

Eine Lösung von 3.8 g (19 mMol) 3,5-Dinitro-2-methylaminopyridin wird in 90 ml Ethanol/Methylenchlorid (2:1) bei 5 bar Wasserstoffdruck mit 1.0 g 10%igem Palladium auf Kohle innerhalb von 2 Stunden hydriert. Der Katalysator wird abfiltriert, und das Lösungsmittel wird im Vakuum abdestilliert. Das schwarze, ölige Rohprodukt wird in 50 ml Pyridin gelöst und bei 0°C mit 7.0 g (36 mMol) 3-(4-Cyanophenyl)propionsäurechlorid versetzt. Nach 2 Stunden wird das Lösungsmittel im Vakuum abdestilliert, der Rückstand in 100 ml Essigester aufgenommen und mit Wasser und gesättigter Natriumchlorid-Lösung gewaschen. Es wird mit Natriumsulfat getrocknet, das Lösungsmittel abdestilliert und der erhaltene Rückstand durch Flash-Chromatographie (Kieselgel, Methylenchlorid/Ethanol = 49:1 bis 19:1) gereinigt.
Ausbeute: 4.8 g (59 % der Theorie),
$R_f$-Wert: 0.40 (Kieselgel; Essigester/Ethanol/Ammoniak = 90:10:1)

b. 3-Methyl-2-[2-(4-cyanophenyl)-ethyl]-6-[3-(4-cyanophenyl)-propionylamido]-imidazo[4,5-b]pyridin

Man erhitzt eine Lösung von 1.6 g (3.5 mMol) 3,5-Di-[3-(4-cyanophenyl)propionylamido]-2-methylamino-pyridin in 30 ml Eisessig 1 Stunden auf 100°C. Anschließend destilliert man das Lösungsmittel im Vakuum ab, nimmt den Rückstand in 80 ml Methylenchlorid auf und neutralisiert mit Natriumhydrogencarbonat-Lösung. Die organische Phase wird mit Natriumsulfat getrocknet, das Lösungsmittel im Vakuum abdestilliert und das Rohprodukt durch Flash-Chromatographie (Kieselgel, Methylenchlorid/Ethanol = 49:1 bis 19:1) gereinigt.
Ausbeute: 0.90 g (60 % der Theorie),
$R_f$-Wert: 0.46 (Kieselgel; Essigester/Ethanol/Ammoniak = 90:10:1)

c. 6-Amino-3-methyl-2-[2-(4-cyanophenyl)-ethyl]-imidazo-[4,5-b]pyridin

0.80 g (1.8 mMol) 3-Methyl-2-[2-(4-cyanophenyl)-ethyl]-6-[3-(4-Cyanophenyl)propionylamido]-imidazo[4,5-b]pyridin wird in 20 ml 0.5N Salzsäure 2 Stunden lang auf 100°C erhitzt. Nach dem Erkalten wird mit Ammoniak alkalisch gestellt und mit Essigester extrahiert. Die organische Phase wird mit gesättigter Natriumchlorid-Lösung gewaschen und das Lösungsmittel abdestilliert.
Ausbeute: 0.42 g (84 % der Theorie),
$R_f$-Wert: 0.30 (Kieselgel; Essigester/Ethanol/Ammoniak = 90:10:1)

d. 2-[2-(4-Cyanophenyl)-ethyl]-3-methyl-6-(chinolin-8-sulfonylamino)-imidazo[4,5-b]pyridin

Hergestellt analog Beispiel 79c aus 0.40 g (1.4 mMol) 6-Amino-3-methyl-2-[2-(4-cyanophenyl)-ethyl]-imidazo

[4,5-b]pyridin mit 0.39 g (1.6 mMol) Chinolin-8-sulfonsäurechlorid.
Ausbeute: 0.60 g (90 % der Theorie),
$R_f$-Wert: 0.74 (Kieselgel; Essigester/Ethanol/Ammoniak = 90:10:1)

e. 2-[2-(4-Cyanophenyl)-ethyl]-3-methyl-6-[N-(ethoxycarbonylmethyl)-chinolin-8-sulfonylamino]-imidazo[4,5-b]pyridin

Hergestellt analog Beispiel 82e aus 0.60 g (1.3 mMol) 2- [2-(4-Cyanophenyl)-ethyl]-3-methyl-6-(chinolin-8-sulfonylamino)-imidazo[4,5-b]pyridin mit 0.33 g (1.5 mMol) Bromessigsäureethylester.
Ausbeute: 0.70 g (98 % der Theorie),
$R_f$-Wert: 0.80 (Kieselgel; Essigester/Ethanol/Ammoniak = 90:10:1)

f. 2-[2-(4-Amidinophenyl)-ethyl]-3-methyl-6-[N-(ethoxycarbonylmethyl)-chinolin-8-sulfonylamino]-imidazo[4,5-b]pyridin

Hergestellt analog Beispiel le aus 2-[2-(4-Cyanophenyl)-ethyl]-3-methyl-6-[N-(ethoxycarbonylmethyl)-chinolin-8-sulfonylamino]-imidazo[4,5-b]pyridin und ethanolischer Salzsäure, Ethanol und Ammoniumcarbonat.
Ausbeute: 91 % der Theorie,
$C_{29}H_{29}N_7O_4S$ (571.66)
$R_f$-Wert: 0.22 (Kieselgel; Essigester/Ethanol/Ammoniak = 50:45:5)

Massenspektrum (SKA): $(M+H)^+$ = 572
$(M+H+Na)^{++}$ = 297.8

Beispiel 93

2-[2-(4-Amidinophenyl)-ethyl]-3-methyl-6-[N-(hydroxycarbonylmethyl)-chinolin-8-sulfonylamino]-imidazo[4,5-b]pyridin

Hergestellt analog Beispiel 3 aus 2-[2-(4-Amidinophenyl)-ethyl]-3-methyl-6-[N-(ethoxycarbonylmethyl)-chinolin-8-sulfonylamino]-imidazo[4,5-b]pyridin und Natronlauge.
Ausbeute: 77 % der Theorie,
$C_{27}H_{25}N_7O_4S$ (543.59)
$R_f$-Wert: 0.16 (Kieselgel; Essigester/Ethanol/Ammoniak = 50:45:5)

Massenspektrum (EKA): $(M+H)^+$ = 544
$(M+H+Na)^{++}$ = 283,8

Beispiel 94

1-Methyl-2-[N-(4-amidinophenyl)-aminomethyl]-5-[N-(ethoxycarbonylmethyl)-chinolin-8-sulfonylamino]-indol

Hergestellt analog Beispiel 1e aus 1-Methyl-2-[N-(4-cyanophenyl)-aminomethyl]-5-[N-(ethoxycarbonylmethyl)-chinolin-8-sulfonylamino]-indol und ethanolischer Salzsäure, Ethanol und Ammoniumcarbonat.
Ausbeute: 55 % der Theorie,
$C_{30}H_{30}N_6O_4S$ (570.68)
$R_f$-Wert: 0.22 (Kieselgel; Dichlormethan/Ethanol = 4:1)

Massenspektrum (EKA): $(M+H)^+$ = 571
$(M+2H)^{++}$ = 286
$(M+H+Na)^{++}$ = 297

Beispiel 95

1-Methyl-2-[N-(4-amidinophenyl)-aminomethyl]-5-[N-(hydroxycarbonylmethyl)-chinolin-8-sulfonylamino]-indol

Hergestellt analog Beispiel 3 aus 1-Methyl-2-[N-(4-amidinophenyl)-aminomethyl]-5-[N-(ethoxycarbonylmethyl)-chinolin-8-sulfonylamino]-indol und Natronlauge.
Ausbeute: 97 % der Theorie,

$C_{28}H_{26}N_6O_4S$ (542.62)

Massenspektrum (EKA): $(M+H)^+ = 543$
$(M+Na)^+ = 565$
$(M+2H)^{++} = 272$
$(M+H+Na)^{++} = 283$
$(M+2Na)^{++} = 294$

$^1$H-NMR ($d_6$-DMSO):
δ = 3.61 (s,3H); 4.50 (d,2H); 4.67 (s,2H); 6.20 (s,1H); 6.30 (d,1H); 6.70 (d,2H); 7.01 (d,1H); 7.29 (t,1H); 7.38 (s,1H); 7.40-7.65 (m,3H); 7.77 (dd,1H); 8.03 (d,1H); 8.20 (d,1H); 8.42 (breites s,2H); 8.55 (dd,1H); 9.20 (dd,1H) ppm

Beispiel 96

1-Methyl-2-[(4-amidinophenyl)-thiomethyl]-5-[N-(ethoxycarbonylmethyl)-chinolin-8-sulfonylamino]-benzimidazol

Hergestellt analog Beispiel le aus 1-Methyl-2-[(4-cyanophenyl)-thiomethyl]-5-[N-(ethoxycarbonylmethyl)-chinolin-8-sulfonylamino]-benzimidazol und ethanolischer Salzsäure, Ethanol und Ammoniumcarbonat.
Ausbeute: 84 % der Theorie,
$C_{29}H_{28}N_6O_4S_2$ (588,71)
$R_f$-Wert: 0.35 (Kieselgel; Essigester/Ethanol/Ammoniak = 50:45:5)

Massenspektrum (EKA): $(M+H)^+ = 589$
$(M+H+Na)^{++} = 306$

Beispiel 97

1-Methyl-2-[(4-amidinophenyl)-thiomethyl]-5-[N-(hydroxycarbonylmethyl)-chinolin-8-sulfonylamino]-benzimidazol

Hergestellt analog Beispiel 3 aus 1-Methyl-2-[(4-amidinophenyl)-thiomethyl]-5-[N-(ethoxycarbonylmethyl)-chinolin-8-sulfonylamino]-benzimidazol und Natronlauge.
Ausbeute: 76 % der Theorie,
$C_{27}H_{24}N_6O_4S$ (560,66)
$R_f$-Wert: 0.21 (Kieselgel; Essigester/Ethanol/Ammoniak = 50:45:5)

Massenspektrum (EKA): $(M+H)^+ = 561$
$(M+Na)^+ = 583$

Beispiel 98

1-Methyl-2-[(4-amidinophenyl)-thiomethyl]-5-(chinolin-8-sulfonylamino)-benzimidazol

Hergestellt analog Beispiel le aus 1-Methyl-2-[(4-cyanophenyl)-thiomethyl]-5-(chinolin-8-sulfonylamino)-benzimidazol und ethanolischer Salzsäure, Ethanol und Ammoniumcarbonat.
Ausbeute: 70 % der Theorie,
$C_{25}H_{22}N_6O_2S_2$ (502,62)
$R_f$-Wert: 0.29 (Kieselgel; Essigester/Ethanol/Ammoniak = 50:45:5)

Massenspektrum (EKA): $(M+H)^+ = 503$

Beispiel 99

2-[(4-Amidinophenyl)-acetyl]-7-(chinolin-8-sulfonylamino)-1,2,3,4-tetrahydro-isochinolin-hydrochlorid

a. 2-[(4-Cyanophenyl)-acetyl]-7-nitro-1,2,3,4-tetrahydro-isochinolin

4.0 g (22.5 mMol) 7-Nitro-1,2,3,4-tetrahydro-isochinolin werden in 100 ml Chlorbenzol gelöst, mit 4.24 g (25 mMol) 4-Cyanophenylessigsäurechlorid versetzt und 2 Stunden unter Rückfluß erhitzt. Nach Abkühlung auf Raumtemperatur wird mit 1 l Petrolether verdünnt und filtriert. Der Rückstand wird in Essigester gelöst und an Kieselgel chromatographiert, wobei anfangs mit Methylenchlorid, später mit Methylenchlorid/Ethanol (50:1 und 25:1) eluiert wird. Die gewünschten Fraktionen werden vereinigt und eingedampft.
Ausbeute: 3.80 g (53 % der Theorie),
$R_f$-Wert: 0.50 (Kieselgel; Methylenchlorid/Ethanol = 19:1)

b. 2-[(4-Cyanophenyl)-acetyl]-7-amino-1,2,3,4-tetrahydro-isochinolin

Hergestellt analog Beispiel 1b aus 2-[(4-Cyanophenyl)-acetyl]-7-nitro-1,2,3,4-tetrahydro-isochinolin und Wasserstoff/-Palladium.
Ausbeute: 27 % der Theorie,
Schmelzpunkt: 186-188°C

c. 2-[(4-Cyanophenyl)-acetyl]-7-(chinolin-8-sulfonylamino)-1,2,3,4-tetrahydro-isochinolin

Hergestellt analog Beispiel 1c aus 2-[(4-Cyanophenyl)-acetyl]-7-amino-1,2,3,4-tetrahydro-isochinolin und Chinolin-8-sulfonylchlorid.
Ausbeute: 80 % der Theorie,
$R_f$-Wert: 0.55 (Kieselgel; Methylenchlorid/Ethanol = 19:1)

d. 2-[(4-Amidinophenyl)-acetyl]-7-(chinolin-8-sulfonylamino)-1,2,3,4-tetrahydro-isochinolin-hydrochlorid

Hergestellt analog Beispiel le aus 2-[(4-Cyanophenyl)-acetyl]-7-(chinolin-8-sulfonylamino)-1,2,3,4-tetrahydro-isochinolin und Salzsäure/Ammoniumcarbonat in Ethanol.
Ausbeute: 35 % der Theorie,
Schmelzpunkt: sintert ab 173°C
$C_{27}H_{25}N_5O_3S$ (499.50)

Massenspektrum: $(M+H)^+ = 500$

Beispiel 100

2-[(4-Amidinophenyl)-acetyl]-7-[N-(ethoxycarbonylmethyl)-chinolin-8-sulfonylamino]-1,2,3,4-tetrahydro-isochinolin

Hergestellt analog Beispiel le aus 2-[(4-Cyanophenyl)-acetyl]-7-[N-(ethoxycarbonylmethyl)-chinolin-8-sulfonylamino]-1,2,3,4-tetrahydro-isochinolin und ethanolischer Salzsäure, Ethanol und Ammoniumcarbonat.
Ausbeute: 35 % der Theorie,
$C_{31}H_{31}N_5O_5S$ (585.68)
$R_f$-Wert: 0.20 (Kieselgel; Dichlormethan/Ethanol = 4:1)

Massenspektrum (EKA): $(M+H)^+ = 586$
$(M+2H)^{++} = 293.6$
$(M+H+Na)^{++} = 304.6$

Beispiel 101

2-[(4-Amidinophenyl)-acetyl]-7-[N-(hydroxycarbonylmethyl)-chinolin-8-sulfonylamino]-1,2,3,4-tetrahydro-isochinolin

Hergestellt analog Beispiel 3 aus 2-[(4-Amidinophenyl)-acetyl]-7-[N-(ethoxycarbonylmethyl)-chinolin-8-sulfonylamino]-1,2,3,4-tetrahydro-isochinolin und Natronlauge.
Ausbeute: 49 % der Theorie,
$C_{29}H_{27}N_5O_5S$ (557.6)
$R_f$-Wert: 0.17 (Kieselgel; Dichlormethan/Ethanol = 3:2)

Massenspektrum (EKA): $(M+H)^+ = 558$
$(M+Na)^+ = 580$
$(M+2H)^{++} = 279.7$
$(M+H+Na)^{++} = 290.7$

$(2M+H+Na)^{++} = 569$

Beispiel 102

2-[(4-Amidinophenyl)-oxymethyl]-4-methyl-7-[N-(ethoxycarbonylmethyl)-chinolin-8-sulfonylamino]-chinolin-hydrochlorid

a. 7-Amino-2,4-dimethyl-chinolin

54.8 g (0.36 Mol) 3-Acetylamino-anilin, 38.0 g (0.38 Mol) Acetylaceton und 32.5 ml Eisessig werden 2 Stunden bei 80°C gerührt. Nach Abkühlung wird das Reaktionsgemisch auf Eiswasser gegossen und mit Natriumhydrogencarbonatlösung neutralisiert. Nach dreifacher Extraktion mit Essigester werden die vereinigten organischen Phasen mit Kochsalzlösung gewaschen, über Natriumsulfat getrocknet und eingedampft. Das so erhaltene Rohprodukt wird mit 200 ml konz. Schwefelsäure 1 Stunde auf 105°C erhitzt. Nach Abkühlung wird das Reaktionsgemisch auf Eiswasser gegossen und mit Ammoniaklösung neutralisiert. Nach dreifacher Extraktion mit Essigester werden die vereinigten organischen Phasen mit Kochsalzlösung gewaschen, über Natriumsulfat getrocknet und eingedampft. Das Rohprodukt wird an Kieselgel chromatographiert, wobei anfangs mit Methylenchlorid, später mit Methylenchlorid/Ethanol (50:1, 25:1, 19:1 und 9:1) eluiert wird. Die gewünschten Fraktionen werden vereinigt, eingedampft und mit Petrolether verrieben.
Ausbeute: 24.15 g (39 % der Theorie),
$C_{11}H_{12}N_2$ (172.20)

Massenspektrum: $M^+ = 172$

b. 7-Phthalimido-2,4-dimethyl-chinolin

6.90 g (40 mMol) 7-Amino-2,4-dimethyl-chinolin, 5.95 g (42 mMol) Phthalsäureanhydrid und 100 ml Eisessig werden 2 Stunden zum Rückfluß erhitzt. Nach Abkühlung wird das Reaktionsgemisch auf Eiswasser gegossen, das ausgefallene Produkt abgesaugt, mit Wasser gewaschen und getrocknet.
Ausbeute: 8.85 g (73 % der Theorie),
Schmelzpunkt: 203-205°C

c. 7-Phthalimido-2,4-dimethyl-chinolin-1-oxid

4.25 g (14 mMol) 7-Phthalimido-2,4-dimethyl-chinolin werden in 500 ml siedendem Methylenchlorid gelöst. Nach Abkühlung auf Raumtemperatur werden 4.80 g 3-Chlorperbenzoesäure (ca. 50%ig) zugegeben. Nach 3 Stunden bei Raumtemperatur wird die Reaktionslösung je 1 x mit Natriumhydrogencarbonatlösung und Kochsalzlösung gewaschen, über Natriumsulfat getrocknet, eingedampft und aus Ethanol umkristallisiert.
Ausbeute: 2.45 g (55 % der Theorie),
Schmelzpunkt: >250°C

d. 2-Chlormethyl-4-methyl-phthalimido-chinolin

4.30 g (13.5 mMol) 7-Phthalimido-2,4-dimethyl-chinolin-1-oxid und 4.20 g (22 mMol) p-Toluolsulfochlorid werden in 300 ml Methylenchlorid 8 Stunden unter Rückfluß erhitzt. Nach Abkühlung auf Raumtemperatur wird die Reaktionslösung je 1 x mit Natriumhydrogencarbonatlösung und Kochsalzlösung gewaschen, über Natriumsulfat getrocknet und eingedampft. Der Rückstand wird an Kieselgel chromatographiert, wobei anfangs mit Methylenchlorid, später mit Methylenchlorid/Ethanol (50:1) eluiert wird. Die gewünschten Fraktionen werden vereinigt, eingedampft und mit Ether verrieben.
Ausbeute: 3.15 g (70 % der Theorie),
Schmelzpunkt: 212-215°C

e. 2-[(4-Cyanophenyl)-oxymethyl]-4-methyl-phthalimido-chinolin

895 mg (6.2 mMol) Kalium-tert.butylat werden in 50 ml Dimethylsulfoxid gelöst, mit 740 mg (6.2 mMol) 4-Hydroxy-benzonitril versetzt und 30 Minuten bei Raumtemperatur gerührt. Nach Zugabe von 2.0 g 2-Chlormethyl-4-methyl-7-phthalimido-chinolin wird das Reaktionsgemisch weitere 12 Stunden bei Raumtemperatur gerührt. Nach Zusatz von Eiswasser wird vom gebildeten Niederschlag abgesaugt, mit Wasser gewaschen und getrocknet. Ausbeute: 2.20

g (89 % der Theorie),
Schmelzpunkt: 231-233°C

f. 2-[(4-Cyanophenyl)-oxymethyl]-4-methyl-7-amino-chinolin

2.15 g (5.1 mMol) 2-[(4-Cyanophenyl)-oxymethyl]-4-methyl-7-phthalimido-chinolin werden in 75 ml Toluol/Methanol (2:1) gelöst, mit 7.5 ml 40%iger wäßriger Methylaminlösung versetzt und 2 Stunden bei Raumtemperatur gerührt. Anschließend wird die Lösung im Vakuum eingedampft, der Rückstand mit 2N Essigsäure verrührt, abgesaugt und getrocknet. Das Rohprodukt wird an Kieselgel chromatographiert, wobei anfangs mit Methylenchlorid, später mit Methylenchlorid/Ethanol (50:1) eluiert wird. Die gewünschten Fraktionen werden vereinigt, eingedampft und mit Ether verrieben.
Ausbeute: 1.05 g (71 % der Theorie),
Schmelzpunkt: 192-194°C

g. 2-[(4-Cyanophenyl)-oxymethyl]-4-methyl-7-(chinolin-8-sulfonylamino)-chinolin

Hergestellt analog Beispiel 1c aus 2-[(4-Cyanophenyl)-oxymethyl]-4-methyl-7-amino-chinolin und Chinolin-8-sulfonylchlorid.
Ausbeute: 67 % der Theorie,
Schmelzpunkt: 240-242°C

h. 2-[(4-Cyanophenyl)-oxymethyl]-4-methyl-7-[N-(ethoxycarbonylmethyl)-chinolin-8-sulfonylamino)-chinolin

Hergestellt analog Beispiel 1d aus 2-[(4-Cyanophenyl)-oxymethyl]-4-methyl-7-(chinolin-8-sulfonylamino)-chinolin und Bromessigsäureethylester.
Ausbeute: 92 % der Theorie,
Schmelzpunkt: sintert ab 85°C

i. 2-[(4-Amidinophenyl)-oxymethyl]-4-methyl-7-[N-(ethoxycarbonylmethyl)-chinolin-8-sulfonylamino)-chinolin-hydrochlorid

Hergestellt analog Beispiel le aus 2-[(4-Cyanophenyl)-oxymethyl]-4-methyl-7-[N-(ethoxycarbonylmethyl)-chinolin-8-sulfonylamino)-chinolin und Salzsäure/Ammoniumcarbonat in Ethanol. Ausbeute: 49 % der Theorie,
Schmelzpunkt: sintert ab 78°C
$C_{31}H_{29}N_5O_5S$ (583.62)

Massenspektrum: $(M+H)^+ = 584$
$(M+H+Na)^+ = 303.7$
$(2M+H)^+ = 1167$

Beispiel 103

2-[(4-Amidinophenyl)-oxymethyl]-4-methyl-7-[N-(hydroxycarbonylmethyl)-chinolin-8-sulfonylamino]-chinolin

Hergestellt analog Beispiel 3 aus 2-[(4-Amidinophenyl)-oxymethyl]-4-methyl-7-[N-(ethoxycarbonylmethyl)-chinolin-8-sulfonylamino]-chinolin und Natronlauge.
Ausbeute: 19 % der Theorie,
$C_{29}H_{25}N_5O_5S$ (555.6)

Massenspektrum (EKA): $(M+H)^+ = 556$
$(M+Na)^+ = 578$
$(M+2Na)^{++} = 300$
$(M-H+2Na)^+ = 600$

Beispiel 104

2-[(4-Amidinophenyl)-oxymethyl]-4-methyl-7-(chinolin-8-sulfonylamino)-chinolin

Hergestellt analog Beispiel le aus 2-[(4-Cyanophenyl)-oxymethyl]-4-methyl-7-(chinolin-8-sulfonylamino)-chinolin und ethanolischer Salzsäure, Ethanol und Ammoniumcarbonat.
Ausbeute: 22 % der Theorie,
$C_{27}H_{23}N_5O_3S$ (497.55)
$R_f$-Wert: 0.23 (Kieselgel; Dichlormethan/Ethanol = 4:1) Schmelzpunkt: sintert ab 195°C

Massenspektrum (EKA): $(M+H)^+$ = 498

Beispiel 105

2-[N-(4-Amidinophenyl)-aminomethyl]-6-(chinolin-8-sulfonylamino)-imidazo[1,2-a]pyridin-hydrochlorid

a. 2-Chlormethyl-5-nitro-imidazo[1,2-a]pyridin

12.6 g (0.1 Mol) 1,3-Dichloraceton werden auf 105°C erhitzt und portionsweise mit 8.25 g (60 mMol) 2-Amino-5-nitro-pyridin versetzt. Nach 10 Minuten bei 105°C wird das Reaktionsgemisch abgekühlt, mit Methylenchlorid/Ethanol (8:2) versetzt und an Kieselgel chromatographiert, wobei anfangs mit Methylenchlorid, später mit Methylenchlorid/Ethanol (25:1, 19:1 und 9:1) eluiert wird. Die gewünschten Fraktionen werden vereinigt, eingedampft und mit Ether verrieben.
Ausbeute: 4.35 g (34 % der Theorie),
Schmelzpunkt: 124-127°C

b. 2-[N-(4-Cyanophenyl)-aminomethyl]-6-nitro-imidazo[1,2-a]-pyridin

3.0 g (25.4 mMol) 4-Aminobenzonitril werden bei 120°C geschmolzen und portionsweise mit 1.30 g (6.3 mMol) 2-Chlormethyl-5-nitro-imidazol[1,2-a]pyridin versetzt. Nach 30 Minuten bei 120°C wird das Reaktionsgemisch abgekühlt, mit Methylenchlorid/Ethanol (8:2) versetzt und an Kieselgel chromatographiert, wobei anfangs mit Methylenchlorid, später mit Methylenchlorid/Ethanol (50:1, 25:1 und 15:1) eluiert wird. Die gewünschten Fraktionen werden vereinigt und eingedampft.
Ausbeute: 0.71 g (39 % der Theorie),
$R_f$-Wert: 0.50 (Kieselgel; Methylenchlorid/Ethanol = 19:1)

c. 2-[N-(4-Cyanophenyl)-aminomethyl]-6-amino-imidazo[1,2-a]-pyridin

Hergestellt analog Beispiel 1b aus 2-[N-(4-Cyanophenyl)-aminomethyl]-6-nitro-imidazo[1,2-a]pyridin und Wasserstoff/Palladium.
Ausbeute: 75 % der Theorie,
$R_f$-Wert: 0.20 (Kieselgel; Methylenchlorid/Ethanol = 9:1)

d. 2-[N-(4-Cyanophenyl)-aminomethyl]-6-(chinolin-8-sulfonylamino)-imidazo[1,2-a]pyridin

Hergestellt analog Beispiel 1c aus 2-[N-(4-Cyanophenyl)-aminomethyl]-6-amino-imidazo[1,2-a]pyridin und Chinolin-8-sulfonylchlorid.
Ausbeute: 35 % der Theorie,
$R_f$-Wert: 0.78 (Kieselgel; Methylenchlorid/Ethanol = 4:1 + Eisessig)

e. 2-[N-(4-Amidinophenyl)-aminomethyl]-6-(chinolin-8-sulfonylamino)-imidazo[1,2-a]pyridin-hydrochlorid

Hergestellt analog Beispiel le aus 2-[N-(4-Cyanophenyl)-aminomethyl]-6-(chinolin-8-sulfonylamino)-imidazo [1,2-a]pyridin und Salzsäure/Ammoniumcarbonat.
Ausbeute: 51 % der Theorie,
$R_f$-Wert: 0.15 (Kieselgel; Methylenchlorid/Ethanol = 4:1 + Eisessig)
$C_{24}H_{27}N_7O_2S$ (471.48)

Massenspektrum: $(M+H)^+$ = 472

Beispiel 106

2-[N-(4-Amidinophenyl)-aminomethyl]-6-[N-(ethoxycarbonylmethyl)-chinolin-8-sulfonylamino]-imidazo[1,2-a]pyridin

Hergestellt analog Beispiel le aus 2-[N-(4-Cyanophenyl)-aminomethyl]-6-[N-(ethoxycarbonylmethyl)-chinolin-8-sulfonylamino]-imidazo[1,2-a]pyridin und ethanolischer Salzsäure, Ethanol und Ammoniumcarbonat.
Ausbeute: 11 % der Theorie,
$C_{28}H_{27}N_7O_4S$ (557.65)

Massenspektrum (EKA): $(M+H)^+$ = 558
$(M+Na)^+$ = 580

Beispiel 107

2-[N-(4-Amidinophenyl)-aminomethyl]-3-methyl-6-[N-(ethoxycarbonylmethyl)-chinolin-8-sulfonylamino]-benzofuran-hydrochlorid

a. 4-Acetylamino-2-hydroxy-acetophenon

16.5 g (0.10 Mol) 3-Methoxy-acetanilid werden in 40 ml Dichlorethan gelöst und nach Zugabe von 19.6 g (0.25 Mol) Acetylchlorid portionsweise bei 5°C mit 42.0 g (0.32 Mol) Aluminiumchlorid versetzt. Nach 2 Stunden bei Raumtemperatur wird das Reaktionsgemisch noch 2 Stunden unter Rückfluß erhitzt. Nach Abkühlung auf Raumtemperatur wird Eis zugegeben, der gebildete Niederschlag abgesaugt, mit Wasser gewaschen und getrocknet.
Ausbeute: 14.8 g (77 % der Theorie),
$R_f$-Wert: 0.40 (Kieselgel; Petrolether/Essigester = 1:1)

b. 4-Amino-2-hydroxy-acetophenon

10.0 g (52 mMol) 4-Acetylamino-2-hydroxy-acetophenon und 100 ml 18%ige Salzsäure werden 15 Minuten zum Rückfluß erhitzt. Nach Abkühlung auf Raumtemperatur wird der gebildete Niederschlag abgesaugt, mit Eiswasser gewaschen und getrocknet. Das Filtrat wird eingedampft, in Wasser aufgenommen und mit konz. Ammoniak versetzt. Der gebildete Niederschlag wird abgesaugt, mit Eiswasser gewaschen, getrocknet und mit dem ersten Niederschlag vereinigt.
Ausbeute: 7.6 g (97 % der Theorie),
$R_f$-Wert: 0.65 (Kieselgel; Petrolether/Essigester = 1:1)

c. 4-Phthalimido-2-hydroxy-acetophenon

Hergestellt analog Beispiel 102b aus 4-Amino-2-hydroxy-acetophenon und Phthalsäureanhydrid.
Ausbeute: 75 % der Theorie,
$R_f$-Wert: 0.55 (Kieselgel; Petrolether/Essigester = 1:1)

d. 4-[(2-Carboxy)-benzoylamino]-2-carboxymethyloxy-acetophenon

18.9 g (67 mMol) 4-Phthalimido-2-hydroxy-acetophenon, 16.5 g (99 mMol) Bromessigsäureethylester und 40.0 g (0.3 Mol) Kaliumcarbonat werden in 100 ml Aceton aufgenommen und 6 Stunden unter Rückfluß erhitzt. Nach Abkühlung auf Raumtemperatur wird der gebildete Niederschlag abgesaugt und getrocknet. Das Filtrat wird eingedampft, in Wasser aufgenommen und 3 x mit Essigester extrahiert. Die vereinigten organischen Extrakte werden mit Wasser gewaschen und getrocknet. Die vereinigten Rohprodukte werden in 50 ml Ethanol gelöst, mit 50 ml 3 N Natronlauge versetzt und 30 Minuten bei Raumtemperatur gerührt. Nach Zugabe von 100 ml Wasser und Ansäuern mit 6 N Salzsäure wird der gebildete Niederschlag abgesaugt, mit kaltem Wasser gewaschen und getrocknet.
Ausbeute: 19.4 g (81 % der Theorie),
$R_f$-Wert: 0.30 (Kieselgel; Methylenchlorid/Ethanol = 7:3)

e. 3-Methyl-6-phthalimido-benzofuran

Eine Mischung von 36.0 g (0.1 Mol) 4-[(2-Carboxy)-benzoylamino]-2-carboxymethyloxy-acetophenon, 30 g (0.37 Mol) Natriumacetat, 770 ml Acetanhydrid und 153 ml Eisessig werden 2.5 Stunden zum Rückfluß erhitzt. Das Reaktionsgemisch wird eingedampft, der Rückstand wird mit Wasser verrieben, abgesaugt, mit Wasser gewaschen und getrocknet.
Ausbeute: 21.6 g (77 % der Theorie),
$R_f$-Wert: 0.85 (Kieselgel; Methylenchlorid + 2,5 % Ethanol)

f. 2-[N-(4-Cyanophenyl)-aminomethyl]-3-methyl-6-phthalimidobenzofuran

5.68 g (20 mMol) 3-Methyl-6-phthalimido-benzofuran werden in 150 ml Methylenchlorid gelöst, mit 5.0 g Paraformaldehyd und 20 g Thionylchlorid versetzt und 60 Stunden bei Raumtemperatur gerührt. Das Reaktionsgemisch wird eingedampft, 2 mal in Methylenchlorid gelöst und erneut zur Trockene eingedampft. Das Rohprodukt wird in 150 ml Toluol gelöst, mit 5.1 g (43 mMol) 4-Aminobenzonitril und 20 g Aluminiumoxid versetzt und 6 Stunden unter Rückfluß erhitzt. Das Reaktionsgemisch wird eingedampft, der Rückstand in Methylenchlorid aufgenommen und an Kieselgel (Methylenchlorid) chromatographiert. Die gewünschten Fraktionen werden vereinigt, eingedampft und mit Petrolether/-Methylenchlorid verrieben.
Ausbeute: 6.0 g (68 % der Theorie),
$R_f$-Wert: 0.30 (Kieselgel; Methylenchlorid)

g. 2-[N-(4-Cyanophenyl)-aminomethyl]-3-methyl-6-amino-benzofuran

Hergestellt analog Beispiel 102f aus 2-[N-(4-Cyanophenyl)-aminomethyl]-3-methyl-6-phthalimido-benzofuran und Methylamin.
Ausbeute: 65 % der Theorie,
$R_f$-Wert: 0.25 (Kieselgel; Methylenchlorid)

h. 2-[N-(4-Cyanophenyl)-aminomethyl]-3-methyl-6-(chinolin-8-sulfonylamino)-benzofuran

Hergestellt analog Beispiel 1c aus 2-[N-(4-Cyanophenyl)-aminomethyl]-3-methyl-6-amino-benzofuran und Chinolin-8-sulfonylchlorid.
Ausbeute: 97 % der Theorie,
$R_f$-Wert: 0.65 (Kieselgel; Methylenchlorid/Ethanol = 95:5)

i. 2-[N-(4-Cyanophenyl)-aminomethyl]-3-methyl-6-[N-(ethoxycarbonylmethyl)-chinolin-8-sulfonylamino)-benzofuran

Hergestellt analog Beispiel 1d aus 2-[N-(4-Cyanophenyl)-aminomethyl]-3-methyl-6-(chinolin-8-sulfonylamino-benzofuran und Bromessigsäureethylester.
Ausbeute: 99 % der Theorie,
$R_f$-Wert: 0.70 (Kieselgel; Methylenchlorid/Ethanol = 95:5)

j. 2-[N-(4-Amidinophenyl)-aminomethyl]-3-methyl-6-[N-(ethoxycarbonylmethyl)-chinolin-8-sulfonylamino]-benzofuran-hydrochlorid

Hergestellt analog Beispiel le aus 2-[N-(4-Cyanophenyl)-aminomethyl]-3-methyl-6-[N-(ethoxycarbonylmethyl)-chinolin-8-sulfonylamino]-benzofuran und Salzsäure/Ammoniumcarbonat.
Ausbeute: 32 % der Theorie,
$R_f$-Wert: 0.21 (Kieselgel; Methylenchlorid/Ethanol = 4:1 + Eisessig)
$C_{30}H_{29}N_5O_5S$ (571.67)

Massenspektrum: $(M+H)^+ = 572$
$(M+2H)^{++} = 286.7$
$(M+H+Na)^{++} = 297.7$

Beispiel 108

2-[N-(4-Amidinophenyl)-aminomethyl]-3-methyl-6-[N-(hydroxycarbonylmethyl)-chinolin-8-sulfonylamino]-benzofuran

Hergestellt analog Beispiel 3 aus 2-[N-(4-Amidinophenyl)-aminomethyl]-3-methyl-6-[N-(ethoxycarbonylmethyl)-chinolin-8-sulfonylamino]-benzofuran und Natronlauge.
Ausbeute: 94 % der Theorie,
$C_{28}H_{25}N_5O_5S$ (543,61)
$R_f$-Wert: 0.12 (Kieselgel; Dichlormethan/Ethanol = 4:1 + Eisessig)

Massenspektrum (EKA): $(M+H)^+$ = 544
$(M+2H)^{++}$ = 272.7
$(M+H+Na)^{++}$ = 283.6
$(M+2Na)^{++}$ = 294.7

Beispiel 109

2-[N-(4-Amidinophenyl)-aminomethyl]-3-methyl-6-(chinolin-8-sulfonylamino)-benzofuran

Hergestellt analog Beispiel le aus 2-[N-(4-Cyanophenyl)-aminomethyl]-3-methyl-6-(chinolin-8-sulfonylamino)-benzofuran und ethanolischer Salzsäure, Ethanol und Ammoniumcarbonat.
Ausbeute: 17 % der Theorie,
$C_{26}H_{23}N_5O_3S$ (485,58)
$R_f$-Wert: 0.17 (Kieselgel; Dichlormethan/Ethanol = 4:1 + Eisessig)

Massenspektrum (EKA): $(M+H)^+$ = 486

Beispiel 110

2-[(4-Amidinophenyl)-oxymethyl]-4-methyl-7-[N-(ethoxycarbonylmethyl)-benzoylamino]-chinolin

Hergestellt analog Beispiel le aus 2-[(4-Cyanophenyl)-oxymethyl]-4-methyl-7-[N-(ethoxycarbonylmethyl)-benzoylamino]-chinolin und ethanolischer Salzsäure, Ethanol und Ammoniumcarbonat.
Ausbeute: 64 % der Theorie,
$C_{29}H_{28}N_4O_4$ (496.6)

Massenspektrum (EKA): $(M+H)^+$ = 497
$(M+H+Na)^{++}$ = 260

Beispiel 111

2-[N-(4-Amidinophenyl)-aminomethyl]-4-methyl-7-[N-(ethoxycarbonylmethyl)-chinolin-8-sulfonylamino]-chinolin

Hergestellt analog Beispiel 1e aus 2-[N-(4-Cyanophenyl)-aminomethyl]-4-methyl-7-[N-(ethoxycarbonylmethyl)-chinolin-8-sulfonylamino]-chinolin und ethanolischer Salzsäure, Ethanol und Ammoniumcarbonat.
Ausbeute: 63 % der Theorie,
$C_{31}H_{30}N_6O_4S$ (582,69)
$R_f$-Wert: 0.15 (Kieselgel; Dichlormethan/Ethanol = 4:1 + Eisessig)

Massenspektrum (EKA): $(M+H)^+$ = 583
$(M+H+Na)^{++}$ = 303

Beispiel 112

2-[N-(4-Amidinophenyl)-aminomethyl]-4-methyl-7-[N-(hydroxycarbonylmethyl)-chinolin-8-sulfonylamino]-chinolin

Hergestellt analog Beispiel 3 aus 2-[N-(4-Amidinophenyl)-aminomethyl]-4-methyl-7-[N-(ethoxycarbonylmethyl)-chinolin-8-sulfonylamino]-chinolin und Natronlauge.

Ausbeute: 49 % der Theorie,
$C_{29}H_{26}N_6O_4S$ (554,64)

Massenspektrum (EKA): $(M+H)^+ = 555$
$(M+Na)^+ = 577$
$(M+2Na)^{++} = 300$
$(2M+3Na)^{3+} = 392.6$

Beispiel 113

2-[(4-Amidinophenyl)-oxymethyl]-4-methyl-7-[N-(hydroxycarbonylmethyl)-benzoylamino]-chinolin

Hergestellt analog Beispiel 3 aus 2-[(4-Amidinophenyl)-oxymethyl]-4-methyl-7-[N-(ethoxycarbonylmethyl)-benzoylamino]-chinolin und Natronlauge.
Ausbeute: 26 % der Theorie,
$C_{27}H_{24}N_4O_4$ (468.49)

Massenspektrum (EKA): $(M+H)^+ = 469$

Beispiel 114

2-[2-(4-Amidinophenyl)-ethyl]-3-methyl-6-[N-(ethoxycarbonylmethyl)-chinolin-8-sulfonylamino]-benzofuran

Hergestellt analog Beispiel 1e aus 2-[2-(4-Cyanophenyl)-ethyl]-3-methyl-6-[N-(ethoxycarbonylmethyl)-chinolin-8-sulfonylamino]-benzofuran und ethanolischer Salzsäure, Ethanol und Ammoniumcarbonat.
Ausbeute: 84 % der Theorie,
$C_{31}H_{30}N_4O_5S$ (570.68)
$R_f$-Wert: 0.24 (Kieselgel; Dichlormethan/Ethanol = 4:1 + Eisessig)

Massenspektrum (EKA): $(M+H)^+ = 571$
$(M+H+Na)^{++} = 297$

Beispiel 115

2-[N-(4-Amidinophenyl)-aminomethyl]-3-methyl-6-[N-(ethoxycarbonylmethyl)-benzolsulfonylamino]-benzofuran

Hergestellt analog Beispiel le aus 2-[N-(4-Cyanophenyl)-aminomethyl]-3-methyl-6-[N-(ethoxycarbonylmethyl)-benzolsulfonylamino]-benzofuran und ethanolischer Salzsäure, Ethanol und Ammoniumcarbonat.
Ausbeute: 36 % der Theorie,
$C_{27}H_{28}N_4O_5S$ (520.62)
$R_f$-Wert: 0.22 (Kieselgel; Dichlormethan/Ethanol = 4:1 + Eisessig)

Massenspektrum (EKA): $(M+H)^+ = 521$

Beispiel 116

2-[2-(4-Amidinophenyl)-ethyl]-3-methyl-6-[N-(hydroxycarbonylmethyl)-chinolin-8-sulfonylamino]-benzofuran

Hergestellt analog Beispiel 3 aus 2-[2-(4-Amidinophenyl)-ethyl]-3-methyl-6-[N-(ethoxycarbonylmethyl)-chinolin-8-sulfonylamino]-benzofuran und Natronlauge.
Ausbeute: 87 % der Theorie,
$C_{29}H_{26}N_4O_5S$ (542,63)
$R_f$-Wert: 0.13 (Kieselgel; Dichlormethan/Ethanol = 4:1 + Eisessig)

Massenspektrum (EKA): $(M+H)^+ = 543$
$(M+Na)^+ = 565$

Beispiel 117

2-[N-(4-Amidinophenyl)-aminomethyl]-3-methyl-6-[N-(hydroxycarbonylmethyl)-benzolsulfonylamino]-benzofuran

Hergestellt analog Beispiel 3 aus 2-[N-(4-Amidinophenyl)-aminomethyl]-3-methyl-6-[N-(ethoxycarbonylmethyl)-benzolsulfonylamino]-benzofuran und Natronlauge.
Ausbeute: 79 % der Theorie,
$C_{25}H_{24}N_4O_5S$ (492,57)
$R_f$-Wert: 0.12 (Kieselgel; Dichlormethan/Ethanol = 4:1 + Eisessig)

Massenspektrum (EKA): $(M+H)^+ = 493$
$(M+Na)^+ = 515$
$(M+2Na)^{++} = 269$

Beispiel 118

2-[N-(4-Amidinophenyl)-aminomethyl]-3-methyl-6-[N-(N'-(ethoxycarbonylmethyl)-aminocarbonylmethyl)-chinolin-8-sulfonylamino]-benzofuran

Hergestellt analog Beispiel le aus 2-[N-(4-Cyanophenyl)-aminomethyl]-3-methyl-6-[N-(N'-(ethoxycarbonylmethyl)-aminocarbonylmethyl)-chinolin-8-sulfonylamino]-benzofuran und ethanolischer Salzsäure, Ethanol und Ammoniumcarbonat.
Ausbeute: 81 % der Theorie,
$C_{32}H_{32}N_6O_6S$ (628,72)

Massenspektrum (EKA): $(M+H)^+ = 629$
$(M+H+Na)^{++} = 326$

Beispiel 119

2-[N-(4-Amidinophenyl)-aminomethyl]-3-methyl-6-[N-(N'-(1H-tetrazol-5-yl)-aminocarbonylmethyl)-chinolin-8-sulfonylamino]-benzofuran

a. 2-[N-(4-Cyanophenyl)-aminomethyl]-3-methyl-6-[N-(N'-(1H-tetrazol-5-yl)-aminocarbonylmethyl)-chinolin-8-sulfonylamino]-benzofuran

0.53 g (1.0 mMol) 2-[N-(4-Cyanophenyl)-aminomethyl]-3-methyl-6-carboxymethyl-chinolin-8-sulfonylamino)-benzofuran werden in 20 ml Tetrahydrofuran gelöst, mit 0.2 g (1.2 mMol) Carbonyldiimidazol und 0.1 g (1.0 mMol) 5-Amino-tetrazol versetzt und 5 Stunden unter Rückfluß erhitzt. Das Reaktionsgemisch wird eingedampft, der Rückstand in Ethanol gelöst und an Kieselgel (Methylenchlorid + 2,5 % Ethanol) chromatographiert. Die gewünschten Fraktionen werden vereinigt und eingedampft.
Ausbeute: 0.11 g (19 % der Theorie),
$C_{29}H_{23}N_9O_4S$ (593.64)
$R_f$-Wert: 0.18 (Kieselgel; Methylenchlorid/Ethanol = 9:1) Massenspektrum: $(M-H)^- = 592$

b. 2-[N-(4-Amidinophenyl)-aminomethyl]-3-methyl-6-[N-(N'-(1Htetrazol-5-yl)-aminocarbonylmethyl)-chinolin-8-sulfonylamino]-benzofuran

Hergestellt analog Beispiel le aus 2-[N-(4-Cyanophenyl)-aminomethyl]-3-methyl-6-[N-(N'-(1H-tetrazol-5-yl)-aminocarbonylmethyl)-chinolin-8-sulfonylamino]-benzofuran und Salzsäure/Ammoniumcarbonat.
Ausbeute: 97 % der Theorie,
$C_{29}H_{26}N_{10}O_4S$ (610.67)

Massenspektrum (EKA): $(M+H)^+ = 611$
$(M+Na)^+ = 633$
$(M+H+Na)^{++} = 317$

Beispiel 120

2-[N-(4-Amidinophenyl)-aminomethyl]-3-methyl-6-[N-(1H-tetrazol-5-yl)-methyl)-chinolin-8-sulfonylamino]-benzofuran

a. 5-Brommethyl-1-(2-cyanoethyl)-tetrazol

1.50 g (7.85 mMol) Bromessigsäure-(2-cyanoethyl)-amid werden in 50 ml Methylenchlorid gelöst und mit 508 mg (7.85 mMol) Natriumazid versetzt. Bei 0°C wird eine Lösung von 2.20 g (7.85 mMol) Trifluoressigsäureanhydrid in 5 ml Methylenchlorid zugetropft. Nach 22 Stunden bei Raumtemperatur wird gesättigte Natriumhydrogencarbonatlösung zugegeben und 3 x mit Methylenchlorid extrahiert. Die vereinigten organischen Phasen werden über Natriumsulfat getrocknet und eingedampft. Das Rohprodukt wird an Kieselgel chromatographiert, wobei anfangs mit Methylenchlorid, später mit Methylenchlorid/Ethanol (50:1) eluiert wird. Die gewünschten Fraktionen werden vereinigt und eingedampft.
Ausbeute: 505 mg (30 % der Theorie),
$C_5H_6BrN_3$ (216.06)

Massenspektrum (EKA): $M^+$ = 215/217 (Br)

b. 2-[N-(4-Cyanophenyl)-aminomethyl]-3-methyl-6-[N-[1-(2-cyanoethyl)-tetrazol-5-yl)-methyl)-chinolin-8-sulfonylamino]-benzofuran

Hergestellt analog Beispiel 1d aus 2-[N-(4-Cyanophenyl)-aminomethyl]-3-methyl-6-(chinolin-8-sulfonylamino)-benzofuran und 5-Brommethyl-1-(2-cyanoethyl)-tetrazol.
Ausbeute: 98 % der Theorie,
$R_f$-Wert: 0.45 (Kieselgel; Methylenchlorid/Ethanol = 95:5)

c. 2-[N-(4-Cyanophenyl)-aminomethyl]-3-methyl-6-[N-(1H-tetrazol-5-yl-methyl)-chinolin-8-sulfonylamino]-benzofuran

0.5 g (0.83 mMol) 2-[N-(4-Cyanophenyl)-aminomethyl]-3-methyl-6-[N-[1-(2-cyanoethyl)-tetrazol-5-yl-methyl]-chinolin-8-sulfonylamino]-benzofuran werden in 50 ml Methylenchlorid gelöst, mit 0.28 g (2.5 mMol) Kalium-tert.butylat versetzt und 90 Minuten bei Raumtemperatur gerührt. Das Reaktionsgemisch wird eingedampft, der Rückstand in Wasser gelöst und mit Eisessig angesäuert. Der gebildete Niederschlag wird abfiltriert, mit Wasser gewaschen und getrocknet. Das Rohprodukt wird an Kieselgel (Methylenchlorid + 1-2 % Ethanol) chromatographiert. Die gewünschten Fraktionen werden vereinigt und eingedampft.
Ausbeute: 110 mg (24 % der Theorie),
$R_f$-Wert: 0.43 (Kieselgel; Dichlormethan/Ethanol = 9:1)

d. 2-[N-(4-Amidinophenyl)-aminomethyl]-3-methyl-6-[N-(1H-tetrazol-5-yl)-methyl)-chinolin-8-sulfonylamino]-benzofuranhydrochlorid

Hergestellt analog Beispiel le aus 2-[N-(4-Cyanophenyl)-aminomethyl]-3-methyl-6-[N-(1-tetrazol-5-yl-methyl)-chinolin-8-sulfonylamino]-benzofuran und Salzsäure/Ammoniumcarbonat. Ausbeute: 97 % der Theorie,
$C_{28}H_{25}N_9O_3S$ (567.66)

Massenspektrum (EKA): $(M+H)^+$ = 568
$(M+Na)^+$ = 590
$(M+H+Na)^{++}$ = 295.6
$(M+2Na)^{++}$ = 306.7

Beispiel 121

2-[2-(4-Amidinophenyl)-ethyl]-3-methyl-6-[N-(N'-(ethoxycarbonylmethyl)-aminocarbonylmethyl)-chinolin-8-sulfonylamino]-benzofuran

Hergestellt analog Beispiel le aus 2-[2-(4-Cyanophenyl)-ethyl]-3-methyl-6-[N-(N'-(ethoxycarbonylmethyl)-aminocarbonylmethyl)-chinolin-8-sulfonylamino]-benzofuran und ethanolischer Salzsäure, Ethanol und Ammoniumcarbonat.
Ausbeute: 61 % der Theorie,
$C_{33}H_{33}N_5O_6S$ (627,73)

$R_f$-Wert: 0.25 (Kieselgel; Dichlormethan/Ethanol = 4:1 + Eisessig)

Massenspektrum (EKA): $(M+H)^+ = 628$
$(M+2H)^{++} = 314.7$
$(M+H+Na)^{++} = 325.7$

Beispiel 122

2-[N-(4-Amidinophenyl)-aminomethyl]-3-methyl-6-[N-(N'-(hydroxycarbonylmethyl)-aminocarbonylmethyl)-chinolin-8-sulfonylamino]-benzofuran

Hergestellt analog Beispiel 3 aus 2-[N-(4-Amidinophenyl)-aminomethyl]-3-methyl-6-[N-(N'-(ethoxycarbonylmethyl)-aminocarbonylmethyl)-chinolin-8-sulfonylamino]-benzofuran und Natronlauge.
Ausbeute: 69 % der Theorie,
$C_{30}H_{28}N_6O_6S$ (600.67)

Massenspektrum (EKA): $(M+H)^+ = 601$
$(M+Na)^+ = 623$
$(M+2H)^{++} = 301$
$(M+H+Na)^{++} = 312$
$(M+2Na)^{++} = 323$

Beispiel 123

2-[2-(4-Amidinophenyl)-ethyl]-4-methyl-7-[N-(1H-tetrazol-5-ylmethyl)-chinolin-8-sulfonylamino]-chinolin

Hergestellt analog Beispiel le aus 2-[2-(4-Cyanophenyl)-ethyl]-4-methyl-7-[N-(1H-tetrazol-5-yl-methyl)-chinolin-8-sulfonylamino]-chinolin und ethanolischer Salzsäure, Ethanol und Ammoniumcarbonat.
Ausbeute: 31 % der Theorie,
$C_{30}H_{27}N_9O_2S$ (577.67)
$R_f$-Wert: 0.15 (Kieselgel; Dichlormethan/Ethanol = 4:1 + Eisessig)

Massenspektrum (EKA): $(M+H)^+ = 578$
$(M+Na)^+ = 600$
$(M-H)^- = 576$

Beispiel 124

1-Methyl-2-[N-(4-(N-n-hexyloxycarbonylamidino)phenyl)-aminomethyl]-5-[N-(ethoxycarbonylmethyl)-chinolin-8-sulfonylamino]-benzimidazol

Hergestellt analog Beispiel 31 aus 1-Methyl-2-[N-(4-amidinophenyl)-aminomethyl]-5-[N-(ethoxycarbonylmethyl)-chinolin-8-sulfonylamino]-benzimidazol und Chlorameisensäure-n-hexylester.
Ausbeute: 61 % der Theorie,
$C_{36}H_{41}N_7O_6S$ (699.84)
$R_f$-Wert: 0.60 (Kieselgel; Dichlormethan/Methanol = 9:1)

Massenspektrum (EKA): $(M+H)^+ = 700$
$(M+Na)^+ = 722$
$(M+H+Na)^{++} = 361.8$

Beispiel 125

1-Methyl-2-[N-(4-(N-n-octyloxycarbonylamidino)phenyl)-aminomethyl]-5-[N-(ethoxycarbonylmethyl)-chinolin-8-sulfonylamino]-benzimidazol

Hergestellt analog Beispiel 31 aus 1-Methyl-2-[N-(4-amidinophenyl)-aminomethyl]-5-[N-(ethoxycarbonylmethyl)-chinolin-8-sulfonylamino]-benzimidazol und Chlorameisensäure-n-octylester.

Ausbeute: 65 % der Theorie,
$C_{38}H_{45}N_7O_6S$ (727.89)
$R_f$-Wert: 0.58 (Kieselgel; Dichlormethan/Methanol = 9:1)

Massenspektrum (EKA): $(M+H)^+ = 728$
$(M+Na)^+ = 750$
$(M+H+Na)^{++} = 375.8$

Beispiel 126

1-Methyl-2-[N-(4-(N-n-butyloxycarbonylamidino)phenyl)-aminomethyl]-5-[N-(ethoxycarbonylmethyl)-chinolin-8-sulfonylamino]-benzimidazol

Hergestellt analog Beispiel 31 aus 1-Methyl-2-[N-(4-amidinophenyl)-aminomethyl]-5-[N-(ethoxycarbonylmethyl)-chinolin-8-sulfonylamino]-benzimidazol und Chlorameisensäure-n-butylester.
Ausbeute: 64 % der Theorie,
$C_{34}H_{37}N_7O_6S$ (671.78)
$R_f$-Wert: 0.57 (Kieselgel; Dichlormethan/Methanol = 9:1)

Massenspektrum (EKA): $(M+H)^+ = 672$
$(M+Na)^+ = 694$
$(M+H+Na)^{++} = 347.8$

Beispiel 127

1-Methyl-2-[N-(4-amidino-2-methoxy-phenyl)-aminomethyl]-5-(N-methyl-benzolsulfonylamino)-benzimidazol

Hergestellt analog Beispiel le aus 1-Methyl-2-[N-(4-cyano-2-methoxy-phenyl)-aminomethyl]-5-(N-methyl-benzolsulfonylamino)-benzimidazol und ethanolischer Salzsäure, Ethanol und Ammoniumcarbonat.
Ausbeute: 57 % der Theorie,
$C_{24}H_{26}N_6O_3S$ (478.6)

Massenspektrum (EKA): $(M+H)^+ = 479$
$(M+Na)^+ = 501$

Beispiel 128

1-Methyl-2-[N-(4-amidinophenyl)-aminomethyl]-5-(N-methyl-phenylacetylamino)-benzimidazol

Hergestellt analog Beispiel le aus 1-Methyl-2-[N-(4-cyanophenyl)-aminomethyl]-5-(N-methyl-phenylacetylamino)-benzimidazol und ethanolischer Salzsäure, Ethanol und Ammoniumcarbonat.
Ausbeute: 54 % der Theorie,
$C_{25}H_{26}N_6O$ (426.53)
$R_f$-Wert: 0.27 (Kieselgel; Dichlormethan/Methanol = 5:1)

Massenspektrum (EKA): $(M+H)^+ = 427$
$(M+2H)^{++} = 214$

Beispiel 129

1-Methyl-2-[N-(4-(N-benzoylamidino)-phenyl)-aminomethyl]-5-[N-(ethoxycarbonylmethyl)-chinolin-8-sulfonylamino]-benzimidazo]

Hergestellt analog Beispiel 31 aus 1-Methyl-2-[N-(4-amidinophenyl)-aminomethyl]-5-[N-(ethoxycarbonylmethyl)-chinolin-8-sulfonylamino]-benzimidazol und Benzoylchlorid.
Ausbeute: 54% der Theorie,
$C_{36}H_{33}N_7O_5S$ (675.77)

Massenspektrum: $(M+H)^+ = 676$
$(M+Na)^+ = 698$

Beispiel 130

1-Methyl-2-[N-(4-(N-benzoylamidino)-phenyl)-aminomethyl]-5-[N-(n-propyloxycarbonylmethyl)-chinolin-8-sulfonylamino]-benzimidazol

Hergestellt analog Beispiel 31 aus 1-Methyl-2-[N-(4-amidinophenyl)-aminomethyl]-5-[N-(n-propyloxycarbonylmethyl)-chinolin-8-sulfonylamino]-benzimidazol und Benzoylchlorid.
Ausbeute: 52% der Theorie,
$C_{37}H_{35}N_7O_5S$ (689.77)

Massenspektrum: $(M+H)^+ = 690$
$(M+Na)^+ = 712$

Beispiel 131

Trockenampulle mit 75 mg Wirkstoff pro 10 ml

Zusammensetzung:

| | |
|---|---|
| Wirkstoff | 75,0 mg |
| Mannitol | 50,0 mg |
| Wasser für Injektionszwecke | ad 10,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 132

Trockenampulle mit 35 mg Wirkstoff pro 2 ml

Zusammensetzung:

| | |
|---|---|
| Wirkstoff | 35,0 mg |
| Mannitol | 100,0 mg |
| Wasser für Injektionszwecke | ad 2,0 ml |

Herstellung:

Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

Beispiel 133

Tablette mit 50 mg Wirkstoff

Zusammensetzung:

| | | |
|---|---|---|
| (1) | Wirkstoff | 50,0 mg |
| (2) | Milchzucker | 98,0 mg |
| (3) | Maisstärke | 50,0 mg |
| (4) | Polyvinylpyrrolidon | 15,0 mg |
| (5) | Magnesiumstearat | 2,0 mg |
| | | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreBt, biplan mit beidseitiger Facette und einseitiger Teilkerbe. Durchmesser der Tabletten: 9 mm.

Beispiel 134

Tablette mit 350 mg Wirkstoff

Zusammensetzung:

| | | |
|---|---|---|
| (1) | Wirkstoff | 350,0 mg |
| (2) | Milchzucker | 136,0 mg |
| (3) | Maisstärke | 80,0 mg |
| (4) | Polyvinylpyrrolidon | 30,0 mg |
| (5) | Magnesiumstearat | 4,0 mg |
| | | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

Beispiel 135

Kapseln mit 50 mg Wirkstoff

Zusammensetzung:

| | | |
|---|---|---|
| (1) | Wirkstoff | 50,0 mg |
| (2) | Maisstärke getrocknet | 58,0 mg |
| (3) | Milchzucker pulverisiert | 50,0 mg |
| (4) | Magnesiumstearat | 2,0 mg |
| | | 160,0 mg |

Herstellung:

(1) wird mit (3) verrieben. Diese Verreibung wird der Mischung aus (2) und (4) unter intensiver Mischung zugegeben.
Diese Pulvermischung wird auf einer Kapselabfüllmaschine in Hartgelatine-Steckkapseln Größe 3 abgefüllt.

Beispiel 136

Kapseln mit 350 mg Wirkstoff

Zusammensetzung:

| | | |
|---|---|---|
| (1) | Wirkstoff | 350,0 mg |
| (2) | Maisstärke getrocknet | 46,0 mg |
| (3) | Milchzucker pulverisiert | 30,0 mg |
| (4) | Magnesiumstearat | 4,0 mg |
| | | 430,0 mg |

Herstellung:

(1) wird mit (3) verrieben. Diese Verreibung wird der Mischung aus (2) und (4) unter intensiver Mischung zugegeben.
Diese Pulvermischung wird auf einer Kapselabfüllmaschine in Hartgelatine-Steckkapseln Größe 0 abgefüllt.

Beispiel 137

Suppositorien mit 100 mg Wirkstoff

| 1 | Zäpfchen enthält: | |
|---|---|---|
| | Wirkstoff | 100,0 mg |
| | Polyethylenglykol (M.G. 1500) | 600,0 mg |
| | Polyethylenglykol (M.G. 6000) | 460,0 mg |
| | Polyethylensorbitanmonostearat | 840,0 mg |
| | | 2 000,0 mg |

Herstellung:

Das Polyethylenglykol wird zusammen mit Polyethylensorbitanmonostearat geschmolzen. Bei 40°C wird die gemahlene Wirksubstanz in der Schmelze homogen dispergiert. Es wird auf 38°C abgekühlt und in schwach vorgekühlte Suppositorienformen ausgegossen.

**Patentansprüche**

1. Disubstituierte bicyclische Heterocyclen der allgemeinen Formel

$$R_a - Het - B - Ar - E , \quad (I)$$

in der
B eine gegebenenfalls durch eine oder zwei $C_{1-3}$-Alkylgruppen substituierte Ethylengruppe, wobei eine Methylengruppen der Ethylengruppe, die entweder mit dem Rest Het oder Ar verknüpft ist, durch ein Sauerstoff- oder Schwefelatom, durch eine Sulfinyl-, Sulfonyl-, Carbonyl- oder -$NR_1$-Gruppe ersetzt sein kann, wobei

$R_1$ ein Wasserstoffatom oder eine $C_{1-6}$-Alkylgruppe darstellt,

oder B auch eine geradkettige $C_{3-5}$-Alkylengruppe, in der eine Methylengruppe, die weder mit dem Rest Het noch mit dem Rest Ar verknüpft ist, durch eine -$NR_1$-Gruppe ersetzt ist, in der $R_1$ wie vorstehend erwähnt definiert ist,

E eine Cyano- oder $R_b$NH-C(=NH)-Gruppe, in der

$R_b$ ein Wasserstoffatom, eine Hydroxygruppe, eine $C_{1-3}$-Alkylgruppe oder einen in vivo abspaltbaren Rest darstellt,

Ar eine gegebenenfalls durch ein Fluor-, Chlor- oder Bromatom, durch eine Trifluormethyl-, $C_{1-3}$-Alkyl- oder $C_{1-3}$-Alkoxygruppe substituierte Phenylen- oder Naphthylengruppe,

eine gegebenenfalls im Kohlenstoffgerüst durch eine $C_{1-3}$-Alkylgruppe substituierte Thienylen-, Thiazolylen-, Pyridinylen-, Pyrimidinylen-, Pyrazinylen- oder Pyridazinylengruppe,

Het einen bicyclischen Heterocyclus der Formel

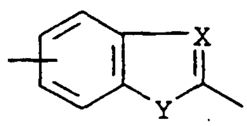

,

in der

X ein Stickstoffatom oder eine gegebenenfalls durch eine $C_{1-3}$-Alkylgruppe substituierte Methingruppe und

Y eine gegebenenfalls durch eine $C_{1-5}$-Alkyl- oder $C_{3-7}$-Cycloalkylgruppe substituierte Iminogruppe, ein Sauerstoffoder Schwefelatom oder

X ein Stickstoffatom und

Y eine durch eine $C_{1-5}$-Alkyl- oder $C_{3-7}$-Cycloalkylgruppe substituierte Iminogruppe, wobei der Alkyl- und Cycloalkylsubstituent jeweils durch eine Carboxygruppe oder eine in-vivo in eine Carboxygruppe überführbare Gruppe substituiert ist, wobei in einem der vorstehend erwähnten Heterocyclen zusätzlich eine nicht angulare Methingruppe durch ein Stickstoffatom ersetzt sein kann,

oder Het eine Gruppe der Formeln

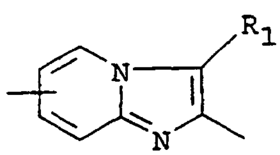

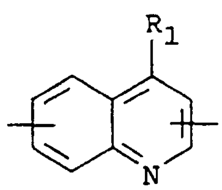

oder

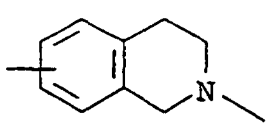

,

wobei

$R_1$ wie vorstehend erwähnt definiert ist,

und $R_a$ eine Phenyl-$C_{1-3}$-alkoxygruppe,

eine Aminogruppe,

eine $C_{1-3}$-Alkylaminogruppe, die am Stickstoffatom zusätzlich durch eine Phenyl-$C_{1-3}$-alkylgruppe substituiert ist, eine $R_3$-CO-$R_4$N- oder $R_3$-$SO_2$-$R_4$N-Gruppe, in denen

$R_3$ eine $C_{1-5}$-Alkyl-, Phenyl-$C_{1-3}$-alkyl-, $C_{3-7}$-Cycloalkyl-, Phenyl-, Naphthyl-, Pyridyl-, Chinolyl-, Isochinolyl-, Tetrahydrochinolyl- oder Tetrahydroisochinolylgruppe und

$R_4$ ein Wasserstoffatom, $C_{1-5}$-Alkyl- oder Phenyl-$C_{1-3}$-alkylgruppe, die jeweils im Alkylteil durch eine in vivo in eine Carboxygruppe überführbare Gruppe, durch eine Carboxy- oder Tetrazolylgruppe, durch eine Aminocarbonyl- oder $C_{1-3}$-Alkylaminocarbonylgruppe, die jeweils am Stickstoffatom zusätzlich durch eine in vivo in eine Carboxy-$C_{1-3}$-alkylgruppe überführbare Gruppe oder durch eine Carboxygruppe substituiert sind, eine endständig durch eine Di-($C_{1-3}$-Alkyl)-aminogruppe substituierte $C_{2-5}$-Alkylgruppe oder eine $C_{3-7}$-Cycloalkylgruppe darstellen,

bedeuten,

deren Tautomere, deren Streoisomere und deren Salze.

**2.** Disubstituierte bicyclische Heterocyclen der allgemeinen Formel I gemäß Anspruch 1, in denen

B eine gegebenenfalls durch eine oder zwei Methylgruppen substituierte Ethylengruppe, wobei eine Methylengruppen der Ethylengruppe, die entweder mit dem Rest Het oder Ar verknüpft ist, durch ein Sauerstoff- oder Schwefelatom, durch eine Carbonyl- oder -$NR_1$-Gruppe ersetzt sein kann, wobei

$R_1$ ein Wasserstoffatom oder eine Methylgruppe darstellt,

oder B auch eine n-Propylengruppe, in der die mittlere Methylengruppe durch eine -$NR_1$-Gruppe ersetzt ist, in der $R_1$ wie vorstehend erwähnt definiert ist,

E eine Cyano- oder $R_b$NH-C(=NH)-Gruppe, in der

$R_b$ ein Wasserstoffatom, eine $C_{1-8}$-Alkyloxy-carbonyl-, $C_{5-7}$-Cycloalkyloxy-carbonyl-, Benzoyl-, Nicotinoyl- oder Isonicotinoylgruppe darstellt,

Ar eine gegebenenfalls durch ein Fluor-, Chlor- oder Bromatom, durch eine Trifluormethyl-, Methyl- oder Methoxygruppe substituierte Phenylengruppe, oder eine gegebenenfalls im Kohlenstoffgerüst durch eine Methylgruppe substituierte Thienylengruppe,

Het einen bicyclischen Heterocyclus der Formel

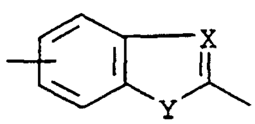

,

in der

X ein Stickstoffatom oder eine gegebenenfalls durch eine Methylgruppe substituierte Methingruppe und

Y eine gegebenenfalls durch eine $C_{1-3}$-Alkyl- oder $C_{3-7}$-Cycloalkylgruppe substituierte Iminogruppe, ein Sauerstoff- oder Schwefelatom oder

X ein Stickstoffatom und

Y eine durch eine $C_{1-3}$-Alkylgruppe substituierte Iminogruppe, wobei der Alkylteil zusätzlich durch eine Carboxyoder $C_{1-3}$-Alkyloxy-carbonylgruppe substituiert ist,

oder Het eine Gruppe der Formeln

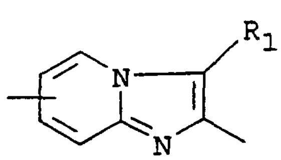

,

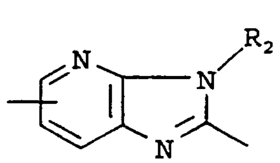

,

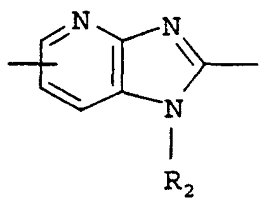 ,

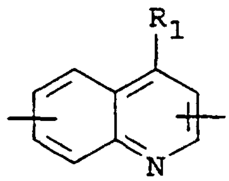

oder

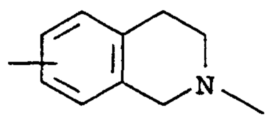 ,

wobei

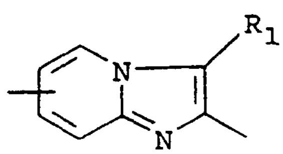 ,

$R_1$ wie vorstehend erwähnt definiert ist und

$R_2$ eine durch eine Carboxy- oder $C_{1-3}$-Alkoxy-carbonylgruppe substituiert $C_{1-3}$-Alkylgruppe darstellt,

und $R_a$ eine Benzyloxygruppe,

eine Aminogruppe,

eine $C_{1-3}$-Alkylaminogruppe, die am Stickstoffatom zusätzlich durch eine Benzylgruppe substituiert ist,

eine $R_3$-CO-$R_4$N- oder $R_3$-$SO_2$-$R_4$N-Gruppe, in denen

$R_3$ eine $C_{1-4}$-Alkyl-, Benzyl-, $C_{5-7}$-Cycloalkyl-, Phenyl-, Pyridyl-, Chinolyl-, Isochinolyl-, Tetrahydrochinolyl- oder Tetrahydroisochinolylgruppe und

$R_4$ ein Wasserstoffatom, eine $C_{1-3}$-Alkylgruppe, die durch eine Carboxy-, $C_{1-3}$-Alkoxy-carbonyl-, Tetrazolyl-, Aminocarbonyloder $C_{1-3}$-Alkylaminocarbonylgruppe substituiert ist, wobei die Aminocarbonyl- und $C_{1-3}$-Alkylaminocarbonylgruppe jeweils am Stickstoffatom zusätzlich durch eine eine Carboxy-$C_{1-3}$-alkyl- oder $C_{1-3}$-Alkoxy-carbonyl-$C_{1-3}$-alkylgruppe substituiert sind, oder eine endständig durch eine Di-($C_{1-3}$-alkyl)-aminogruppe substituierte $C_{2-3}$-Alkylgruppe darstellen,

bedeuten, deren Isomere und deren Salze.

**3.** Disubstituierte bicyclische Heterocyclen der allgemeinen Formel I gemäß Anspruch 1, in denen

B eine gegebenenfalls durch eine oder zwei Methylgruppen substituierte Ethylengruppe, wobei eine Methylengruppen der Ethylengruppe, die entweder mit dem Rest Het oder Ar verknüpft ist, durch ein Sauerstoff- oder Schwefelatom, durch eine Carbonyl- oder -$NR_1$-Gruppe ersetzt sein kann, wobei

$R_1$ ein Wasserstoffatom oder eine Methylgruppe darstellt,

oder B auch eine n-Propylengruppe, in der die mittlere Methylengruppe durch eine -$NR_1$-Gruppe ersetzt ist, in der $R_1$ wie vorstehend erwähnt definiert ist,
E eine $R_b$NH-C(=NH)-Gruppe, in der
$R_b$ ein Wasserstoffatom, eine $C_{1-8}$-Alkyloxy-carbonyl-, $C_{5-7}$-Cycloalkyloxy-carbonyl- oder Benzoylgruppe darstellt,
Ar eine gegebenenfalls durch ein Fluor-, Chlor- oder Bromatom, durch eine Trifluormethyl-, Methyl- oder Methoxygruppe substituierte Phenylengruppe, oder eine gegebenenfalls im Kohlenstoffgerüst durch eine Methylgruppe substituierte Thienylengruppe,
Het einen bicyclischen Heterocyclus der Formel

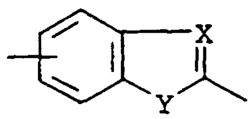 ,

in der
X ein Stickstoffatom oder eine gegebenenfalls durch eine Methylgruppe substituierte Methingruppe und
Y eine gegebenenfalls durch eine $C_{1-3}$-Alkyl- oder $C_{3-7}$-Cycloalkylgruppe substituierte Iminogruppe, ein Sauerstoffoder Schwefelatom oder
X ein Stickstoffatom und
Y eine durch eine $C_{1-3}$-Alkylgruppe substituierte Iminogruppe, wobei der Alkylteil zusätzlich durch eine Carboxyoder $C_{1-3}$-Alkyloxy-carbonylgruppe substituiert ist,
und $R_a$ eine Benzyloxygruppe,
eine Aminogruppe,
eine $C_{1-3}$-Alkylaminogruppe, die am Stickstoffatom zusätzlich durch eine Benzylgruppe substituiert ist,
eine $R_3$-CO-$R_4$N- oder $R_3$-$SO_2$-$R_4$N-Gruppe, in denen
$R_3$ eine $C_{1-4}$-Alkyl-, Benzyl-, $C_{5-7}$-Cycloalkyl-, Phenyl-, Pyridyl-, Chinolyl-, Isochinolyl-, Tetrahydrochinolyloder Tetrahydroisochinolylgruppe und
$R_4$ ein Wasserstoffatom, eine $C_{1-3}$-Alkylgruppe, die durch eine Carboxy-, $C_{1-3}$-Alkoxy-carbonyl-, Tetrazolyl-, Aminocarbonyloder $C_{1-3}$-Alkylaminocarbonylgruppe substituiert ist, wobei die Aminocarbonyl- und $C_{1-3}$-Alkylaminocarbonylgruppe jeweils am Stickstoffatom zusätzlich durch eine eine Carboxy-$C_{1-3}$-alkyl- oder $C_{1-3}$-Alkoxy-carbonyl-$C_{1-3}$-alkylgruppe substituiert sind, oder eine endständig durch eine Di-($C_{1-3}$-alkyl)-aminogruppe substituierte $C_{2-3}$-Alkylgruppe darstellen,
bedeuten, deren Isomere und deren Salze.

**4.** Disubstituierte bicyclische Heterocyclen der allgemeinen Formel I gemäß Anspruch 3, in denen
$R_a$ in 5-Stellung eine $R_3$-CO-$R_4$N- oder $R_3$-$SO_2$-$R_4$N-Gruppe, in denen $R_3$ und $R_4$ wie vorstehend erwähnt definiert sind,
deren Isomere und deren Salze.

**5.** Disubstituierte bicyclische Heterocyclen der allgemeinen Formel Ia, in der

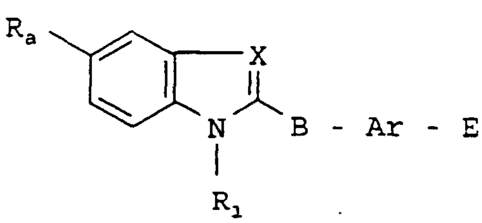 , (Ia)

in der
X eine Methingruppe oder ein Stickstoffatom,
B eine Ethlengruppe, wobei die mit Ar verknüpte Methylengruppe durch ein Sauerstoffatom oder eine Iminogruppe ersetzt sein kann,
Ar eine 1,4-Phenylengruppe,

E eine Amidinogruppe,
$R_1$ eine Methylgruppe und
$R_a$ eine $R_3$-CO-$R_4$N- oder $R_3$-$SO_2$-$R_4$N-Gruppe, wobei
$R_4$ eine durch eine Carboxy-, $C_{1-3}$-Alkoxy-carbonyl-, Carboxymethylaminocarbonyl- oder $C_{1-3}$-Alkoxy-carbonylmethylaminocarbonylgruppe substituierte Methylgruppe und
$R_3$ eine Isochinolin-8-yl-Gruppe darstellen,
deren Isomere und deren Salze.

**6.** Disubstituierte bicyclische Heterocyclen der allgemeinen Formel Ia gemäß Anspruch 5, in denen $R_a$ eine $R_3$-$SO_2$-$R_4$N-Gruppe darstellt,
deren Isomere und deren Salze.

**7.** Folgende disubstituierte bicyclische Heterocyclen der allgemeinen Formel Ia gemäß Anspruch 5:

(a) 1-Methyl-2-[(4-amidinophenyl)-oxymethyl]-5-[N-(hydroxycarbonylmethyl)-chinolin-8-sulfonylamino]-benzimidazol,

(b) 1-Methyl-2-[2-(4-amidinophenyl)-ethyl]-5-[N-(N'-(hydroxycarbonylmethyl)-aminocarbonylmethyl)-chinolin-8-sulfonylamino]-benzimidazol,

(c) 1-Methyl-2-[N-(4-amidinophenyl)-aminomethyl]-5-[N-(hydroxycarbonylmethyl)-chinolin-8-sulfonylamino]-benzimidazol und

(d) 1-Methyl-2-[N-(4-amidinophenyl)-aminomethyl]-5-[N-(hydroxycarbonylmethyl)-chinolin-8-sulfonylamino]-indol

sowie deren Salze.

**8.** Physiologisch verträgliche Salze der Verbindungen gemäß den Ansprüchen 1 bis 7.

**9.** Arzneimittel, enthaltend eine Verbindung nach mindestens einem der Ansprüche 1 bis 7, in denen E eine $R_b$NH-C(=NH)-Gruppe darstellt, oder ein Salz gemäß Anspruch 8 neben gegebenenfalls einem oder mehreren inerten Trägerstoffen und/oder Verdünnungsmitteln.

**10.** Verwendung einer Verbindung nach mindestens einem der Ansprüche 1 bis 7, bis 7, in denen E eine $R_b$NH-C(=NH)-Gruppe darstellt, oder ein Salz gemäß Anspruch 8 zur Herstellung eines Arzneimittels mit einer die Thrombinzeit verlängernder Wirkung, einer thrombinhemmender Wirkung und einer Hemmwirkung auf verwandte Serinproteasen.

**11.** Verfahren zur Herstellung eines Arzneimittels gemäß Anspruch 9, **dadurch gekennzeichnet, daß** auf nichtchemischem Wege eine Verbindung nach mindestens einem der Ansprüche 1 bis 7, in denen E eine $R_b$NH-C(=NH)-Gruppe darstellt, oder ein Salz gemäß Anspruch 8 in einen oder mehrere inerte Trägerstoffe und/oder Verdünnungsmittel eingearbeitet wird.

**12.** Verfahren zur Herstellung der Verbindungen gemäß den Ansprüchen 1 bis 8, **dadurch gekennzeichnet, daß**

a. zur Herstellung einer Verbindung der allgemeinen Formel I, in der E eine $R_b$NH-C(=NH)-Gruppe bedeutet, in der $R_b$ ein Wasserstoffatom, eine Hydroxy- oder $C_{1-3}$-Alkylgruppe darstellt, eine gegebenenfalls im Reaktionsgemisch gebildete Verbindung der allgemeinen Formel

$$R_a - Het - B - Ar - C(=NH) - Z_1 , \qquad (II)$$

in der
B, Ar, Het und $R_a$ wie in den Ansprüchen 1 bis 7 erwähnt definiert sind und
$Z_1$ eine Alkoxy-, Aralkoxy-, Alkylthio- oder Aralkylthiogruppe, mit einem Amin der allgemeinen Formel

$$H_2N - R_b' ,\qquad (III)$$

in der
$R_b'$ ein Wasserstoffatom, eine Hydroxy- oder $C_{1-3}$-Alkylgruppe darstellt, umgesetzt wird oder

b. zur Herstellung einer Verbindung der allgemeinen Formel I, in der die $R_a$-Gruppe und E mit der Maßgabe wie in den Ansprüchen 1 bis 7 erwähnt definiert sind, daß die $R_a$-Gruppe eine Carboxygruppe enthält und E wie in den Ansprüchen 1 bis 7 definiert ist oder die $R_a$-Gruppe wie in den Ansprüchen 1 bis 7 erwähnt definiert ist und E eine $NH_2$-C(=NH)-Gruppe darstellt oder die $R_a$-Gruppe eine Carboxygruppe enthält und E eine $NH_2$-C (=NH)-Gruppe darstellt, eine Verbindung der allgemeinen Formel

$$R_a' - Het - B - Ar - E' ,\qquad (IV)$$

in der
A, B, Ar und Het wie in den Ansprüchen 1 bis 7 definiert sind und
die $R_a'$-Gruppe und E' die für die $R_a$-Gruppe und E in den Ansprüchen 1 bis 7 erwähnten Bedeutungen mit der Maßgabe besitzen, daß die $R_a'$-Gruppe eine durch Hydrolyse, Behandeln mit einer Säure oder Base, Thermolyse oder Hydrogenolyse in eine Carboxylgruppe überführbare Gruppe enthält und E wie in den Ansprüchen 1 bis 7 definiert ist oder E' eine durch Hydrolyse, Behandeln mit einer Säure oder Base, Thermolyse oder Hydrogenolyse in eine $NH_2$-C(=NH)-Gruppe überführbare Gruppe darstellt und die $R_a'$-Gruppe die für die $R_a$-Gruppe in den Ansprüchen 1 bis 7 erwähnten Bedeutungen aufweist oder die $R_a'$-Gruppe eine durch Hydrolyse, Behandeln mit einer Säure oder Base, Thermolyse oder Hydrogenolyse in eine Carboxylgruppe überführbare Gruppe enthält und E' eine durch Hydrolyse, Behandeln mit einer Säure oder Base, Thermolyse oder Hydrogenolyse in eine $NH_2$-C(=NH)-Gruppe überführbare Gruppe darstellt,
mittels Hydrolyse, Behandeln mit einer Säure oder Base, Thermolyse oder Hydrogenolyse in eine Verbindung der allgemeinen Formel I übergefürt wird, in der die $R_a$-Gruppe und E mit der Maßgabe wie in den Ansprüchen 1 bis 7 erwähnt definiert sind, daß die $R_a$-Gruppe eine Carboxygruppe enthält und E wie in den Ansprüchen 1 bis 7 definiert ist oder die $R_a$-Gruppe die in den Ansprüchen 1 bis 7 erwähnten Bedeutungen aufweist und E eine $NH_2$-C(=NH)-Gruppe darstellt oder die $R_a$-Gruppe eine Carboxygruppe enthält und E eine $NH_2$-(C=NH) -Gruppe darstellt, übergeführt wird oder

c. zur Herstellung einer Verbindung der allgemeinen Formel I, in der die $R_a$-Gruppe eine der bei der Definition der $R_a$-Gruppe in den Ansprüchen 1 bis 7 erwähnten Estergruppen enthält, eine Verbindung der allgemeinen Formel

$$R_a'' - Het - B - Ar - E ,\qquad (V)$$

in der
B, E, Ar und Het wie in den Ansprüchen 1 bis 7 definiert sind und
$R_a''$-Gruppe die für die $R_a$-Gruppe in den Ansprüchen 1 bis 7 erwähnten Bedeutungen mit der Maßgabe aufweist, daß die $R_a''$-Gruppe eine Carboxylgruppe oder eine mittels eines Alkohols in eine entsprechende Estergruppe überführbare Gruppe enthält, mit einem Alkohol der allgemeinen Formel

$$HO - R_8 ,\qquad (VI)$$

in der
$R_8$ der Alkylteil einer der in den Ansprüchen 1 bis 7 erwähnten in-vivo abspaltbaren Reste mit Ausnahme der $R_5$-CO-O-($R_5CR_7$)-Gruppe für eine Carboxylgruppe darstellt, oder mit deren Formamidacetalen
oder mit einer Verbindung der allgemeinen Formel

$$Z_2 - R_9 ,\qquad (VII)$$

in der
$R_9$ der Alkylteil einer der in den Ansprüchen 1 bis 7 erwähnten in-vivo abspaltbaren Reste mit Ausnahme der $R_5$-CO-O-($R_5CR_7$)-Gruppe für eine Carboxylgruppe und
$Z_2$ eine Austrittsgruppe darstellen, umgesetzt wird oder

d. zur Herstellung einer Verbindung der allgemeinen Formel I, in der $R_b$ einen in vivo abspaltbaren Rest darstellt, eine Verbindung der allgemeinen Formel

$$R_a - Het - B - Ar - C(=NH) - NH_2\ , \qquad (VIII)$$

in der
$R_a$, Het, B und Ar wie in den Ansprüchen 1 bis 7 definiert sind, mit einer Verbindung der allgemeinen Formel

$$Z_3 - R_{10}\ , \qquad (IX)$$

in der
$R_{10}$ einen in vivo abspaltbaren Rest und
$Z_3$ eine nukleofuge Austrittsgruppe bedeuten, umgesetzt wird oder

e. zur Herstellung einer Verbindung der allgemeinen Formel I, in der $R_a$ eine Aminogruppe und E eine Cyanogruppe darstellen, eine Nitroverbindung der allgemeinen Formel

$$NO_2 - Het - B - Ar - CN\ , \qquad (X)$$

in der
B, Ar und Het wie in den Ansprüchen 1 bis 7 erwähnt definiert sind, reduziert wird oder

f. zur Herstellung einer Verbindung der allgemeinen Formel I, in der $R_a$ eine Aminogruppe und E eine Cyanogruppe darstellen, ein Schutzrest für eine Aminogruppe von einer Verbindung der allgemeinen Formel

$$R_a''' - Het - B - Ar - CN\ , \qquad (XI)$$

in der
B, Ar und Het wie in den Ansprüchen 1 bis 7 erwähnt definiert sind und
$R_a'''$ eine durch einen Schutzrest geschützete Aminogruppe bedeutet, abgespalten wird oder

g. zur Herstellung einer Verbindung der allgemeinen Formel I, in der $R_a$ eine $R_3$-CO-$R_4$N- oder $R_3$-$SO_2$-$R_4$N-Gruppe und E eine Cyanogruppe darstellen, eine Verbindung der allgemeinen Formel

$$R_4NH - Het - B - Ar - CN\ , \qquad (XII)$$

in der
$R_4$, Het, B und Ar wie in den Ansprüchen 1 bis 7 erwähnt definiert sind, mit einer Verbindung der allgemeinen Formel

$$R_3 - X - Z_4\ , \qquad (XIII)$$

in der
$R_3$ wie in den Ansprüchen 1 bis 7 erwähnt definiert ist,
X eine Carbonyl- oder Sulfonylgruppe und
$Z_4$ eine nukleofuge Austrittsgruppe oder auch, falls X eine Carbonylgruppe darstellt, zusammen mit einem

Wasserstoffatom des benachbarten Stickstoffatoms eine weitere Kohlenstoff-Stickstoffbindung bedeuten, umgesetzt wird oder

h. zur Herstellung einer Verbindung der allgemeinen Formel I, in der $R_a$ eine $R_3$-CO-$R_4$N- oder $R_3$-$SO_2$-$R_4$N-Gruppe und E eine Cyanogruppe darstellen, wobei $R_4$ mit Ausnahme des Wasserstoffatoms wie in den Ansprüchen 1 bis 7 erwähnt definiert ist, eine Verbindung der allgemeinen Formel

$$R_3 - X - NH - Het - B - Ar - CN \,, \qquad (XIV)$$

in der
$R_3$, Het, B, Ar und X wie in den Ansprüchen 1 bis 7 erwähnt definiert sind, mit einer Verbindung der allgemeinen Formel

$$R_4' - Z_5 \,, \qquad (XV)$$

in der
$R_4$' mit Ausnahme des Wasserstoffatoms die für $R_4$ in den Ansprüchen 1 bis 7 erwähnten Bedeutungen besitzt und
$Z_5$ eine nukleofuge Austrittsgruppe bedeutet, umgesetzt wird oder

i. zur Herstellung einer Verbindung der allgemeinen Formel I, in der $R_4$ eine $C_{1-5}$-Alkyl- oder Phenyl-$C_{1-3}$-alkylgruppe, die jeweils im Alkylteil durch eine in vivo in eine Carboxygruppe überführbare Gruppe, durch eine Tetrazolylgruppe, durch eine Aminocarbonyl- oder $C_{1-3}$-Alkylaminocarbonylgruppe, die jeweils am Stickstoffatom zusätzlich durch eine durch eine in vivo in eine Carboxy-$C_{1-3}$-alkylgruppe überführbare Gruppe substituiert sind und E eine Cyanogruppe darstellen, eine Verbindung der allgemeinen Formel

$$R_3 - X - NR_4' - Het - B - Ar - CN \,, \qquad (XVI)$$

in der
$R_3$, Het, B, Ar und X wie in den Ansprüchen 1 bis 7 erwähnt definiert sind und
$R_4$' eine $C_{1-5}$-Alkyl- oder Phenyl-$C_{1-3}$-alkylgruppe, die jeweils im Alkylteil durch eine in vivo in eine Carboxygruppe überführbare Gruppe, durch eine Tetrazolylgruppe, durch eine Aminocarbonyl- oder $C_{1-3}$-Alkylaminocarbonylgruppe, die jeweils am Stickstoffatom zusätzlich durch eine durch eine in vivo in eine Carboxy-$C_{1-3}$-alkylgruppe überführbare Gruppe substituiert sind, oder deren reaktionsfähigen Derivaten mit einer Verbindung der allgemeinen Formel

$$R_{11} - H \,, \qquad (XVII)$$

in der
$R_4$' mit Ausnahme des Wasserstoffatoms die für $R_4$ in den Ansprüchen 1 bis 7 erwähnten Bedeutungen besitzt und
$R_{11}$ ein der bei der Definition der Restes $R_4$ in den Ansprüchen 1 bis 7 erwähnten Substituenten der $C_{1-5}$-Alkyl- oder Phenyl-$C_{1-3}$-alkylgruppe darstellt, der über eine Carbonylgruppe mit der Rest $R_{11}$ verbunden ist, umgesetzt wird oder

j. zur Herstellung einer Benzimidazolyl-, Benzthiazolyl- oder Benzoxazolylverbindung der allgemeinen Formel I, in der B eine Ethylengruppe darstellt, eine gegebenenfalls im Reaktionsgemisch gebildete Verbindung der allgemeinen Formel

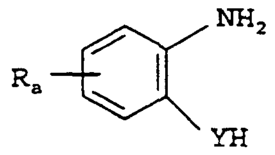

, (XVIII)

in der
$R_a$ und Y wie in den Ansprüchen 1 bis 7 erwähnt definiert sind, mit einer Verbindung der allgemeinen Formel

$$HO\text{-}CO - B' - Ar - E , \qquad (IXX)$$

in der
Ar und E wie in den Ansprüchen 1 bis 7 erwähnt definiert sind und
B' eine gegebenenfalls durch eine oder zwei $C_{1-3}$-Alkylgruppen substituierte Ethylengruppe bedeutet, umgesetzt wird oder

k. zur Herstellung einer Verbindung der allgemeinen Formel I, die eine der in den Ansprüchen 1 bis 7 erwähnten Tetrahydro-chinolin- oder -isochinolinreste enthält, eine Verbindung der allgemeinen Formel I, die eine der in den Ansprüchen 1 bis 7 erwähnten Chinolin- oder -isochinolinreste enthält, hydriert wird und
erforderlichenfalls anschließend ein bei den vorstehend beschriebenen Umsetzungen verwendeter Schutz für gegebenenfalls vorhandene reaktive Gruppen abgespalten wird und/oder
gewünschtenfalls anschließend eine so erhaltene Verbindung der allgemeinen Formel I in ihre Stereoisomere aufgetrennt wird und/oder
eine so erhaltene Verbindung der allgemeinen Formel I in ihre Salze, insbesondere in ihre physiologisch verträglichen Salze übergeführt wird.

**Claims**

**1.** Disubstituted bicyclic heterocycles of general formula

$$R_a - Het - B - Ar - E , \qquad (I)$$

wherein
B denotes an ethylene group optionally substituted by one or two $C_{1-3}$-alkyl groups, whilst a methylene group of the ethylene group, which is linked to either the Het or Ar group, may be replaced by an oxygen or sulphur atom, by a sulphinyl, sulphonyl, carbonyl or $-NR_1$ group, whilst
$R_1$ denotes a hydrogen atom or a $C_{1-6}$-alkyl group,
or B also denotes a straight-chained $C_{3-5}$-alkylene group, in which a methylene group, which is linked neither to the Het group nor to the Ar group, is replaced by an $-NR_1$ group wherein $R_1$ is as hereinbefore defined,
E denotes a cyano or $R_bNH\text{-}C(=NH)$ group wherein
$R_b$ denotes a hydrogen atom, a hydroxy group, a $C_{1-3}$-alkyl group or a group which may be cleaved *in vivo*,
Ar denotes a phenylene or naphthylene group optionally substituted by a fluorine, chlorine or bromine atom, or by a trifluoromethyl, $C_{1-3}$-alkyl or $C_{1-3}$-alkoxy group,
a thienylene, thiazolylene, pyridinylene, pyrimidinylene, pyrazinylene or pyridazinylene group optionally substituted in the carbon skeleton by a $C_{1-3}$-alkyl group,
Het denotes a bicyclic heterocycle of the formula

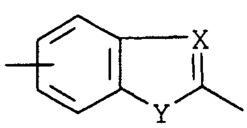

,

wherein

X denotes a nitrogen atom or a methyne group optionally substituted by a $C_{1-3}$-alkyl group and

Y denotes an imino group optionally substituted by a $C_{1-5}$-alkyl or $C_{3-7}$-cycloalkyl group, an oxygen or sulphur atom or

X denotes a nitrogen atom and

Y denotes an imino group substituted by a $C_{1-5}$-alkyl or $C_{3-7}$-cycloalkyl group, wherein the alkyl and cycloalkyl substituent in each case is substituted by a carboxy group or a group which can be converted *in vivo* into a carboxy group, whilst additionally in one of the above-mentioned heterocycles a non-angular methyne group may be replaced by a nitrogen atom,

or Het denotes a group of the formulae

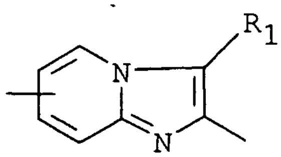

,

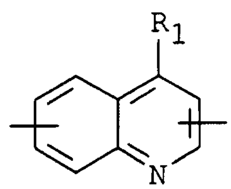

or

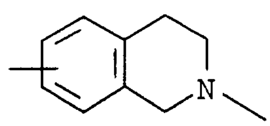

,

wherein

$R_1$ is as hereinbefore defined,

and $R_a$ denotes a phenyl-$C_{1-3}$-alkoxy group,

an amino group,

a $C_{1-3}$-alkylamino group, which is additionally substituted at the nitrogen atom by a phenyl-$C_{1-3}$-alkyl group,

a $R_3$-CO-$R_4$N or $R_3$-$SO_2$-$R_4$N group wherein

$R_3$ denotes a $C_{1-5}$-alkyl, phenyl-$C_{1-3}$-alkyl, $C_{3-7}$-cycloalkyl, phenyl, naphthyl, pyridyl, quinolyl, isoquinolyl, tetrahydroquinolyl or tetrahydroisoquinolyl group and

$R_4$ denotes a hydrogen atom, $C_{1-5}$-alkyl or phenyl-$C_{1-3}$-alkyl group, each of which is substituted in the alkyl moiety by a group which may be converted *in vivo* into a carboxy group, by a carboxy or tetrazolyl group, by an aminocarbonyl or $C_{1-3}$-alkylaminocarbonyl group, each of which is additionally substituted at the nitrogen atom by a group which may be converted *in vivo* into a carboxy-$C_{1-3}$-alkyl group or by a carboxy group, a $C_{2-5}$-alkyl group terminally substituted by a di-($C_{1-3}$-alkyl)-amino group, or a $C_{3-7}$-cycloalkyl group,

the tautomers, stereoisomers and salts thereof.

**2.** Disubstituted bicyclic heterocycles of general formula I according to claim 1 wherein

B denotes an ethylene group optionally substituted by one or two methyl groups, whilst a methylene group of the ethylene group, which is linked to either the Het or Ar group, may be replaced by an oxygen or sulphur atom, by a carbonyl or -$NR_1$ group, whilst

$R_1$ denotes a hydrogen atom or a methyl group,

or B also denotes an n-propylene group wherein the central methylene group is replaced by an -$NR_1$ group wherein

$R_1$ is as hereinbefore defined,
E denotes a cyano or $R_bNH-C(=NH)$ group wherein
$R_b$ denotes a hydrogen atom, a $C_{1-8}$-alkyloxy-carbonyl, $C_{5-7}$-cycloalkyloxy-carbonyl, benzoyl, nicotinoyl or isonicotinoyl group,
Ar denotes a phenylene group optionally substituted by a fluorine, chlorine or bromine atom, by a trifluoromethyl, methyl or methoxy group, or a thienylene group optionally substituted in the carbon skeleton by a methyl group,
Het denotes a bicyclic heterocycle of the formula

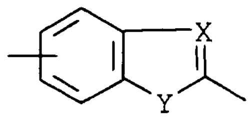

,

wherein
X denotes a nitrogen atom or a methyne group optionally substituted by a methyl group and
Y denotes an imino group optionally substituted by a $C_{1-3}$-alkyl or $C_{3-7}$-cycloalkyl group, an oxygen or sulphur atom or
X denotes a nitrogen atom and
Y denotes an imino group substituted by a $C_{1-3}$-alkyl group, whilst the alkyl moiety is additionally substituted by a carboxy or $C_{1-3}$-alkyloxy-carbonyl group,
or Het denotes a group of the formulae

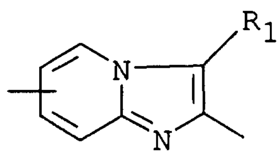

,

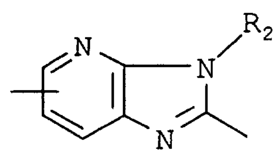

,

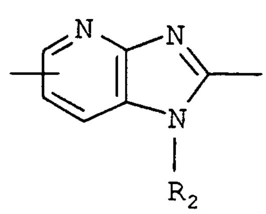

,

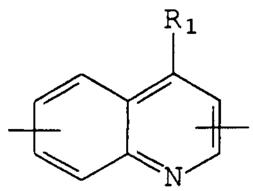 ,

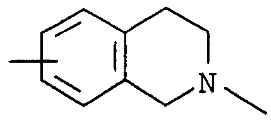 ,

or

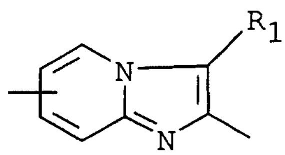 ,

where

$R_1$ is as hereinbefore defined and

$R_2$ denotes a $C_{1-3}$-alkyl group substituted by a carboxy or $C_{1-3}$-alkoxy-carbonyl group,

and $R_a$ denotes a benzyloxy group,

an amino group,

a $C_{1-3}$-alkylamino group, which is additionally substituted at the nitrogen atom by a benzyl group,

a $R_3$-CO-$R_4$N or $R_3$-$SO_2$-$R_4$N group wherein

$R_3$ denotes a $C_{1-4}$-alkyl, benzyl, $C_{5-7}$-cycloalkyl, phenyl, pyridyl, quinolyl, isoquinolyl, tetrahydroquinolyl or tetrahydroisoquinolyl group and

$R_4$ denotes a hydrogen atom, a $C_{1-3}$-alkyl group, which is substituted by a carboxy, $C_{1-3}$-alkoxy-carbonyl, tetrazolyl, aminocarbonyl or $C_{1-3}$-alkylaminocarbonyl group, whilst the aminocarbonyl and

$C_{1-3}$-alkylaminocarbonyl group are each additionally substituted at the nitrogen atom by a carboxy-$C_{1-3}$-alkyl or $C_{1-3}$-alkoxy-carbonyl-$C_{1-3}$-alkyl group, or a $C_{2-3}$-alkyl group terminally substituted by a di-($C_{1-3}$-alkyl)-amino group,

the isomers and the salts thereof.

**3.** Disubstituted bicyclic heterocycles of general formula I according to claim 1 wherein

B denotes an ethylene group optionally substituted by one or two methyl groups, whilst a methylene group of the ethylene group, which is linked to either the Het or Ar group, may be replaced by an oxygen or sulphur atom, by a carbonyl or -$NR_1$ group, wherein

$R_1$ denotes a hydrogen atom or a methyl group,

or B also denotes an n-propylene group wherein the central methylene group is replaced by an -$NR_1$ group wherein $R_1$ is as hereinbefore defined,

E denotes an $R_b$NH-C(=NH) group wherein

$R_b$ denotes a hydrogen atom, a $C_{1-8}$-alkyloxy-carbonyl, $C_{5-7}$-cycloalkyloxy-carbonyl or benzoyl group,

Ar denotes a phenylene group optionally substituted by a fluorine, chlorine or bromine atom, by a trifluoromethyl, methyl or methoxy group, or a thienylene group optionally substituted in the carbon skeleton by a methyl group,

Het denotes a bicyclic heterocycle of the formula

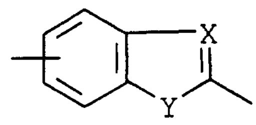

,

wherein

X denotes a nitrogen atom or a methyne group optionally substituted by a methyl group and

Y denotes an imino group optionally substituted by a $C_{1-3}$-alkyl or $C_{3-7}$-cycloalkyl group, or an oxygen or sulphur atom or

X denotes a nitrogen atom and

Y denotes an imino group substituted by a $C_{1-3}$-alkyl group, whilst the alkyl moiety is additionally substituted by a carboxy or $C_{1-3}$-alkyloxy-carbonyl group,

and $R_a$ denotes a benzyloxy group,
an amino group,
a $C_{1-3}$-alkylamino group which is additionally substituted at the nitrogen atom by a benzyl group,
an $R_3$-CO-$R_4$N or $R_3$-$SO_2$-$R_4$N group wherein

$R_3$ denotes a $C_{1-4}$-alkyl, benzyl, $C_{5-7}$-cycloalkyl, phenyl, pyridyl, quinolyl, isoquinolyl, tetrahydroquinolyl or tetrahydroisoquinolyl group and

$R_4$ denotes a hydrogen atom, a $C_{1-3}$-alkyl group which is substituted by a carboxy, $C_{1-3}$-alkoxy-carbonyl, tetrazolyl, aminocarbonyl or $C_{1-3}$-alkylaminocarbonyl group, whilst the aminocarbonyl and $C_{1-3}$-alkylaminocarbonyl group are each additionally substituted at the nitrogen atom by a carboxy-$C_{1-3}$-alkyl or $C_{1-3}$-alkoxy-carbonyl-$C_{1-3}$-alkyl group, or a $C_{2-3}$-alkyl group terminally substituted by a di-($C_{1-3}$-alkyl)-amino group,

the isomers and the salts thereof.

**4.** Disubstituted bicyclic heterocycles of general formula I according to claim 3 wherein
$R_a$ in the 5 position denotes an $R_3$-CO-$R_4$N or $R_3$-$SO_2$-$R_4$N group wherein $R_3$ and $R_4$ are as hereinbefore defined, the isomers and the salts thereof.

**5.** Disubstituted bicyclic heterocycles of general formula Ia

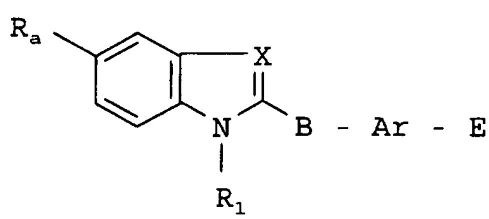

, (Ia)

wherein
X denotes a methyne group or a nitrogen atom,
B denotes an ethylene group, whilst the methylene group linked to Ar may be replaced by an oxygen atom or an imino group,
Ar denotes a 1,4-phenylene group,
E denotes an amidino group,
$R_1$ denotes a methyl group and
$R_a$ denotes an $R_3$-CO-$R_4$N or $R_3$-$SO_2$-$R_4$N group, whilst

$R_4$ denotes a methyl group substituted by a carboxy, $C_{1-3}$-alkoxy-carbonyl, carboxymethylaminocarbonyl or $C_{1-3}$-alkoxy-carbonylmethylaminocarbonyl group and

$R_3$ denotes an isoquinolin-8-yl group,

the isomers and the salts thereof.

**6.** Disubstituted bicyclic heterocycles of general formula Ia according to claim 5 wherein $R_a$ denotes an $R_3$-$SO_2$-$R_4$N group,
the isomers and the salts thereof.

**7.** The following disubstituted bicyclic heterocycles of general formula Ia according to claim 5:

(a) 1-methyl-2-[(4-amidinophenyl)-oxymethyl]-5-[N-(hydroxycarbonylmethyl)-quinoline-8-sulphonylamino]-benzimidazole,

(b) 1-methyl-2-[2-(4-amidinophenyl)-ethyl]-5-[N-(N'-(hydroxycarbonylmethyl)-aminocarbonylmethyl)-quinoline-8-sulphonylamino]-benzimidazole,

(c) 1-methyl-2-[N-(4-amidinophenyl)-aminomethyl]-5-[N-(hydroxycarbonylmethyl)-quinoline-8-sulphonylamino]-benzimidazole and

(d) 1-methyl-2-[N-(4-amidinophenyl)-aminomethyl]-5-[N-(hydroxycarbonylmethyl)-quinoline-8-sulphonylamino]-indole

and the salts thereof.

**8.** Physiologically acceptable salts of the compounds according to claims 1 to 7.

**9.** Pharmaceutical compositions containing a compound according to at least one of claims 1 to 7 wherein E denotes a $R_bNH-C(=NH)$ group, or a salt according to claim 8 optionally together with one or more inert carriers and/or diluents.

**10.** Use of a compound according to at least one of claims 1 to 7 wherein E denotes an $R_bNH-C(=NH)$ group, or a salt according to claim 8 for preparing a pharmaceutical composition with the effects of prolonging the thrombin time, inhibiting thrombin and inhibiting related serine proteases.

**11.** Process for preparing a pharmaceutical composition according to claim 9, **characterised in that** a compound according to at least one of claims 1 to 7 wherein E denotes an $R_bNH-C(=NH)$ group, or a salt according to claim 8, is incorporated in one or more inert carriers and/or diluents by a non-chemical process.

**12.** Process for preparing the compounds according to claims 1 to 8, **characterised in that**

a. in order to prepare a compound of general formula I wherein E denotes a $R_bNH-C(=NH)$ group in which $R_b$ denotes a hydrogen atom, a hydroxy or $C_{1-3}$-alkyl group,
a compound of general formula

$$R_a - Het - B - Ar - C(=NH) - Z_1 , \qquad (II)$$

optionally formed in the reaction mixture wherein
B, Ar, Het and $R_a$ are defined as in claims 1 to 7 and
$Z_1$ denotes an alkoxy, aralkoxy, alkylthio or aralkylthio group, is reacted with an amine of general formula

$$H_2N - R_b' , \qquad (III)$$

wherein
$R_b'$ denotes a hydrogen atom, a hydroxy or $C_{1-3}$-alkyl group, or

b. in order to prepare a compound of general formula I wherein the $R_a$ group and E are defined as in claims 1 to 7 with the proviso that the $R_a$ group contains a carboxy group and E is defined as in claims 1 to 7 or the $R_a$ group is defined as in claims 1 to 7 and E denotes an $NH_2-C(=NH)$ group or the $R_a$ group contains a carboxy group and E denotes an $NH_2-C(=NH)$ group,
a compound of general formula

$$R_a' - Het - B - Ar - E' , \qquad (IV)$$

wherein
A, B, Ar and Het are defined as in claims 1 to 7 and the $R_a$' group and E' have the meanings given for the $R_a$ group and E in claims 1 to 7, with the proviso that the $R_a$' group contains a group which may be converted into a carboxyl group by hydrolysis, treatment with an acid or base, thermolysis or hydrogenolysis and E is defined as in claims 1 to 7 or E' denotes a group which may be converted into an $NH_2$-C(=NH) group by hydrolysis, treatment with an acid or base, thermolysis or hydrogenolysis and the $R_a$' group has the meanings given for the $R_a$ group in claims 1 to 7 or the $R_a$' group contains a group which may be converted into a carboxyl group by hydrolysis, treatment with an acid or base, thermolysis or hydrogenolysis and E' denotes a group which may be converted into an $NH_2$-C(=NH) group by hydrolysis, treatment with an acid or base, thermolysis or hydrogenolysis,
is converted by hydrolysis, treatment with an acid or base, thermolysis or hydrogenolysis into a compound of general formula I wherein the $R_a$ group and E are defined as in claims 1 to 7 with the proviso that the $R_a$ group contains a carboxy group and E is defined as in claims 1 to 7 or the $R_a$ group has the meanings given in claims 1 to 7 and E denotes a $NH_2$-C(=NH) group or the $R_a$ group contains a carboxy group and E denotes a $NH_2$-(C=NH) group, or

c. in order to prepare a compound of general formula I wherein the $R_a$ group contains one of the ester groups mentioned in the definition of the $R_a$ group in claims 1 to 7, a compound of general formula

$$R_a'' - Het - B - Ar - E , \qquad (V)$$

wherein
B, E, Ar and Het are defined as in claims 1 to 7 and $R_a$" group has the meanings given for the $R_a$ group in claims 1 to 7, with the proviso that the $R_a$" group contains a carboxyl group or a group which can be converted into a corresponding ester group by means of an alcohol, is reacted with an alcohol of general formula

$$HO - R_8 , \qquad (VI)$$

wherein
$R_8$ represents the alkyl moiety of one of the groups mentioned in claims 1 to 7 which can be cleaved *in vivo* with the exception of the $R_5$-CO-O-($R_5CR_7$) group for a carboxyl group, or with the formamide acetals thereof or with a compound of general formula

$$Z_2 - R_9 , \qquad (VII)$$

wherein
$R_9$ denotes the alkyl moiety of one of the groups which can be cleaved *in vivo* mentioned in claims 1 to 7, with the exception of the $R_5$-CO-O-($R_5CR_7$) group for a carboxyl group and
$Z_2$ denotes a leaving group, or

d. in order to prepare a compound of general formula I wherein $R_b$ denotes a group which may be cleaved *in vivo*,
a compound of general formula

$$R_a - Het - B - Ar - C(=NH) - NH_2 , \qquad (VIII)$$

wherein
$R_a$, Het, B and Ar are defined as in claims 1 to 7, is reacted with a compound of general formula

$$Z_3 - R_{10} , \qquad (IX)$$

wherein

$R_{10}$ denotes a group which may be cleaved *in vivo* and
$Z_3$ denotes a nucleofugic leaving group, or

e. in order to prepare a compound of general formula I wherein $R_a$ denotes an amino group and E denotes a cyano group, a nitro compound of general formula

$$NO_2 - Het - B - Ar - CN , \qquad (X)$$

wherein
B, Ar and Het are defined as in claims 1 to 7, is reduced or

f. in order to prepare a compound of general formula I wherein $R_a$ denotes an amino group and E denotes a cyano group, a protecting group for an amino group is cleaved from a compound of general formula

$$R_a''' - Het - B - Ar - CN , \qquad (XI)$$

wherein
B, Ar and Het are defined as in claims 1 to 7 and
$R_a'''$ denotes an amino group protected by a protecting group, or

g. in order to prepare a compound of general formula I wherein $R_a$ denotes an $R_3$-CO-$R_4$N or $R_3$-$SO_2$-$R_4$N group and E denotes a cyano group, a compound of general formula

$$R_4NH - Het - B - Ar - CN , \qquad (XII)$$

wherein
$R_4$, Het, B and Ar are defined as in claims 1 to 7, is reacted with a compound of general formula

$$R_3 - X - Z_4 , \qquad (XIII)$$

wherein
$R_3$ is defined as in claims 1 to 7,
X denotes a carbonyl or sulphonyl group and
$Z_4$ denotes a nucleofugic leaving group, or, if X represents a carbonyl group, $Z_4$ together with a hydrogen atom of the adjacent nitrogen atom represents another carbon-nitrogen bond, or

h. in order to prepare a compound of general formula I wherein $R_a$ denotes an $R_3$-CO-$R_4$N or $R_3$-$SO_2$-$R_4$N group and E denotes a cyano group where $R_4$ has the meanings given in claims 1 to 7, with the exception of the hydrogen atom, a compound of general formula

$$R_3 - X - NH - Het - B - Ar - CN , \qquad (XIV)$$

wherein
$R_3$, Het, B, Ar and X are defined as in claims 1 to 7, is reacted with a compound of general formula

$$R_4' - Z_5 , \qquad (XV)$$

wherein
$R_4'$ has the meanings given for $R_4$ in claims 1 to 7 with the exception of the hydrogen atom and
$Z_5$ denotes a nucleofugic leaving group, or

i. in order to prepare a compound of general formula I wherein $R_4$ denotes a $C_{1-5}$-alkyl or phenyl-$C_{1-3}$-alkyl group, each of which is substituted in the alkyl moiety by a group which may be converted in vivo into a carboxy group, by a tetrazolyl group, by an aminocarbonyl or $C_{1-3}$-alkylaminocarbonyl group, each of which is additionally substituted at the nitrogen atom by a group which can be converted *in vivo* into a carboxy-$C_{1-3}$-alkyl group and E denotes a cyano group, a compound of general formula

$$R_3 - X - NR_4' - Het - B - Ar - CN , \qquad (XVI)$$

wherein
$R_3$, Het, B, Ar and X are defined as in claims 1 to 7 and $R_4$' denotes a $C_{1-5}$-alkyl or phenyl-$C_{1-3}$-alkyl group, each of which is substituted in the alkyl moiety by a group which may be converted *in vivo* into a carboxy group, by a tetrazolyl group, by an aminocarbonyl or $C_{1-3}$-alkylaminocarbonyl group, each of which is additionally substituted at the nitrogen atom by a group which may be converted *in vivo* into a carboxy-$C_{1-3}$-alkyl group, or the reactive derivatives thereof, is reacted with a compound of general formula

$$R_{11} - H , \qquad (XVII)$$

wherein
$R_4$' has the meanings given for $R_4$ in claims 1 to 7 with the exception of the hydrogen atom and
$R_{11}$ denotes one of the substituents of the $C_{1-5}$-alkyl or phenyl-$C_{1-3}$-alkyl group mentioned in the definition of the group $R_4$ in claims 1 to 7 which is attached to the group $R_{11}$ via a carbonyl group, or

j. in order to prepare a benzimidazolyl, benzothiazolyl or benzoxazolyl compound of general formula I wherein B denotes an ethylene group, a compound of general formula

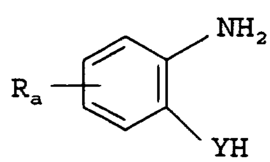

, (XVIII)

optionally formed in the reaction mixture
wherein
$R_a$ and Y are defined as in claims 1 to 7, is reacted with a compound of general formula

$$HO-CO - B' - Ar - E , \qquad (IXX)$$

wherein
Ar and E are defined as in claims 1 to 7 and
B' denotes an ethylene group optionally substituted by one or two $C_{1-3}$-alkyl groups, or

k. In order to prepare a compound of general formula I, which contains one of the above-mentioned tetrahydroquinoline or isoquinoline groups mentioned in claims 1 to 7, a compound of general formula I which contains one of the quinoline or isoquinoline groups mentioned in claims 1 to 7 is hydrogenated, and
subsequently, if necessary, any protecting group used in the reactions described above for any reactive groups which may be present is cleaved and/or
subsequently, if desired, a compound of general formula I thus obtained is resolved into its stereoisomers and/or
a compound of general formula I thus obtained is converted into the salts thereof, particularly the physiologically acceptable salts thereof.

## Revendications

1. Hétérocycles bicycliques disubstitués, de la formule générale :

$$R_a - Het - B - Ar - E \qquad (I)$$

dans laquelle :

B représente un radical éthylène le cas échéant substitué par un ou deux radicaux alkyle en $C_{1-3}$, où un radical méthylène du radical éthylène, qui est relié au reste Het ou Ar, peut être remplacé par un atome d'oxygène ou de soufre, par un radical sulfinyle, sulfonyle, carbonyle ou $-NR_1-$, où
$R_1$ représente un atome d'hydrogène ou un radical alkyle en $C_{1-6}$, ou
B représente également, un radical alkylène linéaire en $C_{3-5}$, dans lequel un radical méthylène, qui n'est relié ni au reste Het, ni au reste Ar, est remplacé par un radical $-NR_1-$, dans lequel $R_1$ est comme défini précédemment,
E représente un radical cyano ou $R_bNH-C(=NH)-$, dans lequel
$R_b$ représente un atome d'hydrogène, un radical hydroxyle, un radical alkyle en $C_{1-3}$ ou un reste clivable in vivo ;
Ar représente un reste phénylène ou naphtylène le cas échéant substitué par l'atome de fluor, de chlore ou de brome, par un radical trifluorométhyle, alkyle en $C_{1-3}$ ou alcoxy en $C_{1-3}$,
représente un radical thiénylène, thiazolylène, pyridinylène, pyrimidinylène, pyrazinylène ou pyridazinylène le cas échéant substitué dans le squelette carboné, par un radical alkyle en $C_{1-3}$ ;
Het représente un hétérocycle bicyclique de la formule :

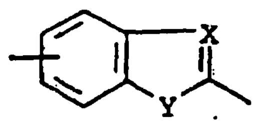

dans laquelle :

X représente l'atome d'azote ou un radical méthine le cas échéant substitué par un radical alkyle en $C_{1-3}$, et
Y représente un radical imino le cas échéant substitué par un radical alkyle en $C_{1-5}$ ou cycloalkyle en $C_{3-7}$, un atome d'oxygène ou un atome de soufre, ou
X représente un atome d'azote, et
Y représente un radical imino substitué par un radical alkyle en $C_{1-5}$ ou cycloalkyle en $C_{3-7}$, où le substituant alkyle ou cycloalkyle est chaque fois substitué par un radical carboxy ou un radical convertible in vivo en un radical carboxy, où dans un des hétérocycles indiqués précédemment, un radical méthine non angulaire peut en outre, être remplacé par un atome d'azote, ou
Het représente un radical de la formule :

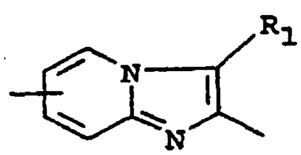

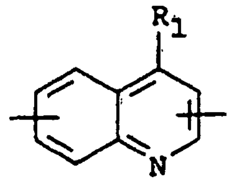

ou

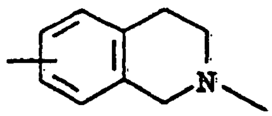

où

$R_1$ est défini comme indiqué précédemment, et

$R_a$ représente un radical phényl(alcoxy en $C_{1-3}$), un radical amino, un radical (alkyle en $C_{1-3}$)amino, qui est substitué en outre sur l'atome d'azote, par un phényl (alkyle en $C_{1-3}$), un radical $R_3$-CO-$R_4$N- ou $R_3$-$SO_2$-$R_4$N-, dans lequel :

$R_3$ représente un radical alkyle en $C_{1-5}$, phényl (alkyle en $C_{1-3}$), cycloalkyle en $C_{3-7}$, phényle, naphtyle, pyridyle, quinoléyle, isoquinoléyle, tétrahydroquinoléyle ou tétrahydroisoquinoléyle, et
$R_4$ représente un atome d'hydrogène, un radical alkyle en $C_{1-5}$ ou phényl (alkyle en $C_{1-3}$), qui sont substitués chaque fois dans la partie alkyle, par un radical convertible in vivo en un radical carboxy, par un radical carboxy ou tétrazolyle, par un radical aminocarbonyle ou (alkyle en $C_{1-3}$)aminocarbonyle, qui sont substitués en outre chaque fois sur l'atome d'azote, par un radical convertible in vivo en un radical carboxy (alkyle en $C_{1-3}$) ou par un radical carboxy, un radical alkyle en $C_{2-5}$ substitué de manière terminale par un radical di (alkyl en $C_{1-3}$) amino ou un radical cycloalkyle en $C_{3-7}$,

leurs tautomères, leurs stéréoisomères et leurs sels.

**2.** Hétérocycles bicycliques disubstitués, de la formule générale I selon la revendication 1, dans laquelle :

B représente un radical éthylène le cas échéant substitué par un ou deux radicaux méthyle, où un radical méthylène du radical éthylène, qui est relié au reste Het ou Ar, peut être remplacé par un atome d'oxygène ou de soufre, par un radical carbonyle ou -$NR_1$-, où
$R_1$ représente un atome d'hydrogène ou un radical méthyle, ou
B représente également, un radical n-propylène, dans lequel le radical méthylène du milieu est remplacé par un radical -$NR_1$-, dans lequel $R_1$ est comme défini précédemment,
E représente un radical cyano ou $R_b$NH-C(=NH)-, dans lequel

$R_b$ représente un atome d'hydrogène, un radical (alkoxy en $C_{1-8}$)carbonyle, (cycloalkoxy en $C_{5-7}$) carbonyle, benzoyle, nicotinoyle ou isonicotinoyle ;
Ar représente un reste phénylène le cas échéant substitué par l'atome de fluor, de chlore ou de brome, par un radical trifluorométhyle, méthyle ou méthoxy, ou représente un radical thiénylène, le cas échéant substitué dans le squelette carboné, par un radical méthyle ;
Het représente un hétérocycle bicyclique de la formule :

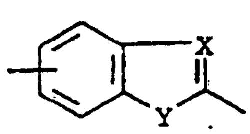

dans laquelle :

X représente l'atome d'azote ou un radical méthine le cas échéant substitué par un radical méthyle, et
Y représente un radical imino le cas échéant substitué par un radical alkyle en $C_{1-3}$ ou cycloalkyle en $C_{3-7}$, un atome d'oxygène ou un atome de soufre, ou
X représente l'atome d'azote, et
Y représente un radical imino substitué par un radical alkyle en $C_{1-3}$, où la partie alkyle est en outre, substituée par un radical carboxy ou un radical (alkyloxy en $C_{1-3}$)carbonyle, ou
Het représente un radical de la formule :

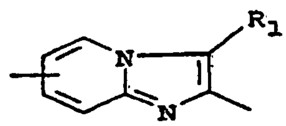

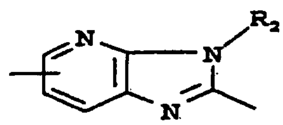

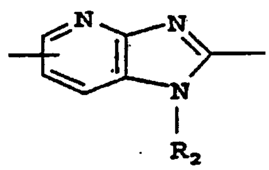

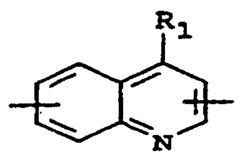

ou

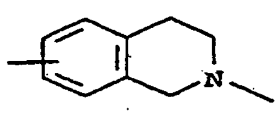

où
$R_1$ est défini comme indiqué précédemment, et
$R_2$ représente un radical alkyle en $C_{1-3}$, qui est substitué par un radical carboxy ou (alcoxy en $C_{1-3}$) carbonyle, et

$R_a$ représente un radical benzyloxy, un radical amino, un radical (alkyl en $C_{1-3}$) amino, qui est en outre substitué sur l'atome d'azote, par un radical benzyle, un radical $R_3$-CO-$R_4$N- ou $R_3$-$SO_2$-$R_4$N-, dans lequel :

$R_3$ représente un radical alkyle en $C_{1-4}$, benzyle, cycloalkyle en $C_{5-7}$, phényle, pyridyle, quinoléyle, isoquinoléyle, tétrahydroquinoléyle ou tétrahydroisoquinoléyle, et
$R_4$ représente un atome d'hydrogène, un radical alkyle en $C_{1-3}$, qui est substitué par un radical carboxy, (alcoxy en $C_{1-3}$)carbonyle, tétrazolyle, aminocarbonyle ou (alkyl en $C_{1-3}$) aminocarbonyle, où les radicaux aminocarbonyle et (alkyl en $C_{1-3}$)aminocarbonyle sont substitués en outre chaque fois sur l'atome d'azote, par un carboxy (alkyle en $C_{1-3}$) ou (alcoxy en $C_{1-3}$) carbonyl (alkyle en $C_{1-3}$), ou représente un radical alkyle en $C_{2-3}$ substitué de manière terminale par un radical di(alkyl en $C_{1-3}$)amino,

leurs isomères et leurs sels.

**3.** Hétérocycles bicycliques disubstitués, de la formule générale I selon la revendication 1, dans laquelle :

B représente un radical éthylène le cas échéant substitué par un ou deux radicaux méthyle, où un radical méthylène du radical éthylène, qui est relié au reste Het ou Ar, peut être remplacé par un atome d'oxygène ou de soufre, par un radical carbonyle ou $-NR_1-$, où
$R_1$ représente un atome d'hydrogène ou un radical méthyle, ou
B représente également, le radical n-propylène, dans lequel le radical méthylène du milieu est remplacé par un radical $-NR_1-$, dans lequel $R_1$ est comme défini précédemment,
E représente un radical $R_bNH-C(=NH)-$, dans lequel
$R_b$ représente un atome d'hydrogène, un radical (alkoxy en $C_{1-8}$)carbonyle, (cycloalkoxy en $C_{5-7}$)carbonyle ou benzoyle ;
Ar représente un reste phénylène le cas échéant substitué par l'atome de fluor, de chlore ou de brome, par un radical trifluorométhyle, méthyle ou méthoxy, ou un radical thiénylène, le cas échéant substitué dans le squelette carboné, par le radical méthyle ;
Het représente un hétérocycle bicyclique de la formule :

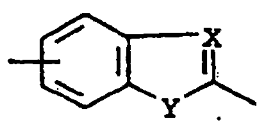

dans laquelle :

X représente un atome d'azote ou un radical méthine le cas échéant substitué par un radical méthyle, et
Y représente un radical imino le cas échéant substitué par un radical alkyle en $C_{1-3}$ ou cycloalkyle en $C_{3-7}$, représente un atome d'oxygène ou un atome de soufre, ou
X représente un atome d'azote, et
Y représente un radical imino substitué par un radical alkyle en $C_{1-3}$, où la partie alkyle est en outre, substituée par un radical carboxy ou (alkyloxy en $C_{1-3}$)carbonyle, et
$R_a$ représente un radical benzyloxy, un radical amino, un radical (alkyl en $C_{1-3}$) amino, qui est en outre substitué sur l'atome d'azote, par un radical benzyle, représente un radical $R_3-CO-R_4N-$ ou $R_3-SO_2-R_4N-$, dans lequel :

$R_3$ représente un radical alkyle en $C_{1-4}$, benzyle, cycloalkyle en $C_{5-7}$, phényle, pyridyle, quinoléyle, isoquinoléyle, tétrahydroquinoléyle ou tétrahydroisoquinoléyle, et
$R_4$ représente un atome d'hydrogène, un radical alkyle en $C_{1-3}$, qui est substitué par un radical carboxy, (alcoxy en $C_{1-3}$)carbonyle, tétrazolyle, aminocarbonyle ou (alkyl en $C_{1-3}$)aminocarbonyle, où les radicaux aminocarbonyle et (alkyl en $C_{1-3}$)aminocarbonyle sont substitués en outre chaque fois sur l'atome d'azote, par un radical carboxy (alkyle en $C_{1-3}$) ou (alcoxy en $C_{1-3}$) carbonyl(alkyle en $C_{1-3}$), ou représente un radical alkyle en $C_{2-3}$ substitué de manière terminale par un radical di(alkyl en $C_{1-3}$)amino,

leurs isomères et leurs sels.

**4.** Hétérocycles bicycliques disubstitués, de la formule générale I selon la revendication 3, dans laquelle :

$R_a$ représente un radical $R_3-CO-R_4N-$ ou $R_3-SO_2-R_4N-$, dans lequel $R_3$ et $R_4$ sont comme définis ci-dessus,

leurs isomères et leurs sels.

**5.** Hétérocycles bicycliques disubstitués, de la formule générale (Ia) :

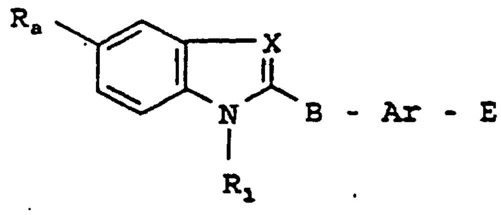

, (Ia)

dans laquelle :

X représente un radical méthine ou un atome d'azote,
B représente un radical éthylène, où le radical méthylène relié au reste Ar, peut être remplacé par un atome d'oxygène ou par un radical imino,
Ar représente le radical 1,4-phénylène,
E représente un radical amidino,
$R_1$ représente le radical méthyle, et
$R_a$ représente un radical $R_3$-CO-$R_4$N- ou $R_3$-$SO_2$-$R_4$N-,

dans lequel :

$R_4$ représente le radical méthyle, substitué par un radical carboxy, (alcoxy en $C_{1-3}$)carbonyle, carboxyméthylaminocarbonyle ou (alcoxy en $C_{1-3}$)carbonylméthylaminocarbonyle, et
$R_3$ représente un radical isoquinoléin-8-yle,

leurs isomères et leurs sels.

**6.** Hétérocycles bicycliques disubstitués, de la formule générale (Ia) selon la revendication 5, dans laquelle $R_a$ représente un radical $R_3$-$SO_2$-$R_4$N-,
leurs isomères et leurs sels.

**7.** Hétérocycles bicycliques disubstitués de la formule générale (Ia) selon la revendication 5, suivants :

(a) le 1-méthyl-2-[(4-amidinophényl)oxyméthyl]-5-[N-(hydroxycarbonylméthyl)quinoléin-8-sulfonylamino]-benzimidazole ;
(b) le 1-méthyl-2-[2-(4-amidinophényl)éthyl]-5-[N-(N'-hydroxycarbonylméthyl)aminocarbonylméthyl)quinoléin-8-sulfonylamino]benzimidazole ;
(c) le 1-méthyl-2-[N-(4-amidinophényl)aminométhyl]-5-[N-(hydroxycarbonylméthyl)quinoléin-8-sulfonylamino]-benzimidazole, et
(d) le 1-méthyl-2-[N-(4-amidinophényl)aminométhyl]-5-[N-(hydroxycarbonylméthyl)quinoléin-8-sulfonylamino]-indole,

ainsi que leurs sels.

**8.** Sels physiologiquement compatibles des composés selon les revendications 1 à 7.

**9.** Agent pharmaceutique, contenant un composé selon au moins une des revendications 1 à 7, où E représente un radical $R_b$NH-C(=NH)-, ou un sel selon la revendication 8, en plus de le cas échéant, un ou plusieurs supports et/ou agents de dilution inertes.

**10.** Utilisation d'un composé selon au moins une des revendications 1 à 7, où E représente un radical $R_b$NH-C(=NH)-, ou un sel selon la revendication 8, pour la préparation d'un agent pharmaceutique avec une action prolongée de la durée de la thrombine, une action d'inhibition de la thrombine et une action inhibitrice sur les protéases à sérine

apparentées.

**11.** Procédé de préparation d'un agent pharmaceutique selon la revendication 9, **caractérisé en ce que** l'on incorpore par une voie non chimique, un composé selon au moins une des revendications 1 à 7, où E représente un radical $R_bNH-C(=NH)-$, ou un sel selon la revendication 8, dans un ou plusieurs supports et/ou agents de dilution inertes.

**12.** Procédé de préparation de composés selon les revendications 1 à 8, **caractérisé en ce que** :

a. pour la préparation d'un composé de la formule générale I, dans laquelle E représente un radical $R_bNH-C(=NH)-$, dans laquelle $R_b$ représente un atome d'hydrogène, un radical hydroxy ou alkyle en $C_{1-3}$, on fait réagir un composé le cas échéant formé dans le mélange réactionnel, de la formule générale :

$$R_a - Het - B - Ar - C(=NH) - Z_1 \quad (II)$$

dans laquelle :

B, Ar, Het et $R_a$ sont définis comme indiqué aux revendications 1 à 7, et
$Z_1$ représente un radical alcoxy, aralcoxy, alkylthio ou aralkylthio,
avec une amine de la formule générale :

$$H_2N - R_b' \quad (III)$$

dans laquelle :

$R_b'$ représente un atome d'hydrogène, un radical hydroxy ou alkyle en $C_{1-3}$, ou

b. pour la préparation d'un composé de la formule générale I, dans laquelle le radical $R_a$ et E sont définis comme indiqué au revendications 1 à 7, avec la condition que le radical $R_a$ contient un radical carboxy et que E est défini comme indiqué au revendications 1 à 7, ou que le radical $R_a$ est défini comme indiqué au revendications 1 à 7 et que E représente un radical $NH_2-C(=NH)-$, ou que le radical $R_a$ contient un radical carboxy et que E représente un radical $NH_2-C(=NH)-$,
on convertit par hydrolyse, traitement avec un acide ou une base, thermolyse ou hydrogénolyse, un composé de la formule générale :

$$R_a' - Het - B - Ar - E' \quad (IV)$$

dans laquelle :

A, B, Ar et Het sont définis comme indiqué aux revendications 1 à 7, et
le radical $R_a'$ et E' possèdent les significations indiquées dans les revendications 1 à 7 pour le radical $R_a$ et E, avec la condition que le radical $R_a'$ contient un radical convertible par hydrolyse, traitement avec un acide ou une base, thermolyse ou hydrogénolyse, en un radical carboxyle et E est défini comme indiqué aux revendications 1 à 7, ou E' représente un radical convertible par hydrolyse, traitement avec un acide ou une base, thermolyse ou hydrogénolyse, en un radical $NH_2-C(=NH)-$ et le radical $R_a'$ présente les significations indiquées dans les revendications 1 à 7 pour le radical $R_a$, ou le radical $R_a'$ contient un radical convertible par hydrolyse, traitement avec un acide ou une base, thermolyse ou hydrogénolyse, en un radical carboxyle et E' représente un radical convertible par hydrolyse, traitement avec un acide ou une base, thermolyse ou hydrogénolyse, en un radical $NH_2-C(=NH)-$
en un composé de la formule générale I, dans laquelle le radical $R_a$ et E sont définis comme indiqué au revendications 1 à 7, avec la condition que le radical $R_a$ contient un radical carboxy et que E est défini comme indiqué aux revendications 1 à 7, ou que le radical $R_a$ présente les significations indiquées aux revendications 1 à 7 et que E représente un radical $NH_2-C(=NH)-$, ou que le radical $R_a$ contient un radical carboxy et que E représente un radical $NH_2-C(=NH)-$, ou

c. pour la préparation d'un composé de la formule générale I, dans laquelle le radical $R_a$ contient un des radicaux ester indiqués dans la définition du radical $R_a$ dans les revendications 1 à 7,

on fait réagir un composé de la formule générale :

$$R_a'' - Het - B - Ar - E \qquad (V)$$

dans laquelle :

B, E, Ar et Het sont définis comme indiqué aux revendications 1 à 7, et
le radical $R_a''$ présente les significations indiquées dans les revendications 1 à 7 pour le radical $R_a$, avec la condition que le radical $R_a''$ contient un radical carboxyle ou un radical convertissable en un radical ester à l'aide d'un alcool,
avec un alcool de la formule générale :

$$HO - R_8 \qquad (VI)$$

dans laquelle:

$R_8$ représente la partie alkyle d'un des restes clivables in vivo indiqués dans les revendications 1 à 7, à l'exception du radical $R_5$-CO-O-($R_5CR_7$)- pour un radical carboxyle, ou
avec leurs acétals formamide, ou
avec un composé de la formule générale:

$$Z_2 - R_9 \qquad (VII)$$

dans laquelle:

$R_9$ représente la partie alkyle d'un des restes clivables in vivo indiqués dans les revendications 1 à 7 pour un radical carboxyle, à l'exception du radical $R_5$-CO-O-($R_5CR_7$)-, et
$Z_2$ représente un groupe partant, ou

d. pour la préparation d'un composé de la formule générale I, dans laquelle le radical $R_b$ représente un reste clivable in vivo,

on fait réagir un composé de la formule générale :

$$R_a - Het - B - Ar - C(=NH) - NH_2 \qquad (VIII)$$

dans laquelle :

$R_a$, Het, B et Ar sont définis comme indiqué comme indiqué aux revendications 1 à 7,
avec un composé de la formule générale :

$$Z_3 - R_{10} \qquad (IX)$$

dans laquelle:

$R_{10}$ représente un reste clivable *in vivo,* et
$Z_3$ représente un groupe partant nucléofuge, ou

e. pour la préparation d'un composé de la formule générale I, dans laquelle le radical $R_a$ représente un radical amino et E représente le radical cyano,

on réduit un composé nitro de la formule générale :

$$NO_2 - Het - B - Ar - CN \quad (X)$$

dans laquelle :

B, Ar et Het sont définis comme indiqué aux revendications 1 à 7, ou

f. pour la préparation d'un composé de la formule générale I, dans laquelle le radical $R_a$ représente un radical amino et E représente le radical cyano,
on clive un radical protecteur de radical amino d'un composé de la formule générale :

$$R_a''' - Het - B - Ar - CN \quad (XI)$$

dans laquelle :

B, Ar et Het sont définis comme indiqué aux revendications 1 à 7, et
$R_a'''$ représente un radical amino protégé par un groupe protecteur, ou

g. pour la préparation d'un composé de la formule générale I, dans laquelle le radical $R_a$ représente un radical $R_3$-CO-$R_4$N- ou $R_3$-$SO_2$-$R_4$N- et E représente le radical cyano,
on fait réagir un composé de la formule générale :

$$R_4NH - Het - B - Ar - CN \quad (XII)$$

dans laquelle :

$R_4$, Het, B et Ar sont définis comme indiqué aux revendications 1 à 7,
avec un composé de la formule générale :

$$R_3 - X - Z_4 \quad (XIII)$$

dans laquelle:

$R_3$ est défini comme indiqué aux revendications 1 à 7,
X représente un radical carbonyle ou sulfonyle, et
$Z_4$ représente un groupe partant nucléofuge, ou également, si X représente un radical carbonyle, représente avec un atome d'hydrogène de l'atome d'azote voisin, une autre liaison carbone-azote, ou

h. pour la préparation d'un composé de la formule générale I, dans laquelle le radical $R_a$ représente un radical $R_3$-CO-$R_4$N- ou $R_3$-$SO_2$-$R_4$N- et E représente un radical cyano, où $R_4$ est défini comme indiqué dans les revendications 1 à 7, à l'exception de l'atome d'hydrogène,
on fait réagir un composé de la formule générale :

$$R_3 - X - NH - Het - B - Ar - CN \quad (XIV)$$

dans laquelle :

$R_3$, Het, B, Ar et X sont définis comme indiqué dans les revendications 1 à 7,
avec un composé de la formule générale :

$$R_4' - Z_5 \quad (XV)$$

dans laquelle:

$R_4$' possède les significations pour $R_4$ comme indiqué aux revendications 1 à 7, à l'exception de l'atome d'hydrogène, et
$Z_5$ représente un groupe partant nucléofuge, ou

i. pour la préparation d'un composé de la formule générale I, dans laquelle le radical $R_4$ représente un radical alkyle en $C_{1-5}$ ou phényl(alkyle en $C_{1-3}$), qui sont substitués chaque fois dans la partie alkyle, par un radical convertissable in vivo en un radical carboxy, par un radical tétrazolyle, par un radical aminocarbonyle ou (alkyl en $C_{1-3}$)aminocarbonyle, qui est substitué chaque fois sur l'atome d'azote, en outre par un radical convertissable in vivo en un radical carboxy(alkyle en $C_{1-3}$), et E représente le radical cyano,
on fait réagir un composé de la formule générale :

$$R_3 - X - NR_4' - Het - B - Ar - CN \qquad (XVI)$$

dans laquelle :

$R_3$, Het, B, Ar et X sont définis comme indiqué dans les revendications 1 à 7, et
$R_4$' représente un radical alkyle en $C_{1-5}$ ou phényl(alkyle en $C_{1-3}$), qui sont substitués chaque fois dans la partie alkyle, par un radical convertissable in vivo en un radical carboxy, par un radical tétrazolyle, par un radical aminocarbonyle ou (alkyl en $C_{1-3}$)amino-carbonyle, qui est substitué chaque fois sur l'atome d'azote, en outre par un radical convertissable in vivo en un radical carboxy(alkyle en $C_{1-3}$), ou leurs dérivés réactifs,
avec un composé de la formule générale :

$$R_{11} - H \qquad (XVII)$$

dans laquelle:

$R_{4'}$ possède les significations pour $R_4$ comme indiqué aux revendications 1 à 7 à l'exception de l'atome d'hydrogène,
$R_{11}$ représente un des substituants du radical alkyle en $C_{1-5}$ ou phényl (alkyle en $C_{1-3}$), indiqués dans la définition du reste $R_4$ aux revendications 1 à 7 qui est lié par un groupe carbonyle au reste $R_{11}$, ou

j. pour la préparation d'un composé benzimidazolyle, benzthiazolyle ou benzoxazolyle de la formule générale I, dans laquelle B représente un radical éthylène,
on fait réagir un composé le cas échéant formé dans le mélange réactionnel, de la formule générale :

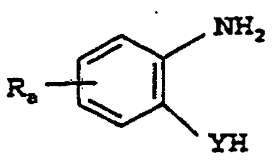

, (XVIII)

dans laquelle :

$R_a$ et Y sont définis comme indiqué dans les revendications 1 à 7,
avec un composé de la formule générale :

$$HO - CO - B' - Ar - E \qquad (IXX)$$

dans laquelle:

Ar et E sont définis comme indiqué dans les revendications 1 à 7, et
B' représente un radical éthylène le cas échéant substitué par un ou deux radicaux alkyle en $C_{1-3}$, ou

k. pour la préparation d'un composé de la formule générale I, qui contient un des restes tétrahydroquinoléine ou tétrahydroisoquinoléine indiqués aux revendications 1 à 7,

on réalise l'hydrogénation d'un composé de la formule générale I, qui contient un des restes quinoléine ou isoquinoléine indiqués aux revendications 1 à 7, et

si nécessaire, on clive un radical protecteur utilisé dans les réactions décrites ci-dessus, pour des radicaux réactifs le cas échéant présents, et/ou

si souhaité, on sépare un composé de la formule générale I ainsi obtenu en ses stéréoisomères, et/ou

on convertit un composé de la formule générale I ainsi obtenu en son sel, en particulier en son sel physiologiquement compatible.